# EUROPEAN PATENT APPLICATION

(11) **EP 1 069 188 A1**
(43) Date of publication of application: **17.01.2001**
(21) Application number: 99401767.1
(22) Date of filing: 15.07.1999
(51) Int. Cl.: C12N 15/57, C12N 9/64, C12N 5/10, C07K 16/40, A61K 38/48, A61K 48/00, C12Q 1/68, C12Q 1/37

(54) **Three neprilysin-like membrane metallopeptidases**

(71) Applicant: Sanofi-Synthélabo, 75013 Paris (FR)
(72) Inventor: Jagerschmidt, Alexandre, 92340 Bourg-la-Reine (FR); Agnel, Magali, 92500 Rueil Malmaison (FR); Culouscou, Jean-Michel, 78110 Le Vésinet (FR)
(74) Representative: Thouret-Lemaitre, Elisabeth

(57) **Abstract**

SNEPa, SNEPb or SNEPc polypeptides, the corresponding polynucleotide sequences and methods for producing such polypeptides by recombinant techniques are disclosed. Also disclosed are methods for utilizing SNEPa, SNEPb or SNEPc polypeptides and polynucleotides in the design of protocols for the treatment of diseases, to mimic or antagonize effect of endogenous neurotransmitters and hormones, or to diagnose or treat any disorder related to abnormal expression of these proteins, among others and diagnostic assays for such conditions.

SEQUENCE LISTING

<110> SANOFI-SYNTHELABO

<120> THREE MEMBRANE METALLOPEPTIDASES, THEIR ISOLATION AND
      USE

<130> snep

<140>
<141>

<160> 6

<170> PatentIn Ver. 2.1

<210> 1
<211> 2714
<212> DNA
<213> Homo sapiens

<220>
<221> CDS
<222> (8)..(2347)

<220>
<221> 3'UTR
<222> (2348)..(2713)

<220>
<221> 5'UTR
<222> (1)..(7)

<400> 1
cctgctg atg ggg aag tcc gaa ggc ccc gtg ggg atg gtg gag agc gct    49
        Met Gly Lys Ser Glu Gly Pro Val Gly Met Val Glu Ser Ala
          1               5                  10

ggc cgt gca ggg cag aag cgc ccg ggg ttc ctg gag ggg ggg ctg ctg    97
Gly Arg Ala Gly Gln Lys Arg Pro Gly Phe Leu Glu Gly Gly Leu Leu
 15                  20                  25                  30

ctg ctg ctg ctg ctg gtg acc gct gcc ctg gtg gcc ttg ggt gtc ctc    145
Leu Leu Leu Leu Leu Val Thr Ala Ala Leu Val Ala Leu Gly Val Leu
                 35                  40                  45

tac gcc gac cgc aga ggg aag cag ctg cca cgc ctt gct agc cgg ctg    193
Tyr Ala Asp Arg Arg Gly Lys Gln Leu Pro Arg Leu Ala Ser Arg Leu
             50                  55                  60

tgc ttc tta cag gag gag agg acc ttt gta aaa cga aaa ccc cga ggg    241
Cys Phe Leu Gln Glu Glu Arg Thr Phe Val Lys Arg Lys Pro Arg Gly
         65                  70                  75

atc cca gag gcc caa gag gtg agc gag gtc tgc acc acc cct ggc tgc    289
Ile Pro Glu Ala Gln Glu Val Ser Glu Val Cys Thr Thr Pro Gly Cys
     80                  85                  90

gtg ata gca gct gcc agg atc ctc cag aac atg gac ccg acc acg gaa    337
Val Ile Ala Ala Ala Arg Ile Leu Gln Asn Met Asp Pro Thr Thr Glu
 95                 100                 105                 110

ccg tgt gac gac ttc tac cag ttt gca tgc gga ggc tgg ctg cgg cgc    385
Pro Cys Asp Asp Phe Tyr Gln Phe Ala Cys Gly Gly Trp Leu Arg Arg
                115                 120                 125

cac gtg atc cct gag acc aac tca aga tac agc atc ttt gac gtc ctc    433
His Val Ile Pro Glu Thr Asn Ser Arg Tyr Ser Ile Phe Asp Val Leu
            130                 135                 140

cgc gac gag ctg gag gtc atc ctc aaa gcg gtg ctg gag aat tcg act    481
Arg Asp Glu Leu Glu Val Ile Leu Lys Ala Val Leu Glu Asn Ser Thr

145                 150                 155

gcc aag gac cgg ccg gct gtg gag aag gcc agg acg ctg tac cgc tcc     529
Ala Lys Asp Arg Pro Ala Val Glu Lys Ala Arg Thr Leu Tyr Arg Ser
    160                 165                 170

tgc atg aac cag agt gtg ata gag aag cga ggc tct cag ccc ctg ctg     577
Cys Met Asn Gln Ser Val Ile Glu Lys Arg Gly Ser Gln Pro Leu Leu
175                 180                 185                 190

gac atc ttg gag gtg gtg gga ggc tgg ccg gtg gcg atg gac agg tgg     625
Asp Ile Leu Glu Val Val Gly Gly Trp Pro Val Ala Met Asp Arg Trp
                195                 200                 205

aac gag acc gta gga ctc gag tgg gag ctg gag cgg cag ctg gcg ctg     673
Asn Glu Thr Val Gly Leu Glu Trp Glu Leu Glu Arg Gln Leu Ala Leu
            210                 215                 220

atg aac tca cag ttc aac agg cgc gtc ctc atc gac ctc ttc atc tgg     721
Met Asn Ser Gln Phe Asn Arg Arg Val Leu Ile Asp Leu Phe Ile Trp
        225                 230                 235

aac gac gac cag aac tcc agc cgg cac atc atc tac ata gac cag ccc     769
Asn Asp Asp Gln Asn Ser Ser Arg His Ile Ile Tyr Ile Asp Gln Pro
    240                 245                 250

acc ttg ggc atg ccc tcc cga gag tac tac ttc aac ggc ggc agc aac     817
Thr Leu Gly Met Pro Ser Arg Glu Tyr Tyr Phe Asn Gly Gly Ser Asn
255                 260                 265                 270

cgg aag gtg cgg gaa gcc tac ctg cag ttc atg gtg tca gtg gcc acg     865
Arg Lys Val Arg Glu Ala Tyr Leu Gln Phe Met Val Ser Val Ala Thr
                275                 280                 285

ttg ctg cgg gag gat gca aac ctg ccc agg gac agc tgc ctg gtg cag     913
Leu Leu Arg Glu Asp Ala Asn Leu Pro Arg Asp Ser Cys Leu Val Gln
            290                 295                 300

gag gac atg gtg cag gtg ctg gag ctg gag aca cag ctg gcc aag gcc     961
Glu Asp Met Val Gln Val Leu Glu Leu Glu Thr Gln Leu Ala Lys Ala
        305                 310                 315

acg gta ccc cag gag gag aga cac gac gtc atc gcc ttg tac cac cgg     1009
Thr Val Pro Gln Glu Glu Arg His Asp Val Ile Ala Leu Tyr His Arg
    320                 325                 330

atg gga ctg gag gag ctg caa agc cag ttt ggc ctg aag gga ttt gac     1057
Met Gly Leu Glu Glu Leu Gln Ser Gln Phe Gly Leu Lys Gly Phe Asp
335                 340                 345                 350

tgg act ctg ttc ata caa act gtg cta tcc tct gtc aaa atc aag ctg     1105
Trp Thr Leu Phe Ile Gln Thr Val Leu Ser Ser Val Lys Ile Lys Leu
                355                 360                 365

ctg cca gat gag gaa gtg gtg gtc tat ggc atc ccc tac ctg cag aac     1153
Leu Pro Asp Glu Glu Val Val Val Tyr Gly Ile Pro Tyr Leu Gln Asn
            370                 375                 380

ctt gaa aac atc atc gac acc tac tca gcc agg acc ata cag aac tac     1201
Leu Glu Asn Ile Ile Asp Thr Tyr Ser Ala Arg Thr Ile Gln Asn Tyr
        385                 390                 395

ctg gtc tgg cgc ctg gtg ctg gac cgc att ggt agc cta agc cag aga     1249
Leu Val Trp Arg Leu Val Leu Asp Arg Ile Gly Ser Leu Ser Gln Arg
    400                 405                 410

ttc aag gac aca cga gtg aac tac cgc aag gcg ctg ttt ggc aca atg     1297
Phe Lys Asp Thr Arg Val Asn Tyr Arg Lys Ala Leu Phe Gly Thr Met
415                 420                 425                 430

gtg gag gag gtg cgc tgg cgt gaa tgt gtg ggc tac gtc aac agc aac     1345
Val Glu Glu Val Arg Trp Arg Glu Cys Val Gly Tyr Val Asn Ser Asn

                435                 440                 445

atg gag aac gcc gtg ggc tcc ctc tac gtc agg gag gcg ttc cct gga 1393
Met Glu Asn Ala Val Gly Ser Leu Tyr Val Arg Glu Ala Phe Pro Gly
            450                 455                 460

gac agc aag agc atg gtc aga gaa ctc att gac aag gtg cgg aca gtg 1441
Asp Ser Lys Ser Met Val Arg Glu Leu Ile Asp Lys Val Arg Thr Val
        465                 470                 475

ttt gtg gag acg ctg gac gag ctg ggc tgg atg gac gag gag tcc aag 1489
Phe Val Glu Thr Leu Asp Glu Leu Gly Trp Met Asp Glu Glu Ser Lys
    480                 485                 490

aag aag gcg cag gag aag gcc atg agc atc cgg gag cag atc ggg cac 1537
Lys Lys Ala Gln Glu Lys Ala Met Ser Ile Arg Glu Gln Ile Gly His
495                 500                 505                 510

cct gac tac atc ctg gag gag acg aac agg cgc ctg gac gag gag tac 1585
Pro Asp Tyr Ile Leu Glu Glu Thr Asn Arg Arg Leu Asp Glu Glu Tyr
                515                 520                 525

tcc aat ctg aac ttc tca gag gac ctg tac ttt gag aac agt ctg cag 1633
Ser Asn Leu Asn Phe Ser Glu Asp Leu Tyr Phe Glu Asn Ser Leu Gln
            530                 535                 540

aac ctc aag gtg ggc gcc cag cgg agc ctc agg aag ctt cgg gaa aag 1681
Asn Leu Lys Val Gly Ala Gln Arg Ser Leu Arg Lys Leu Arg Glu Lys
        545                 550                 555

gtg gac cca aat ctc tgg atc atc ggg gcg gcg gtg gtc aat gcg ttc 1729
Val Asp Pro Asn Leu Trp Ile Ile Gly Ala Ala Val Val Asn Ala Phe
    560                 565                 570

tac tcc cca aac cga aac cag att gta ttc cct gcc ggg atc ctc cag 1777
Tyr Ser Pro Asn Arg Asn Gln Ile Val Phe Pro Ala Gly Ile Leu Gln
575                 580                 585                 590

ccc ccc ttc ttc agc aag gag cag cca cag gcc ttg aac ttt gga ggc 1825
Pro Pro Phe Phe Ser Lys Glu Gln Pro Gln Ala Leu Asn Phe Gly Gly
                595                 600                 605

att ggg atg gtg atc ggg cac gag atc acg cac ggc ttt gac gac aat 1873
Ile Gly Met Val Ile Gly His Glu Ile Thr His Gly Phe Asp Asp Asn
            610                 615                 620

ggc cgg aac ttc gac aag aat ggc aac atg atg gat tgg tgg agt aac 1921
Gly Arg Asn Phe Asp Lys Asn Gly Asn Met Met Asp Trp Trp Ser Asn
        625                 630                 635

ttc tcc acc cag cac ttc cgg gag cag tca gag tgc atg atc tac cag 1969
Phe Ser Thr Gln His Phe Arg Glu Gln Ser Glu Cys Met Ile Tyr Gln
    640                 645                 650

tac ggc aac tac tcc tgg gac ctg gca gac gaa cag aac gtg aac gga 2017
Tyr Gly Asn Tyr Ser Trp Asp Leu Ala Asp Glu Gln Asn Val Asn Gly
655                 660                 665                 670

ttc aac acc ctt ggg gaa aac att gct gac aac gga ggg gtg cgg caa 2065
Phe Asn Thr Leu Gly Glu Asn Ile Ala Asp Asn Gly Gly Val Arg Gln
                675                 680                 685

gcc tat aag gcc tac ctc aag tgg atg gca gag ggt ggc aag gac cag 2113
Ala Tyr Lys Ala Tyr Leu Lys Trp Met Ala Glu Gly Gly Lys Asp Gln
            690                 695                 700

cag ctg ccc ggc ctg gat ctc acc cat gag cag ctc ttc ttc atc aac 2161
Gln Leu Pro Gly Leu Asp Leu Thr His Glu Gln Leu Phe Phe Ile Asn
        705                 710                 715

tat gcc cag gtg tgg tgc ggg tcc tac cgg ccc gag ttc gcc atc caa 2209
Tyr Ala Gln Val Trp Cys Gly Ser Tyr Arg Pro Glu Phe Ala Ile Gln

720                 725                 730

tcc atc aag aca gac gtc cac agt ccc ctg aag tac agg gta ctg ggg     2257
Ser Ile Lys Thr Asp Val His Ser Pro Leu Lys Tyr Arg Val Leu Gly
735                 740                 745                 750

tcg ctg cag aac ctg gcc gcc ttc gca gac acg ttc cac tgt gcc cgg     2305
Ser Leu Gln Asn Leu Ala Ala Phe Ala Asp Thr Phe His Cys Ala Arg
                755                 760                 765

ggc acc ccc atg cac ccc aag gag cga tgc cgc gtg tgg tag             2347
Gly Thr Pro Met His Pro Lys Glu Arg Cys Arg Val Trp
            770                 775                 780

ccaaggccct gccgcgctgt gcggcccacg cccacctgct gctcggaggc atctgtgcga 2407

aggtgcagct agcggcgacc cagtgtacgt cccgccccgg ccaaccatgc caagcctgcc 2467

tgccaggcct ctgcgcctgg cctagggtgc agccacctgc ctgacaccca gggatgagca 2527

gtgtccagtg cagtacctgg accggagccc cctccacaga cacccgcggg gctcagtgcc 2587

cccgtcacag ctctgtagag acaatcaact gtgtcctgcc caccctccaa ggtgcattgt 2647

cttccagtat ctacagcttc agacttgagc taagtaaatg cttcaaagaa aaaaaaaaaa 2707

aaaaaad                                                           2714

<210> 2
<211> 780
<212> PRT
<213> Homo sapiens

<400> 2
Met Gly Lys Ser Glu Gly Pro Val Gly Met Val Glu Ser Ala Gly Arg
1               5                   10                  15

Ala Gly Gln Lys Arg Pro Gly Phe Leu Glu Gly Gly Leu Leu Leu Leu
            20                  25                  30

Leu Leu Leu Val Thr Ala Ala Leu Val Ala Leu Gly Val Leu Tyr Ala
        35                  40                  45

Asp Arg Arg Gly Lys Gln Leu Pro Arg Leu Ala Ser Arg Leu Cys Phe
    50                  55                  60

Leu Gln Glu Glu Arg Thr Phe Val Lys Arg Lys Pro Arg Gly Ilé Pro
65                  70                  75                  80

Glu Ala Gln Glu Val Ser Glu Val Cys Thr Thr Pro Gly Cys Val Ile
                85                  90                  95

Ala Ala Ala Arg Ile Leu Gln Asn Met Asp Pro Thr Thr Glu Pro Cys
            100                 105                 110

Asp Asp Phe Tyr Gln Phe Ala Cys Gly Gly Trp Leu Arg Arg His Val
        115                 120                 125

Ile Pro Glu Thr Asn Ser Arg Tyr Ser Ile Phe Asp Val Leu Arg Asp
    130                 135                 140

Glu Leu Glu Val Ile Leu Lys Ala Val Leu Glu Asn Ser Thr Ala Lys
145                 150                 155                 160

Asp Arg Pro Ala Val Glu Lys Ala Arg Thr Leu Tyr Arg Ser Cys Met
                165                 170                 175

Asn Gln Ser Val Ile Glu Lys Arg Gly Ser Gln Pro Leu Leu Asp Ile
            180                 185                 190

Leu Glu Val Val Gly Gly Trp Pro Val Ala Met Asp Arg Trp Asn Glu

195                 200                 205

Thr Val Gly Leu Glu Trp Glu Leu Glu Arg Gln Leu Ala Leu Met Asn
    210                 215                 220

Ser Gln Phe Asn Arg Arg Val Leu Ile Asp Leu Phe Ile Trp Asn Asp
225                 230                 235                 240

Asp Gln Asn Ser Ser Arg His Ile Ile Tyr Ile Asp Gln Pro Thr Leu
                245                 250                 255

Gly Met Pro Ser Arg Glu Tyr Tyr Phe Asn Gly Gly Ser Asn Arg Lys
            260                 265                 270

Val Arg Glu Ala Tyr Leu Gln Phe Met Val Ser Val Ala Thr Leu Leu
        275                 280                 285

Arg Glu Asp Ala Asn Leu Pro Arg Asp Ser Cys Leu Val Gln Glu Asp
    290                 295                 300

Met Val Gln Val Leu Glu Leu Glu Thr Gln Leu Ala Lys Ala Thr Val
305                 310                 315                 320

Pro Gln Glu Glu Arg His Asp Val Ile Ala Leu Tyr His Arg Met Gly
                325                 330                 335

Leu Glu Glu Leu Gln Ser Gln Phe Gly Leu Lys Gly Phe Asp Trp Thr
            340                 345                 350

Leu Phe Ile Gln Thr Val Leu Ser Ser Val Lys Ile Lys Leu Leu Pro
        355                 360                 365

Asp Glu Glu Val Val Val Tyr Gly Ile Pro Tyr Leu Gln Asn Leu Glu
    370                 375                 380

Asn Ile Ile Asp Thr Tyr Ser Ala Arg Thr Ile Gln Asn Tyr Leu Val
385                 390                 395                 400

Trp Arg Leu Val Leu Asp Arg Ile Gly Ser Leu Ser Gln Arg Phe Lys
                405                 410                 415

Asp Thr Arg Val Asn Tyr Arg Lys Ala Leu Phe Gly Thr Met Val Glu
            420                 425                 430

Glu Val Arg Trp Arg Glu Cys Val Gly Tyr Val Asn Ser Asn Met Glu
        435                 440                 445

Asn Ala Val Gly Ser Leu Tyr Val Arg Glu Ala Phe Pro Gly Asp Ser
    450                 455                 460

Lys Ser Met Val Arg Glu Leu Ile Asp Lys Val Arg Thr Val Phe Val
465                 470                 475                 480

Glu Thr Leu Asp Glu Leu Gly Trp Met Asp Glu Glu Ser Lys Lys Lys
                485                 490                 495

Ala Gln Glu Lys Ala Met Ser Ile Arg Glu Gln Ile Gly His Pro Asp
            500                 505                 510

Tyr Ile Leu Glu Glu Thr Asn Arg Arg Leu Asp Glu Glu Tyr Ser Asn
        515                 520                 525

Leu Asn Phe Ser Glu Asp Leu Tyr Phe Glu Asn Ser Leu Gln Asn Leu
    530                 535                 540

Lys Val Gly Ala Gln Arg Ser Leu Arg Lys Leu Arg Glu Lys Val Asp
545                 550                 555                 560

Pro Asn Leu Trp Ile Ile Gly Ala Ala Val Val Asn Ala Phe Tyr Ser
                565                 570                 575

Pro Asn Arg Asn Gln Ile Val Phe Pro Ala Gly Ile Leu Gln Pro Pro

580                 585                 590

Phe Phe Ser Lys Glu Gln Pro Gln Ala Leu Asn Phe Gly Gly Ile Gly
        595                 600                 605

Met Val Ile Gly His Glu Ile Thr His Gly Phe Asp Asp Asn Gly Arg
    610                 615                 620

Asn Phe Asp Lys Asn Gly Asn Met Met Asp Trp Trp Ser Asn Phe Ser
625                 630                 635                 640

Thr Gln His Phe Arg Glu Gln Ser Glu Cys Met Ile Tyr Gln Tyr Gly
                645                 650                 655

Asn Tyr Ser Trp Asp Leu Ala Asp Glu Gln Asn Val Asn Gly Phe Asn
            660                 665                 670

Thr Leu Gly Glu Asn Ile Ala Asp Asn Gly Gly Val Arg Gln Ala Tyr
        675                 680                 685

Lys Ala Tyr Leu Lys Trp Met Ala Glu Gly Gly Lys Asp Gln Gln Leu
    690                 695                 700

Pro Gly Leu Asp Leu Thr His Glu Gln Leu Phe Phe Ile Asn Tyr Ala
705                 710                 715                 720

Gln Val Trp Cys Gly Ser Tyr Arg Pro Glu Phe Ala Ile Gln Ser Ile
                725                 730                 735

Lys Thr Asp Val His Ser Pro Leu Lys Tyr Arg Val Leu Gly Ser Leu
            740                 745                 750

Gln Asn Leu Ala Ala Phe Ala Asp Thr Phe His Cys Ala Arg Gly Thr
        755                 760                 765

Pro Met His Pro Lys Glu Arg Cys Arg Val Trp
    770                 775                 780


<210> 3
<211> 2636
<212> DNA
<213> Homo sapiens

<220>
<221> 5'UTR
<222> (1)..(7)

<220>
<221> CDS
<222> (8)..(2269)

<220>
<221> 3'UTR
<222> (2270)..(2635)

<400> 3
cctgctg atg ggg aag tcc gaa ggc ccc gtg ggg atg gtg gag agc gct      49
        Met Gly Lys Ser Glu Gly Pro Val Gly Met Val Glu Ser Ala
        1               5                   10

ggc cgt gca ggg cag aag cgc ccg ggg ttc ctg gag ggg ggg ctg ctg      97
Gly Arg Ala Gly Gln Lys Arg Pro Gly Phe Leu Glu Gly Gly Leu Leu
15                  20                  25                  30

ctg ctg ctg ctg ctg gtg acc gct gcc ctg gtg gcc ttg ggt gtc ctc      145
Leu Leu Leu Leu Leu Val Thr Ala Ala Leu Val Ala Leu Gly Val Leu
                35                  40                  45

tac gcc gac cgc aga ggg atc cca gag gcc caa gag gtg agc gag gtc      193
Tyr Ala Asp Arg Arg Gly Ile Pro Glu Ala Gln Glu Val Ser Glu Val
            50                  55                  60

tgc acc acc cct ggc tgc gtg ata gca gct gcc agg atc ctc cag aac 241
Cys Thr Thr Pro Gly Cys Val Ile Ala Ala Ala Arg Ile Leu Gln Asn
        65                  70                  75

atg gac ccg acc acg gaa ccg tgt gac gac ttc tac cag ttt gca tgc 289
Met Asp Pro Thr Thr Glu Pro Cys Asp Asp Phe Tyr Gln Phe Ala Cys
    80                  85                  90

gga ggc tgg ctg cgg cgc cac gtg atc cct gag acc aac tca aga tac 337
Gly Gly Trp Leu Arg Arg His Val Ile Pro Glu Thr Asn Ser Arg Tyr
95                  100                 105                 110

agc atc ttt gac gtc ctc cgc gac gag ctg gag gtc atc ctc aaa gcg 385
Ser Ile Phe Asp Val Leu Arg Asp Glu Leu Glu Val Ile Leu Lys Ala
                115                 120                 125

gtg ctg gag aat tcg act gcc aag gac cgg ccg gct gtg gag aag gcc 433
Val Leu Glu Asn Ser Thr Ala Lys Asp Arg Pro Ala Val Glu Lys Ala
            130                 135                 140

agg acg ctg tac cgc tcc tgc atg aac cag agt gtg ata gag aag cga 481
Arg Thr Leu Tyr Arg Ser Cys Met Asn Gln Ser Val Ile Glu Lys Arg
        145                 150                 155

ggc tct cag ccc ctg ctg gac atc ttg gag gtg gtg gga ggc tgg ccg 529
Gly Ser Gln Pro Leu Leu Asp Ile Leu Glu Val Val Gly Gly Trp Pro
    160                 165                 170

gtg gcg atg gac agg tgg aac gag acc gta gga ctc gag tgg gag ctg 577
Val Ala Met Asp Arg Trp Asn Glu Thr Val Gly Leu Glu Trp Glu Leu
175                 180                 185                 190

gag cgg cag ctg gcg ctg atg aac tca cag ttc aac agg cgc gtc ctc 625
Glu Arg Gln Leu Ala Leu Met Asn Ser Gln Phe Asn Arg Arg Val Leu
                195                 200                 205

atc gac ctc ttc atc tgg aac gac gac cag aac tcc agc cgg cac atc 673
Ile Asp Leu Phe Ile Trp Asn Asp Asp Gln Asn Ser Ser Arg His Ile
            210                 215                 220

atc tac ata gac cag ccc acc ttg ggc atg ccc tcc cga gag tac tac 721
Ile Tyr Ile Asp Gln Pro Thr Leu Gly Met Pro Ser Arg Glu Tyr Tyr
        225                 230                 235

ttc aac ggc ggc agc aac cgg aag gtg cgg gaa gcc tac ctg cag ttc 769
Phe Asn Gly Gly Ser Asn Arg Lys Val Arg Glu Ala Tyr Leu Gln Phe
    240                 245                 250

atg gtg tca gtg gcc acg ttg ctg cgg gag gat gca aac ctg ccc agg 817
Met Val Ser Val Ala Thr Leu Leu Arg Glu Asp Ala Asn Leu Pro Arg
255                 260                 265                 270

gac agc tgc ctg gtg cag gag gac atg gtg cag gtg ctg gag ctg gag 865
Asp Ser Cys Leu Val Gln Glu Asp Met Val Gln Val Leu Glu Leu Glu
                275                 280                 285

aca cag ctg gcc aag gcc acg gta ccc cag gag gag aga cac gac gtc 913
Thr Gln Leu Ala Lys Ala Thr Val Pro Gln Glu Glu Arg His Asp Val
            290                 295                 300

atc gcc ttg tac cac cgg atg gga ctg gag gag ctg caa agc cag ttt 961
Ile Ala Leu Tyr His Arg Met Gly Leu Glu Glu Leu Gln Ser Gln Phe
        305                 310                 315

ggc ctg aag gga ttt gac tgg act ctg ttc ata caa act gtg cta tcc 1009
Gly Leu Lys Gly Phe Asp Trp Thr Leu Phe Ile Gln Thr Val Leu Ser
    320                 325                 330

tct gtc aaa atc aag ctg ctg cca gat gag gaa gtg gtg gtc tat ggc 1057
Ser Val Lys Ile Lys Leu Leu Pro Asp Glu Glu Val Val Val Tyr Gly
335                 340                 345                 350

atc ccc tac ctg cag aac ctt gaa aac atc atc gac acc tac tca gcc     1105
Ile Pro Tyr Leu Gln Asn Leu Glu Asn Ile Ile Asp Thr Tyr Ser Ala
                355                 360                 365

agg acc ata cag aac tac ctg gtc tgg cgc ctg gtg ctg gac cgc att     1153
Arg Thr Ile Gln Asn Tyr Leu Val Trp Arg Leu Val Leu Asp Arg Ile
            370                 375                 380

ggt agc cta agc cag aga ttc aag gac aca cga gtg aac tac cgc aag     1201
Gly Ser Leu Ser Gln Arg Phe Lys Asp Thr Arg Val Asn Tyr Arg Lys
        385                 390                 395

gcg ctg ttt ggc aca atg gtg gag gag gtg cgc tgg cgt gaa tgt gtg     1249
Ala Leu Phe Gly Thr Met Val Glu Glu Val Arg Trp Arg Glu Cys Val
    400                 405                 410

ggc tac gtc aac agc aac atg gag aac gcc gtg ggc tcc ctc tac gtc     1297
Gly Tyr Val Asn Ser Asn Met Glu Asn Ala Val Gly Ser Leu Tyr Val
415                 420                 425                 430

agg gag gcg ttc cct gga gac agc aag agc atg gtc aga gaa ctc att     1345
Arg Glu Ala Phe Pro Gly Asp Ser Lys Ser Met Val Arg Glu Leu Ile
                435                 440                 445

gac aag gtg cgg aca gtg ttt gtg gag acg ctg gac gag ctg ggc tgg     1393
Asp Lys Val Arg Thr Val Phe Val Glu Thr Leu Asp Glu Leu Gly Trp
            450                 455                 460

atg gac gag gag tcc aag aag aag gcg cag gag aag gcc atg agc atc     1441
Met Asp Glu Glu Ser Lys Lys Lys Ala Gln Glu Lys Ala Met Ser Ile
        465                 470                 475

cgg gag cag atc ggg cac cct gac tac atc ctg gag gag acg aac agg     1489
Arg Glu Gln Ile Gly His Pro Asp Tyr Ile Leu Glu Glu Thr Asn Arg
    480                 485                 490

cgc ctg gac gag gag tac tcc aat ctg aac ttc tca gag gac ctg tac     1537
Arg Leu Asp Glu Glu Tyr Ser Asn Leu Asn Phe Ser Glu Asp Leu Tyr
495                 500                 505                 510

ttt gag aac agt ctg cag aac ctc aag gtg ggc gcc cag cgg agc ctc     1585
Phe Glu Asn Ser Leu Gln Asn Leu Lys Val Gly Ala Gln Arg Ser Leu
                515                 520                 525

agg aag ctt cgg gaa aag gtg gac cca aat ctc tgg atc atc ggg gcg     1633
Arg Lys Leu Arg Glu Lys Val Asp Pro Asn Leu Trp Ile Ile Gly Ala
            530                 535                 540

gcg gtg gtc aat gcg ttc tac tcc cca aac cga aac cag att gta ttc     1681
Ala Val Val Asn Ala Phe Tyr Ser Pro Asn Arg Asn Gln Ile Val Phe
        545                 550                 555

cct gcc ggg atc ctc cag ccc ccc ttc ttc agc aag gag cag cca cag     1729
Pro Ala Gly Ile Leu Gln Pro Pro Phe Phe Ser Lys Glu Gln Pro Gln
    560                 565                 570

gcc ttg aac ttt gga ggc att ggg atg gtg atc ggg cac gag atc acg     1777
Ala Leu Asn Phe Gly Gly Ile Gly Met Val Ile Gly His Glu Ile Thr
575                 580                 585                 590

cac ggc ttt gac gac aat ggc cgg aac ttc gac aag aat ggc aac atg     1825
His Gly Phe Asp Asp Asn Gly Arg Asn Phe Asp Lys Asn Gly Asn Met
                595                 600                 605

atg gat tgg tgg agt aac ttc tcc acc cag cac ttc cgg gag cag tca     1873
Met Asp Trp Trp Ser Asn Phe Ser Thr Gln His Phe Arg Glu Gln Ser
            610                 615                 620

gag tgc atg atc tac cag tac ggc aac tac tcc tgg gac ctg gca gac     1921
Glu Cys Met Ile Tyr Gln Tyr Gly Asn Tyr Ser Trp Asp Leu Ala Asp
        625                 630                 635

gaa cag aac gtg aac gga ttc aac acc ctt ggg gaa aac att gct gac    1969
Glu Gln Asn Val Asn Gly Phe Asn Thr Leu Gly Glu Asn Ile Ala Asp
    640                 645                 650

aac gga ggg gtg cgg caa gcc tat aag gcc tac ctc aag tgg atg gca    2017
Asn Gly Gly Val Arg Gln Ala Tyr Lys Ala Tyr Leu Lys Trp Met Ala
655                 660                 665                 670

gag ggt ggc aag gac cag cag ctg ccc ggc ctg gat ctc acc cat gag    2065
Glu Gly Gly Lys Asp Gln Gln Leu Pro Gly Leu Asp Leu Thr His Glu
                675                 680                 685

cag ctc ttc ttc atc aac tat gcc cag gtg tgg tgc ggg tcc tac cgg    2113
Gln Leu Phe Phe Ile Asn Tyr Ala Gln Val Trp Cys Gly Ser Tyr Arg
            690                 695                 700

ccc gag ttc gcc atc caa tcc atc aag aca gac gtc cac agt ccc ctg    2161
Pro Glu Phe Ala Ile Gln Ser Ile Lys Thr Asp Val His Ser Pro Leu
        705                 710                 715

aag tac agg gta ctg ggg tcg ctg cag aac ctg gcc gcc ttc gca gac    2209
Lys Tyr Arg Val Leu Gly Ser Leu Gln Asn Leu Ala Ala Phe Ala Asp
    720                 725                 730

acg ttc cac tgt gcc cgg ggc acc ccc atg cac ccc aag gag cga tgc    2257
Thr Phe His Cys Ala Arg Gly Thr Pro Met His Pro Lys Glu Arg Cys
735                 740                 745                 750

cgc gtg tgg tag ccaaggccct gccgcgctgt gcggcccacg cccacctgct       2309
Arg Val Trp

gctcggaggc atctgtgcga aggtgcagct agcggcgacc cagtgtacgt cccgccccgg 2369

ccaaccatgc caagcctgcc tgccaggcct ctgcgcctgg cctagggtgc agccacctgc 2429

ctgacaccca gggatgagca gtgtccagtg cagtacctgg accggagccc cctccacaga 2489

cacccgcggg gctcagtgcc cccgtcacag ctctgtagag acaatcaact gtgtcctgcc 2549

caccctccaa ggtgcattgt cttccagtat ctacagcttc agacttgagc taagtaaatg 2609

cttcaaagaa aaaaaaaaaa aaaaaad                                     2636

<210> 4
<211> 754
<212> PRT
<213> Homo sapiens

<400> 4
Met Gly Lys Ser Glu Gly Pro Val Gly Met Val Glu Ser Ala Gly Arg
1               5                   10                  15

Ala Gly Gln Lys Arg Pro Gly Phe Leu Glu Gly Gly Leu Leu Leu Leu
            20                  25                  30

Leu Leu Leu Val Thr Ala Ala Leu Val Ala Leu Gly Val Leu Tyr Ala
        35                  40                  45

Asp Arg Arg Gly Ile Pro Glu Ala Gln Glu Val Ser Glu Val Cys Thr
    50                  55                  60

Thr Pro Gly Cys Val Ile Ala Ala Ala Arg Ile Leu Gln Asn Met Asp
65                  70                  75                  80

Pro Thr Thr Glu Pro Cys Asp Asp Phe Tyr Gln Phe Ala Cys Gly Gly
                85                  90                  95

Trp Leu Arg Arg His Val Ile Pro Glu Thr Asn Ser Arg Tyr Ser Ile
            100                 105                 110

Phe Asp Val Leu Arg Asp Glu Leu Glu Val Ile Leu Lys Ala Val Leu

115                 120                 125

Glu Asn Ser Thr Ala Lys Asp Arg Pro Ala Val Glu Lys Ala Arg Thr
    130                 135                 140

Leu Tyr Arg Ser Cys Met Asn Gln Ser Val Ile Glu Lys Arg Gly Ser
145                 150                 155                 160

Gln Pro Leu Leu Asp Ile Leu Glu Val Val Gly Gly Trp Pro Val Ala
                165                 170                 175

Met Asp Arg Trp Asn Glu Thr Val Gly Leu Glu Trp Glu Leu Glu Arg
            180                 185                 190

Gln Leu Ala Leu Met Asn Ser Gln Phe Asn Arg Arg Val Leu Ile Asp
        195                 200                 205

Leu Phe Ile Trp Asn Asp Asp Gln Asn Ser Ser Arg His Ile Ile Tyr
    210                 215                 220

Ile Asp Gln Pro Thr Leu Gly Met Pro Ser Arg Glu Tyr Tyr Phe Asn
225                 230                 235                 240

Gly Gly Ser Asn Arg Lys Val Arg Glu Ala Tyr Leu Gln Phe Met Val
                245                 250                 255

Ser Val Ala Thr Leu Leu Arg Glu Asp Ala Asn Leu Pro Arg Asp Ser
            260                 265                 270

Cys Leu Val Gln Glu Asp Met Val Gln Val Leu Glu Leu Glu Thr Gln
        275                 280                 285

Leu Ala Lys Ala Thr Val Pro Gln Glu Glu Arg His Asp Val Ile Ala
    290                 295                 300

Leu Tyr His Arg Met Gly Leu Glu Glu Leu Gln Ser Gln Phe Gly Leu
305                 310                 315                 320

Lys Gly Phe Asp Trp Thr Leu Phe Ile Gln Thr Val Leu Ser Ser Val
                325                 330                 335

Lys Ile Lys Leu Leu Pro Asp Glu Glu Val Val Val Tyr Gly Ile Pro
            340                 345                 350

Tyr Leu Gln Asn Leu Glu Asn Ile Ile Asp Thr Tyr Ser Ala Arg Thr
        355                 360                 365

Ile Gln Asn Tyr Leu Val Trp Arg Leu Val Leu Asp Arg Ile Gly Ser
    370                 375                 380

Leu Ser Gln Arg Phe Lys Asp Thr Arg Val Asn Tyr Arg Lys Ala Leu
385                 390                 395                 400

Phe Gly Thr Met Val Glu Glu Val Arg Trp Arg Glu Cys Val Gly Tyr
                405                 410                 415

Val Asn Ser Asn Met Glu Asn Ala Val Gly Ser Leu Tyr Val Arg Glu
            420                 425                 430

Ala Phe Pro Gly Asp Ser Lys Ser Met Val Arg Glu Leu Ile Asp Lys
        435                 440                 445

Val Arg Thr Val Phe Val Glu Thr Leu Asp Glu Leu Gly Trp Met Asp
    450                 455                 460

Glu Glu Ser Lys Lys Lys Ala Gln Glu Lys Ala Met Ser Ile Arg Glu
465                 470                 475                 480

Gln Ile Gly His Pro Asp Tyr Ile Leu Glu Glu Thr Asn Arg Arg Leu
                485                 490                 495

Asp Glu Glu Tyr Ser Asn Leu Asn Phe Ser Glu Asp Leu Tyr Phe Glu

500                 505                 510

Asn Ser Leu Gln Asn Leu Lys Val Gly Ala Gln Arg Ser Leu Arg Lys
        515                 520                 525

Leu Arg Glu Lys Val Asp Pro Asn Leu Trp Ile Ile Gly Ala Ala Val
    530                 535                 540

Val Asn Ala Phe Tyr Ser Pro Asn Arg Asn Gln Ile Val Phe Pro Ala
545                 550                 555                 560

Gly Ile Leu Gln Pro Pro Phe Phe Ser Lys Glu Gln Pro Gln Ala Leu
                565                 570                 575

Asn Phe Gly Gly Ile Gly Met Val Ile Gly His Glu Ile Thr His Gly
            580                 585                 590

Phe Asp Asp Asn Gly Arg Asn Phe Asp Lys Asn Gly Asn Met Met Asp
        595                 600                 605

Trp Trp Ser Asn Phe Ser Thr Gln His Phe Arg Glu Gln Ser Glu Cys
    610                 615                 620

Met Ile Tyr Gln Tyr Gly Asn Tyr Ser Trp Asp Leu Ala Asp Glu Gln
625                 630                 635                 640

Asn Val Asn Gly Phe Asn Thr Leu Gly Glu Asn Ile Ala Asp Asn Gly
                645                 650                 655

Gly Val Arg Gln Ala Tyr Lys Ala Tyr Leu Lys Trp Met Ala Glu Gly
            660                 665                 670

Gly Lys Asp Gln Gln Leu Pro Gly Leu Asp Leu Thr His Glu Gln Leu
        675                 680                 685

Phe Phe Ile Asn Tyr Ala Gln Val Trp Cys Gly Ser Tyr Arg Pro Glu
    690                 695                 700

Phe Ala Ile Gln Ser Ile Lys Thr Asp Val His Ser Pro Leu Lys Tyr
705                 710                 715                 720

Arg Val Leu Gly Ser Leu Gln Asn Leu Ala Ala Phe Ala Asp Thr Phe
                725                 730                 735

His Cys Ala Arg Gly Thr Pro Met His Pro Lys Glu Arg Cys Arg Val
            740                 745                 750

Trp

<210> 5
<211> 2663
<212> DNA
<213> Homo sapiens

<220>
<221> 5'UTR
<222> (1)..(7)

<220>
<221> CDS
<222> (8)..(2293)

<220>
<221> 3'UTR
<222> (2294)..(2663)

<400> 5
cctgctg atg ggg aag tcc gaa ggc ccc gtg ggg atg gtg gag agc gct      49
        Met Gly Lys Ser Glu Gly Pro Val Gly Met Val Glu Ser Ala
        1               5                   10

ggc cgt gca ggg cag aag cgc ccg ggg ttc ctg gag ggg ggc ctg ctg    97
Gly Arg Ala Gly Gln Lys Arg Pro Gly Phe Leu Glu Gly Gly Leu Leu
 15                  20                  25                  30

ctg ctg ctg ctg ctg gtg acc gct gcc ctg gtg gcc ttg ggt gtc ctc    145
Leu Leu Leu Leu Leu Val Thr Ala Ala Leu Val Ala Leu Gly Val Leu
                 35                  40                  45

tac gcc gac cgc aga ggg aag cag ctg cca cgc ctt gct agc cgg ctg    193
Tyr Ala Asp Arg Arg Gly Lys Gln Leu Pro Arg Leu Ala Ser Arg Leu
             50                  55                  60

tgc ttc tta cag gag gag agg acc ttt gta aaa cga aaa ccc cga ggg    241
Cys Phe Leu Gln Glu Glu Arg Thr Phe Val Lys Arg Lys Pro Arg Gly
         65                  70                  75

atc cca gag gcc caa gag gtg agc gag gtc tgc acc acc cct ggc tgc    289
Ile Pro Glu Ala Gln Glu Val Ser Glu Val Cys Thr Thr Pro Gly Cys
     80                  85                  90

gtg ata gca gct gcc agg atc ctc cag aac atg gac ccg acc acg gaa    337
Val Ile Ala Ala Ala Arg Ile Leu Gln Asn Met Asp Pro Thr Thr Glu
 95                 100                 105                 110

ccg tgt gac gac ttc tac cag ttt gca tgc gga ggc tgg ctg cgg cgc    385
Pro Cys Asp Asp Phe Tyr Gln Phe Ala Cys Gly Gly Trp Leu Arg Arg
                115                 120                 125

cac gtg atc cct gag acc aac tca aga tac agc atc ttt gac gtc ctc    433
His Val Ile Pro Glu Thr Asn Ser Arg Tyr Ser Ile Phe Asp Val Leu
            130                 135                 140

cgc gac gag ctg gag gtc atc ctc aaa gcg gtg ctg gag aat tcg act    481
Arg Asp Glu Leu Glu Val Ile Leu Lys Ala Val Leu Glu Asn Ser Thr
        145                 150                 155

gcc aag gac cgg ccg gct gtg gag aag gcc agg acg ctg tac cgc tcc    529
Ala Lys Asp Arg Pro Ala Val Glu Lys Ala Arg Thr Leu Tyr Arg Ser
    160                 165                 170

tgc atg aac cag agt gtg ata gag aag cga ggc tct cag ccc ctg ctg    577
Cys Met Asn Gln Ser Val Ile Glu Lys Arg Gly Ser Gln Pro Leu Leu
175                 180                 185                 190

gac atc ttg gag gtg gtg gga ggc tgg ccg gtg gcg atg gac agg tgg    625
Asp Ile Leu Glu Val Val Gly Gly Trp Pro Val Ala Met Asp Arg Trp
                195                 200                 205

aac gag acc gta gtt caa cag gcg cgt cct cat cga cct ctt cat ctg    673
Asn Glu Thr Val Val Gln Gln Ala Arg Pro His Arg Pro Leu His Leu
            210                 215                 220

gaa cga cga cca gaa ctc cag ccg gca cat cat cta cac cag ccc acc    721
Glu Arg Arg Pro Glu Leu Gln Pro Ala His His Leu His Gln Pro Thr
        225                 230                 235

ttg ggc atg ccc tcc cga gag tac tac ttc aac ggc ggc agc aac cgg    769
Leu Gly Met Pro Ser Arg Glu Tyr Tyr Phe Asn Gly Gly Ser Asn Arg
    240                 245                 250

aag gtg cgg gaa gcc tac ctg cag ttc atg gtg tca gtg gcc acg ttg    817
Lys Val Arg Glu Ala Tyr Leu Gln Phe Met Val Ser Val Ala Thr Leu
255                 260                 265                 270

ctg cgg gag gat gca aac ctg ccc agg gac agc tgc ctg gtg cag gag    865
Leu Arg Glu Asp Ala Asn Leu Pro Arg Asp Ser Cys Leu Val Gln Glu
                275                 280                 285

gac atg gtg cag gtg ctg gag ctg gag aca cag ctg gcc aag gcc acg    913
Asp Met Val Gln Val Leu Glu Leu Glu Thr Gln Leu Ala Lys Ala Thr
            290                 295                 300

gta ccc cag gag gag aga cac gac gtc atc gcc ttg tac cac cgg atg    961
Val Pro Gln Glu Glu Arg His Asp Val Ile Ala Leu Tyr His Arg Met
        305                 310                 315

gga ctg gag gag ctg caa agc cag ttt ggc ctg aag gga ttt gac tgg    1009
Gly Leu Glu Glu Leu Gln Ser Gln Phe Gly Leu Lys Gly Phe Asp Trp
    320                 325                 330

act ctg ttc ata caa act gtg cta tcc tct gtc aaa atc aag ctg ctg    1057
Thr Leu Phe Ile Gln Thr Val Leu Ser Ser Val Lys Ile Lys Leu Leu
335                 340                 345                 350

cca gat gag gaa gtg gtg gtc tat ggc atc ccc tac ctg cag aac ctt    1105
Pro Asp Glu Glu Val Val Val Tyr Gly Ile Pro Tyr Leu Gln Asn Leu
                355                 360                 365

gaa aac atc atc gac acc tac tca gcc agg acc ata cag aac tac ctg    1153
Glu Asn Ile Ile Asp Thr Tyr Ser Ala Arg Thr Ile Gln Asn Tyr Leu
            370                 375                 380

gtc tgg cgc ctg gtg ctg gac cgc att ggt agc cta agc cag aga ttc    1201
Val Trp Arg Leu Val Leu Asp Arg Ile Gly Ser Leu Ser Gln Arg Phe
        385                 390                 395

aag gac aca cga gtg aac tac cgc aag gcg ctg ttt ggc aca atg gtg    1249
Lys Asp Thr Arg Val Asn Tyr Arg Lys Ala Leu Phe Gly Thr Met Val
    400                 405                 410

gag gag gtg cgc tgg cgt gaa tgt gtg ggc tac gtc aac agc aac atg    1297
Glu Glu Val Arg Trp Arg Glu Cys Val Gly Tyr Val Asn Ser Asn Met
415                 420                 425                 430

gag aac gcc gtg ggc tcc ctc tac gtc agg gag gcg ttc cct gga gac    1345
Glu Asn Ala Val Gly Ser Leu Tyr Val Arg Glu Ala Phe Pro Gly Asp
                435                 440                 445

agc aag agc atg gtc aga gaa ctc att gac aag gtg cgg aca gtg ttt    1393
Ser Lys Ser Met Val Arg Glu Leu Ile Asp Lys Val Arg Thr Val Phe
            450                 455                 460

gtg gag acg ctg gac gag ctg ggc tgg atg gac gag gag tcc aag aag    1441
Val Glu Thr Leu Asp Glu Leu Gly Trp Met Asp Glu Glu Ser Lys Lys
        465                 470                 475

aag gcg cag gag aag gcc atg agc atc cgg gag cag atc ggg cac cct    1489
Lys Ala Gln Glu Lys Ala Met Ser Ile Arg Glu Gln Ile Gly His Pro
    480                 485                 490

gac tac atc ctg gag gag acg aac agg cgc ctg gac gag gag tac tcc    1537
Asp Tyr Ile Leu Glu Glu Thr Asn Arg Arg Leu Asp Glu Glu Tyr Ser
495                 500                 505                 510

aat ctg aac ttc tca gag gac ctg tac ttt gag aac agt ctg cag aac    1585
Asn Leu Asn Phe Ser Glu Asp Leu Tyr Phe Glu Asn Ser Leu Gln Asn
                515                 520                 525

ctc aag gtg ggc gcc cag cgg agc ctc agg aag ctt cgg gaa aag gtg    1633
Leu Lys Val Gly Ala Gln Arg Ser Leu Arg Lys Leu Arg Glu Lys Val
            530                 535                 540

gac cca aat ctc tgg atc atc ggg gcg gcg gtg gtc aat gcg ttc tac    1681
Asp Pro Asn Leu Trp Ile Ile Gly Ala Ala Val Val Asn Ala Phe Tyr
        545                 550                 555

tcc cca aac cga aac cag att gta ttc cct gcc ggg atc ctc cag ccc    1729
Ser Pro Asn Arg Asn Gln Ile Val Phe Pro Ala Gly Ile Leu Gln Pro
    560                 565                 570

ccc ttc ttc agc aag gag cag cca cag gcc ttg aac ttt gga ggc att    1777
Pro Phe Phe Ser Lys Glu Gln Pro Gln Ala Leu Asn Phe Gly Gly Ile
575                 580                 585                 590

ggg atg gtg atc ggg cac gag atc acg cac ggc ttt gac gac aat ggc    1825
Gly Met Val Ile Gly His Glu Ile Thr His Gly Phe Asp Asp Asn Gly
                595                 600                 605

cgg aac ttc gac aag aat ggc aac atg atg gat tgg tgg agt aac ttc    1873
Arg Asn Phe Asp Lys Asn Gly Asn Met Met Asp Trp Trp Ser Asn Phe
            610                 615                 620

tcc acc cag cac ttc cgg gag cag tca gag tgc atg atc tac cag tac    1921
Ser Thr Gln His Phe Arg Glu Gln Ser Glu Cys Met Ile Tyr Gln Tyr
        625                 630                 635

ggc aac tac tcc tgg gac ctg gca gac gaa cag aac gtg aac gga ttc    1969
Gly Asn Tyr Ser Trp Asp Leu Ala Asp Glu Gln Asn Val Asn Gly Phe
    640                 645                 650

aac acc ctt ggg gaa aac att gct gac aac gga ggg gtg cgg caa gcc    2017
Asn Thr Leu Gly Glu Asn Ile Ala Asp Asn Gly Gly Val Arg Gln Ala
655                 660                 665                 670

tat aag gcc tac ctc aag tgg atg gca gag ggt ggc aag gac cag cag    2065
Tyr Lys Ala Tyr Leu Lys Trp Met Ala Glu Gly Gly Lys Asp Gln Gln
                675                 680                 685

ctg ccc ggc ctg gat ctc acc cat gag cag ctc ttc ttc atc aac tat    2113
Leu Pro Gly Leu Asp Leu Thr His Glu Gln Leu Phe Phe Ile Asn Tyr
            690                 695                 700

gcc cag gtg tgg tgc ggg tcc tac cgg ccc gag ttc gcc atc caa tcc    2161
Ala Gln Val Trp Cys Gly Ser Tyr Arg Pro Glu Phe Ala Ile Gln Ser
        705                 710                 715

atc aag aca gac gtc cac agt ccc ctg aag tac agg gta ctg ggg tcg    2209
Ile Lys Thr Asp Val His Ser Pro Leu Lys Tyr Arg Val Leu Gly Ser
    720                 725                 730

ctg cag aac ctg gcc gcc ttc gca gac acg ttc cac tgt gcc cgg ggc    2257
Leu Gln Asn Leu Ala Ala Phe Ala Asp Thr Phe His Cys Ala Arg Gly
735                 740                 745                 750

acc ccc atg cac ccc aag gag cga tgc cgc gtg tgg tagccaaggc         2303
Thr Pro Met His Pro Lys Glu Arg Cys Arg Val Trp
                755                 760

cctgccgcgc tgtgcggccc acgcccacct gctgctcgga ggcatctgtg cgaaggtgca 2363

gctagcggcg acccagtgta cgtcccgccc cggccaacca tgccaagcct gcctgccagg 2423

cctctgcgcc tggcctaggg tgcagccacc tgcctgacac ccagggatga gcagtgtcca 2483

gtgcagtacc tggaccggag ccccctccac agacacccgc ggggctcagt gcccccgtca 2543

cagctctgta gagacaatca actgtgtcct gcccaccctc caaggtgcat tgtcttccag 2603

tatctacagc ttcagacttg agctaagtaa atgcttcaaa gaaaaaaaaa aaaaaaaaad 2663

<210> 6
<211> 762
<212> PRT
<213> Homo sapiens

<400> 6
Met Gly Lys Ser Glu Gly Pro Val Gly Met Val Glu Ser Ala Gly Arg
  1               5                  10                  15

Ala Gly Gln Lys Arg Pro Gly Phe Leu Glu Gly Gly Leu Leu Leu Leu
             20                  25                  30

Leu Leu Leu Val Thr Ala Ala Leu Val Ala Leu Gly Val Leu Tyr Ala
         35                  40                  45

Asp Arg Arg Gly Lys Gln Leu Pro Arg Leu Ala Ser Arg Leu Cys Phe
    50                  55                  60

Leu Gln Glu Glu Arg Thr Phe Val Lys Arg Lys Pro Arg Gly Ile Pro
65                  70                  75                  80

Glu Ala Gln Glu Val Ser Glu Val Cys Thr Thr Pro Gly Cys Val Ile
                85                  90                  95

Ala Ala Ala Arg Ile Leu Gln Asn Met Asp Pro Thr Thr Glu Pro Cys
            100                 105                 110

Asp Asp Phe Tyr Gln Phe Ala Cys Gly Gly Trp Leu Arg Arg His Val
        115                 120                 125

Ile Pro Glu Thr Asn Ser Arg Tyr Ser Ile Phe Asp Val Leu Arg Asp
    130                 135                 140

Glu Leu Glu Val Ile Leu Lys Ala Val Leu Glu Asn Ser Thr Ala Lys
145                 150                 155                 160

Asp Arg Pro Ala Val Glu Lys Ala Arg Thr Leu Tyr Arg Ser Cys Met
                165                 170                 175

Asn Gln Ser Val Ile Glu Lys Arg Gly Ser Gln Pro Leu Leu Asp Ile
            180                 185                 190

Leu Glu Val Val Gly Gly Trp Pro Val Ala Met Asp Arg Trp Asn Glu
        195                 200                 205

Thr Val Val Gln Gln Ala Arg Pro His Arg Pro Leu His Leu Glu Arg
    210                 215                 220

Arg Pro Glu Leu Gln Pro Ala His His Leu His Gln Pro Thr Leu Gly
225                 230                 235                 240

Met Pro Ser Arg Glu Tyr Tyr Phe Asn Gly Gly Ser Asn Arg Lys Val
                245                 250                 255

Arg Glu Ala Tyr Leu Gln Phe Met Val Ser Val Ala Thr Leu Leu Arg
            260                 265                 270

Glu Asp Ala Asn Leu Pro Arg Asp Ser Cys Leu Val Gln Glu Asp Met
        275                 280                 285

Val Gln Val Leu Glu Leu Glu Thr Gln Leu Ala Lys Ala Thr Val Pro
    290                 295                 300

Gln Glu Glu Arg His Asp Val Ile Ala Leu Tyr His Arg Met Gly Leu
305                 310                 315                 320

Glu Glu Leu Gln Ser Gln Phe Gly Leu Lys Gly Phe Asp Trp Thr Leu
                325                 330                 335

Phe Ile Gln Thr Val Leu Ser Ser Val Lys Ile Lys Leu Leu Pro Asp
            340                 345                 350

Glu Glu Val Val Val Tyr Gly Ile Pro Tyr Leu Gln Asn Leu Glu Asn
        355                 360                 365

Ile Ile Asp Thr Tyr Ser Ala Arg Thr Ile Gln Asn Tyr Leu Val Trp
    370                 375                 380

Arg Leu Val Leu Asp Arg Ile Gly Ser Leu Ser Gln Arg Phe Lys Asp
385                 390                 395                 400

Thr Arg Val Asn Tyr Arg Lys Ala Leu Phe Gly Thr Met Val Glu Glu
                405                 410                 415

Val Arg Trp Arg Glu Cys Val Gly Tyr Val Asn Ser Asn Met Glu Asn
            420                 425                 430

Ala Val Gly Ser Leu Tyr Val Arg Glu Ala Phe Pro Gly Asp Ser Lys
        435                 440                 445

Ser Met Val Arg Glu Leu Ile Asp Lys Val Arg Thr Val Phe Val Glu
    450                 455                 460

Thr Leu Asp Glu Leu Gly Trp Met Asp Glu Glu Ser Lys Lys Lys Ala
465                 470                 475                 480

Gln Glu Lys Ala Met Ser Ile Arg Glu Gln Ile Gly His Pro Asp Tyr
                485                 490                 495

Ile Leu Glu Glu Thr Asn Arg Arg Leu Asp Glu Glu Tyr Ser Asn Leu
            500                 505                 510

Asn Phe Ser Glu Asp Leu Tyr Phe Glu Asn Ser Leu Gln Asn Leu Lys
        515                 520                 525

Val Gly Ala Gln Arg Ser Leu Arg Lys Leu Arg Glu Lys Val Asp Pro
    530                 535                 540

Asn Leu Trp Ile Ile Gly Ala Ala Val Val Asn Ala Phe Tyr Ser Pro
545                 550                 555                 560

Asn Arg Asn Gln Ile Val Phe Pro Ala Gly Ile Leu Gln Pro Pro Phe
                565                 570                 575

Phe Ser Lys Glu Gln Pro Gln Ala Leu Asn Phe Gly Gly Ile Gly Met
            580                 585                 590

Val Ile Gly His Glu Ile Thr His Gly Phe Asp Asp Asn Gly Arg Asn
        595                 600                 605

Phe Asp Lys Asn Gly Asn Met Met Asp Trp Trp Ser Asn Phe Ser Thr
    610                 615                 620

Gln His Phe Arg Glu Gln Ser Glu Cys Met Ile Tyr Gln Tyr Gly Asn
625                 630                 635                 640

Tyr Ser Trp Asp Leu Ala Asp Glu Gln Asn Val Asn Gly Phe Asn Thr
                645                 650                 655

Leu Gly Glu Asn Ile Ala Asp Asn Gly Gly Val Arg Gln Ala Tyr Lys
            660                 665                 670

Ala Tyr Leu Lys Trp Met Ala Glu Gly Gly Lys Asp Gln Gln Leu Pro
        675                 680                 685

Gly Leu Asp Leu Thr His Glu Gln Leu Phe Phe Ile Asn Tyr Ala Gln
    690                 695                 700

Val Trp Cys Gly Ser Tyr Arg Pro Glu Phe Ala Ile Gln Ser Ile Lys
705                 710                 715                 720

Thr Asp Val His Ser Pro Leu Lys Tyr Arg Val Leu Gly Ser Leu Gln
                725                 730                 735

Asn Leu Ala Ala Phe Ala Asp Thr Phe His Cys Ala Arg Gly Thr Pro
            740                 745                 750

Met His Pro Lys Glu Arg Cys Arg Val Trp
        755                 760

### Annex to the application documents - subsequently filed sequences listing

SEQUENCE LISTING

<110> SANOFI-SYNTHELABO

<120> THREE MEMBRANE METALLOPEPTIDASES, THEIR ISOLATION AND
      USE

<130> snep

<160> 6

<170> PatentIn Ver. 2.1

<210> 1
<211> 2714
<212> DNA
<213> Homo sapiens

<220>
<221> CDS
<222> (8)..(2347)

<220>
<221> 3'UTR
<222> (2348)..(2713)

<220>
<221> 5'UTR
<222> (1)..(7)

<400> 1
cctgctg atg ggg aag tcc gaa ggc ccc gtg ggg atg gtg gag agc gct    49
        Met Gly Lys Ser Glu Gly Pro Val Gly Met Val Glu Ser Ala
          1               5                  10

ggc cgt gca ggg cag aag cgc ccg ggg ttc ctg gag ggg ggg ctg ctg    97
Gly Arg Ala Gly Gln Lys Arg Pro Gly Phe Leu Glu Gly Gly Leu Leu
 15                  20                  25                  30

ctg ctg ctg ctg ctg gtg acc gct gcc ctg gtg gcc ttg ggt gtc ctc    145
Leu Leu Leu Leu Leu Val Thr Ala Ala Leu Val Ala Leu Gly Val Leu
                 35                  40                  45

tac gcc gac cgc aga ggg aag cag ctg cca cgc ctt gct agc cgg ctg    193
Tyr Ala Asp Arg Arg Gly Lys Gln Leu Pro Arg Leu Ala Ser Arg Leu
             50                  55                  60

tgc ttc tta cag gag gag agg acc ttt gta aaa cga aaa ccc cga ggg    241
Cys Phe Leu Gln Glu Glu Arg Thr Phe Val Lys Arg Lys Pro Arg Gly
         65                  70                  75

atc cca gag gcc caa gag gtg agc gag gtc tgc acc acc cct ggc tgc    289
Ile Pro Glu Ala Gln Glu Val Ser Glu Val Cys Thr Thr Pro Gly Cys
     80                  85                  90

gtg ata gca gct gcc agg atc ctc cag aac atg gac ccg acc acg gaa    337
Val Ile Ala Ala Ala Arg Ile Leu Gln Asn Met Asp Pro Thr Thr Glu
 95                 100                 105                 110

ccg tgt gac gac ttc tac cag ttt gca tgc gga ggc tgg ctg cgg cgc    385
Pro Cys Asp Asp Phe Tyr Gln Phe Ala Cys Gly Gly Trp Leu Arg Arg
                115                 120                 125

cac gtg atc cct gag acc aac tca aga tac agc atc ttt gac gtc ctc 433
His Val Ile Pro Glu Thr Asn Ser Arg Tyr Ser Ile Phe Asp Val Leu
            130                 135                 140

cgc gac gag ctg gag gtc atc ctc aaa gcg gtg ctg gag aat tcg act 481
Arg Asp Glu Leu Glu Val Ile Leu Lys Ala Val Leu Glu Asn Ser Thr
        145                 150                 155

gcc aag gac cgg ccg gct gtg gag aag gcc agg acg ctg tac cgc tcc 529
Ala Lys Asp Arg Pro Ala Val Glu Lys Ala Arg Thr Leu Tyr Arg Ser
    160                 165                 170

tgc atg aac cag agt gtg ata gag aag cga ggc tct cag ccc ctg ctg 577
Cys Met Asn Gln Ser Val Ile Glu Lys Arg Gly Ser Gln Pro Leu Leu
175                 180                 185                 190

gac atc ttg gag gtg gtg gga ggc tgg ccg gtg gcg atg gac agg tgg 625
Asp Ile Leu Glu Val Val Gly Gly Trp Pro Val Ala Met Asp Arg Trp
                195                 200                 205

aac gag acc gta gga ctc gag tgg gag ctg gag cgg cag ctg gcg ctg 673
Asn Glu Thr Val Gly Leu Glu Trp Glu Leu Glu Arg Gln Leu Ala Leu
            210                 215                 220

atg aac tca cag ttc aac agg cgc gtc ctc atc gac ctc ttc atc tgg 721
Met Asn Ser Gln Phe Asn Arg Arg Val Leu Ile Asp Leu Phe Ile Trp
        225                 230                 235

aac gac gac cag aac tcc agc cgg cac atc atc tac ata gac cag ccc 769
Asn Asp Asp Gln Asn Ser Ser Arg His Ile Ile Tyr Ile Asp Gln Pro
    240                 245                 250

acc ttg ggc atg ccc tcc cga gag tac tac ttc aac ggc ggc agc aac 817
Thr Leu Gly Met Pro Ser Arg Glu Tyr Tyr Phe Asn Gly Gly Ser Asn
255                 260                 265                 270

cgg aag gtg cgg gaa gcc tac ctg cag ttc atg gtg tca gtg gcc acg 865
Arg Lys Val Arg Glu Ala Tyr Leu Gln Phe Met Val Ser Val Ala Thr
                275                 280                 285

ttg ctg cgg gag gat gca aac ctg ccc agg gac agc tgc ctg gtg cag 913
Leu Leu Arg Glu Asp Ala Asn Leu Pro Arg Asp Ser Cys Leu Val Gln
            290                 295                 300

gag gac atg gtg cag gtg ctg gag ctg gag aca cag ctg gcc aag gcc 961
Glu Asp Met Val Gln Val Leu Glu Leu Glu Thr Gln Leu Ala Lys Ala
        305                 310                 315

acg gta ccc cag gag gag aga cac gac gtc atc gcc ttg tac cac cgg 1009
Thr Val Pro Gln Glu Glu Arg His Asp Val Ile Ala Leu Tyr His Arg
    320                 325                 330

atg gga ctg gag gag ctg caa agc cag ttt ggc ctg aag gga ttt gac 1057
Met Gly Leu Glu Glu Leu Gln Ser Gln Phe Gly Leu Lys Gly Phe Asp
335                 340                 345                 350

tgg act ctg ttc ata caa act gtg cta tcc tct gtc aaa atc aag ctg 1105
Trp Thr Leu Phe Ile Gln Thr Val Leu Ser Ser Val Lys Ile Lys Leu
                355                 360                 365

ctg cca gat gag gaa gtg gtg gtc tat ggc atc ccc tac ctg cag aac 1153
Leu Pro Asp Glu Glu Val Val Val Tyr Gly Ile Pro Tyr Leu Gln Asn
            370                 375                 380

ctt gaa aac atc atc gac acc tac tca gcc agg acc ata cag aac tac    1201
Leu Glu Asn Ile Ile Asp Thr Tyr Ser Ala Arg Thr Ile Gln Asn Tyr
            385                 390                 395

ctg gtc tgg cgc ctg gtg ctg gac cgc att ggt agc cta agc cag aga    1249
Leu Val Trp Arg Leu Val Leu Asp Arg Ile Gly Ser Leu Ser Gln Arg
    400                 405                 410

ttc aag gac aca cga gtg aac tac cgc aag gcg ctg ttt ggc aca atg    1297
Phe Lys Asp Thr Arg Val Asn Tyr Arg Lys Ala Leu Phe Gly Thr Met
415                 420                 425                 430

gtg gag gag gtg cgc tgg cgt gaa tgt gtg ggc tac gtc aac agc aac    1345
Val Glu Glu Val Arg Trp Arg Glu Cys Val Gly Tyr Val Asn Ser Asn
                435                 440                 445

atg gag aac gcc gtg ggc tcc ctc tac gtc agg gag gcg ttc cct gga    1393
Met Glu Asn Ala Val Gly Ser Leu Tyr Val Arg Glu Ala Phe Pro Gly
            450                 455                 460

gac agc aag agc atg gtc aga gaa ctc att gac aag gtg cgg aca gtg    1441
Asp Ser Lys Ser Met Val Arg Glu Leu Ile Asp Lys Val Arg Thr Val
        465                 470                 475

ttt gtg gag acg ctg gac gag ctg ggc tgg atg gac gag gag tcc aag    1489
Phe Val Glu Thr Leu Asp Glu Leu Gly Trp Met Asp Glu Glu Ser Lys
    480                 485                 490

aag aag gcg cag gag aag gcc atg agc atc cgg gag cag atc ggg cac    1537
Lys Lys Ala Gln Glu Lys Ala Met Ser Ile Arg Glu Gln Ile Gly His
495                 500                 505                 510

cct gac tac atc ctg gag gag acg aac agg cgc ctg gac gag gag tac    1585
Pro Asp Tyr Ile Leu Glu Glu Thr Asn Arg Arg Leu Asp Glu Glu Tyr
                515                 520                 525

tcc aat ctg aac ttc tca gag gac ctg tac ttt gag aac agt ctg cag    1633
Ser Asn Leu Asn Phe Ser Glu Asp Leu Tyr Phe Glu Asn Ser Leu Gln
            530                 535                 540

aac ctc aag gtg ggc gcc cag cgg agc ctc agg aag ctt cgg gaa aag    1681
Asn Leu Lys Val Gly Ala Gln Arg Ser Leu Arg Lys Leu Arg Glu Lys
        545                 550                 555

gtg gac cca aat ctc tgg atc atc ggg gcg gcg gtg gtc aat gcg ttc    1729
Val Asp Pro Asn Leu Trp Ile Ile Gly Ala Ala Val Val Asn Ala Phe
    560                 565                 570

tac tcc cca aac cga aac cag att gta ttc cct gcc ggg atc ctc cag    1777
Tyr Ser Pro Asn Arg Asn Gln Ile Val Phe Pro Ala Gly Ile Leu Gln
575                 580                 585                 590

ccc ccc ttc ttc agc aag gag cag cca cag gcc ttg aac ttt gga ggc    1825
Pro Pro Phe Phe Ser Lys Glu Gln Pro Gln Ala Leu Asn Phe Gly Gly
                595                 600                 605

att ggg atg gtg atc ggg cac gag atc acg cac ggc ttt gac gac aat    1873
Ile Gly Met Val Ile Gly His Glu Ile Thr His Gly Phe Asp Asp Asn
            610                 615                 620

ggc cgg aac ttc gac aag aat ggc aac atg atg gat tgg tgg agt aac    1921
Gly Arg Asn Phe Asp Lys Asn Gly Asn Met Met Asp Trp Trp Ser Asn
        625                 630                 635

ttc tcc acc cag cac ttc cgg gag cag tca gag tgc atg atc tac cag    1969
Phe Ser Thr Gln His Phe Arg Glu Gln Ser Glu Cys Met Ile Tyr Gln
    640                 645                 650

tac ggc aac tac tcc tgg gac ctg gca gac gaa cag aac gtg aac gga    2017
Tyr Gly Asn Tyr Ser Trp Asp Leu Ala Asp Glu Gln Asn Val Asn Gly
655                 660                 665                 670

ttc aac acc ctt ggg gaa aac att gct gac aac gga ggg gtg cgg caa    2065
Phe Asn Thr Leu Gly Glu Asn Ile Ala Asp Asn Gly Gly Val Arg Gln
                675                 680                 685

gcc tat aag gcc tac ctc aag tgg atg gca gag ggt ggc aag gac cag    2113
Ala Tyr Lys Ala Tyr Leu Lys Trp Met Ala Glu Gly Gly Lys Asp Gln
            690                 695                 700

cag ctg ccc ggc ctg gat ctc acc cat gag cag ctc ttc ttc atc aac    2161
Gln Leu Pro Gly Leu Asp Leu Thr His Glu Gln Leu Phe Phe Ile Asn
        705                 710                 715

tat gcc cag gtg tgg tgc ggg tcc tac cgg ccc gag ttc gcc atc caa    2209
Tyr Ala Gln Val Trp Cys Gly Ser Tyr Arg Pro Glu Phe Ala Ile Gln
    720                 725                 730

tcc atc aag aca gac gtc cac agt ccc ctg aag tac agg gta ctg ggg    2257
Ser Ile Lys Thr Asp Val His Ser Pro Leu Lys Tyr Arg Val Leu Gly
735                 740                 745                 750

tcg ctg cag aac ctg gcc gcc ttc gca gac acg ttc cac tgt gcc cgg    2305
Ser Leu Gln Asn Leu Ala Ala Phe Ala Asp Thr Phe His Cys Ala Arg
                755                 760                 765

ggc acc ccc atg cac ccc aag gag cga tgc cgc gtg tgg tag            2347
Gly Thr Pro Met His Pro Lys Glu Arg Cys Arg Val Trp
            770                 775                 780

ccaaggccct gccgcgctgt gcggcccacg cccacctgct gctcggaggc atctgtgcga 2407

aggtgcagct agcggcgacc cagtgtacgt cccgccccgg ccaaccatgc caagcctgcc 2467

tgccaggcct ctgcgcctgg cctagggtgc agccacctgc ctgacaccca gggatgagca 2527

gtgtccagtg cagtacctgg accggagccc cctccacaga cacccgcggg gctcagtgcc 2587

cccgtcacag ctctgtagag acaatcaact gtgtcctgcc caccctccaa ggtgcattgt 2647

cttccagtat ctacagcttc agacttgagc taagtaaatg cttcaaagaa aaaaaaaaaa 2707

aaaaaad                                                           2714

<210> 2
<211> 779
<212> PRT
<213> Homo sapiens

<400> 2
Met Gly Lys Ser Glu Gly Pro Val Gly Met Val Glu Ser Ala Gly Arg
  1               5                  10                  15

Ala Gly Gln Lys Arg Pro Gly Phe Leu Glu Gly Gly Leu Leu Leu Leu
             20                  25                  30

Leu Leu Leu Val Thr Ala Ala Leu Val Ala Leu Gly Val Leu Tyr Ala

35                  40                  45

Asp Arg Arg Gly Lys Gln Leu Pro Arg Leu Ala Ser Arg Leu Cys Phe
    50                  55                  60

Leu Gln Glu Glu Arg Thr Phe Val Lys Arg Lys Pro Arg Gly Ile Pro
65                  70                  75                  80

Glu Ala Gln Glu Val Ser Glu Val Cys Thr Thr Pro Gly Cys Val Ile
                85                  90                  95

Ala Ala Ala Arg Ile Leu Gln Asn Met Asp Pro Thr Thr Glu Pro Cys
            100                 105                 110

Asp Asp Phe Tyr Gln Phe Ala Cys Gly Gly Trp Leu Arg Arg His Val
        115                 120                 125

Ile Pro Glu Thr Asn Ser Arg Tyr Ser Ile Phe Asp Val Leu Arg Asp
    130                 135                 140

Glu Leu Glu Val Ile Leu Lys Ala Val Leu Glu Asn Ser Thr Ala Lys
145                 150                 155                 160

Asp Arg Pro Ala Val Glu Lys Ala Arg Thr Leu Tyr Arg Ser Cys Met
                165                 170                 175

Asn Gln Ser Val Ile Glu Lys Arg Gly Ser Gln Pro Leu Leu Asp Ile
            180                 185                 190

Leu Glu Val Val Gly Gly Trp Pro Val Ala Met Asp Arg Trp Asn Glu
        195                 200                 205

Thr Val Gly Leu Glu Trp Glu Leu Glu Arg Gln Leu Ala Leu Met Asn
    210                 215                 220

Ser Gln Phe Asn Arg Arg Val Leu Ile Asp Leu Phe Ile Trp Asn Asp
225                 230                 235                 240

Asp Gln Asn Ser Ser Arg His Ile Ile Tyr Ile Asp Gln Pro Thr Leu
                245                 250                 255

Gly Met Pro Ser Arg Glu Tyr Tyr Phe Asn Gly Gly Ser Asn Arg Lys
            260                 265                 270

Val Arg Glu Ala Tyr Leu Gln Phe Met Val Ser Val Ala Thr Leu Leu
        275                 280                 285

Arg Glu Asp Ala Asn Leu Pro Arg Asp Ser Cys Leu Val Gln Glu Asp
    290                 295                 300

Met Val Gln Val Leu Glu Leu Glu Thr Gln Leu Ala Lys Ala Thr Val
305                 310                 315                 320

Pro Gln Glu Glu Arg His Asp Val Ile Ala Leu Tyr His Arg Met Gly
                325                 330                 335

Leu Glu Glu Leu Gln Ser Gln Phe Gly Leu Lys Gly Phe Asp Trp Thr
            340                 345                 350

Leu Phe Ile Gln Thr Val Leu Ser Ser Val Lys Ile Lys Leu Leu Pro
        355                 360                 365

Asp Glu Glu Val Val Val Tyr Gly Ile Pro Tyr Leu Gln Asn Leu Glu
    370                 375                 380

Asn Ile Ile Asp Thr Tyr Ser Ala Arg Thr Ile Gln Asn Tyr Leu Val
385                 390                 395                 400

Trp Arg Leu Val Leu Asp Arg Ile Gly Ser Leu Ser Gln Arg Phe Lys
                405                 410                 415

Asp Thr Arg Val Asn Tyr Arg Lys Ala Leu Phe Gly Thr Met Val Glu
            420                 425                 430

Glu Val Arg Trp Arg Glu Cys Val Gly Tyr Val Asn Ser Asn Met Glu
        435                 440                 445

Asn Ala Val Gly Ser Leu Tyr Val Arg Glu Ala Phe Pro Gly Asp Ser
    450                 455                 460

Lys Ser Met Val Arg Glu Leu Ile Asp Lys Val Arg Thr Val Phe Val
465                 470                 475                 480

Glu Thr Leu Asp Glu Leu Gly Trp Met Asp Glu Glu Ser Lys Lys Lys
                485                 490                 495

Ala Gln Glu Lys Ala Met Ser Ile Arg Glu Gln Ile Gly His Pro Asp
            500                 505                 510

Tyr Ile Leu Glu Glu Thr Asn Arg Arg Leu Asp Glu Glu Tyr Ser Asn
        515                 520                 525

Leu Asn Phe Ser Glu Asp Leu Tyr Phe Glu Asn Ser Leu Gln Asn Leu
    530                 535                 540

Lys Val Gly Ala Gln Arg Ser Leu Arg Lys Leu Arg Glu Lys Val Asp
545                 550                 555                 560

Pro Asn Leu Trp Ile Ile Gly Ala Ala Val Val Asn Ala Phe Tyr Ser
                565                 570                 575

Pro Asn Arg Asn Gln Ile Val Phe Pro Ala Gly Ile Leu Gln Pro Pro
            580                 585                 590

Phe Phe Ser Lys Glu Gln Pro Gln Ala Leu Asn Phe Gly Gly Ile Gly
        595                 600                 605

Met Val Ile Gly His Glu Ile Thr His Gly Phe Asp Asp Asn Gly Arg
    610                 615                 620

Asn Phe Asp Lys Asn Gly Asn Met Met Asp Trp Trp Ser Asn Phe Ser
625                 630                 635                 640

Thr Gln His Phe Arg Glu Gln Ser Glu Cys Met Ile Tyr Gln Tyr Gly
                645                 650                 655

Asn Tyr Ser Trp Asp Leu Ala Asp Glu Gln Asn Val Asn Gly Phe Asn
            660                 665                 670

Thr Leu Gly Glu Asn Ile Ala Asp Asn Gly Gly Val Arg Gln Ala Tyr
        675                 680                 685

Lys Ala Tyr Leu Lys Trp Met Ala Glu Gly Gly Lys Asp Gln Gln Leu
    690                 695                 700

Pro Gly Leu Asp Leu Thr His Glu Gln Leu Phe Phe Ile Asn Tyr Ala
705                 710                 715                 720

Gln Val Trp Cys Gly Ser Tyr Arg Pro Glu Phe Ala Ile Gln Ser Ile
                725                 730                 735

Lys Thr Asp Val His Ser Pro Leu Lys Tyr Arg Val Leu Gly Ser Leu
            740                 745                 750

Gln Asn Leu Ala Ala Phe Ala Asp Thr Phe His Cys Ala Arg Gly Thr
        755                 760                 765

Pro Met His Pro Lys Glu Arg Cys Arg Val Trp
    770                 775                 780

<210> 3
<211> 2636
<212> DNA
<213> Homo sapiens

<220>
<221> 5'UTR
<222> (1)..(7)

<220>
<221> CDS
<222> (8)..(2269)

<220>
<221> 3'UTR
<222> (2270)..(2635)

<400> 3
cctgctg atg ggg aag tcc gaa ggc ccc gtg ggg atg gtg gag agc gct    49
        Met Gly Lys Ser Glu Gly Pro Val Gly Met Val Glu Ser Ala
          1               5                  10

ggc cgt gca ggg cag aag cgc ccg ggg ttc ctg gag ggg ggg ctg ctg    97
Gly Arg Ala Gly Gln Lys Arg Pro Gly Phe Leu Glu Gly Gly Leu Leu
 15                  20                  25                  30

ctg ctg ctg ctg ctg gtg acc gct gcc ctg gtg gcc ttg ggt gtc ctc    145
Leu Leu Leu Leu Leu Val Thr Ala Ala Leu Val Ala Leu Gly Val Leu
                 35                  40                  45

tac gcc gac cgc aga ggg atc cca gag gcc caa gag gtg agc gag gtc    193
Tyr Ala Asp Arg Arg Gly Ile Pro Glu Ala Gln Glu Val Ser Glu Val
             50                  55                  60

tgc acc acc cct ggc tgc gtg ata gca gct gcc agg atc ctc cag aac    241
Cys Thr Thr Pro Gly Cys Val Ile Ala Ala Ala Arg Ile Leu Gln Asn
         65                  70                  75

atg gac ccg acc acg gaa ccg tgt gac gac ttc tac cag ttt gca tgc    289
Met Asp Pro Thr Thr Glu Pro Cys Asp Asp Phe Tyr Gln Phe Ala Cys
     80                  85                  90

gga ggc tgg ctg cgg cgc cac gtg atc cct gag acc aac tca aga tac    337
Gly Gly Trp Leu Arg Arg His Val Ile Pro Glu Thr Asn Ser Arg Tyr
 95                 100                 105                 110

agc atc ttt gac gtc ctc cgc gac gag ctg gag gtc atc ctc aaa gcg    385
Ser Ile Phe Asp Val Leu Arg Asp Glu Leu Glu Val Ile Leu Lys Ala
                115                 120                 125

gtg ctg gag aat tcg act gcc aag gac cgg ccg gct gtg gag aag gcc    433

Val Leu Glu Asn Ser Thr Ala Lys Asp Arg Pro Ala Val Glu Lys Ala
            130                 135                 140

agg acg ctg tac cgc tcc tgc atg aac cag agt gtg ata gag aag cga 481
Arg Thr Leu Tyr Arg Ser Cys Met Asn Gln Ser Val Ile Glu Lys Arg
        145                 150                 155

ggc tct cag ccc ctg ctg gac atc ttg gag gtg gtg gga ggc tgg ccg 529
Gly Ser Gln Pro Leu Leu Asp Ile Leu Glu Val Val Gly Gly Trp Pro
    160                 165                 170

gtg gcg atg gac agg tgg aac gag acc gta gga ctc gag tgg gag ctg 577
Val Ala Met Asp Arg Trp Asn Glu Thr Val Gly Leu Glu Trp Glu Leu
175                 180                 185                 190

gag cgg cag ctg gcg ctg atg aac tca cag ttc aac agg cgc gtc ctc 625
Glu Arg Gln Leu Ala Leu Met Asn Ser Gln Phe Asn Arg Arg Val Leu
                195                 200                 205

atc gac ctc ttc atc tgg aac gac gac cag aac tcc agc cgg cac atc 673
Ile Asp Leu Phe Ile Trp Asn Asp Asp Gln Asn Ser Ser Arg His Ile
            210                 215                 220

atc tac ata gac cag ccc acc ttg ggc atg ccc tcc cga gag tac tac 721
Ile Tyr Ile Asp Gln Pro Thr Leu Gly Met Pro Ser Arg Glu Tyr Tyr
        225                 230                 235

ttc aac ggc ggc agc aac cgg aag gtg cgg gaa gcc tac ctg cag ttc 769
Phe Asn Gly Gly Ser Asn Arg Lys Val Arg Glu Ala Tyr Leu Gln Phe
    240                 245                 250

atg gtg tca gtg gcc acg ttg ctg cgg gag gat gca aac ctg ccc agg 817
Met Val Ser Val Ala Thr Leu Leu Arg Glu Asp Ala Asn Leu Pro Arg
255                 260                 265                 270

gac agc tgc ctg gtg cag gag gac atg gtg cag gtg ctg gag ctg gag 865
Asp Ser Cys Leu Val Gln Glu Asp Met Val Gln Val Leu Glu Leu Glu
                275                 280                 285

aca cag ctg gcc aag gcc acg gta ccc cag gag gag aga cac gac gtc 913
Thr Gln Leu Ala Lys Ala Thr Val Pro Gln Glu Glu Arg His Asp Val
            290                 295                 300

atc gcc ttg tac cac cgg atg gga ctg gag gag ctg caa agc cag ttt 961
Ile Ala Leu Tyr His Arg Met Gly Leu Glu Glu Leu Gln Ser Gln Phe
        305                 310                 315

ggc ctg aag gga ttt gac tgg act ctg ttc ata caa act gtg cta tcc 1009
Gly Leu Lys Gly Phe Asp Trp Thr Leu Phe Ile Gln Thr Val Leu Ser
    320                 325                 330

tct gtc aaa atc aag ctg ctg cca gat gag gaa gtg gtg gtc tat ggc 1057
Ser Val Lys Ile Lys Leu Leu Pro Asp Glu Glu Val Val Val Tyr Gly
335                 340                 345                 350

atc ccc tac ctg cag aac ctt gaa aac atc atc gac acc tac tca gcc 1105
Ile Pro Tyr Leu Gln Asn Leu Glu Asn Ile Ile Asp Thr Tyr Ser Ala
                355                 360                 365

agg acc ata cag aac tac ctg gtc tgg cgc ctg gtg ctg gac cgc att 1153
Arg Thr Ile Gln Asn Tyr Leu Val Trp Arg Leu Val Leu Asp Arg Ile
            370                 375                 380

ggt agc cta agc cag aga ttc aag gac aca cga gtg aac tac cgc aag 1201

Gly Ser Leu Ser Gln Arg Phe Lys Asp Thr Arg Val Asn Tyr Arg Lys
        385                 390                 395

gcg ctg ttt ggc aca atg gtg gag gag gtg cgc tgg cgt gaa tgt gtg 1249
Ala Leu Phe Gly Thr Met Val Glu Glu Val Arg Trp Arg Glu Cys Val
    400                 405                 410

ggc tac gtc aac agc aac atg gag aac gcc gtg ggc tcc ctc tac gtc 1297
Gly Tyr Val Asn Ser Asn Met Glu Asn Ala Val Gly Ser Leu Tyr Val
415                 420                 425                 430

agg gag gcg ttc cct gga gac agc aag agc atg gtc aga gaa ctc att 1345
Arg Glu Ala Phe Pro Gly Asp Ser Lys Ser Met Val Arg Glu Leu Ile
                435                 440                 445

gac aag gtg cgg aca gtg ttt gtg gag acg ctg gac gag ctg ggc tgg 1393
Asp Lys Val Arg Thr Val Phe Val Glu Thr Leu Asp Glu Leu Gly Trp
            450                 455                 460

atg gac gag gag tcc aag aag aag gcg cag gag aag gcc atg agc atc 1441
Met Asp Glu Glu Ser Lys Lys Lys Ala Gln Glu Lys Ala Met Ser Ile
        465                 470                 475

cgg gag cag atc ggg cac cct gac tac atc ctg gag gag acg aac agg 1489
Arg Glu Gln Ile Gly His Pro Asp Tyr Ile Leu Glu Glu Thr Asn Arg
    480                 485                 490

cgc ctg gac gag gag tac tcc aat ctg aac ttc tca gag gac ctg tac 1537
Arg Leu Asp Glu Glu Tyr Ser Asn Leu Asn Phe Ser Glu Asp Leu Tyr
495                 500                 505                 510

ttt gag aac agt ctg cag aac ctc aag gtg ggc gcc cag cgg agc ctc 1585
Phe Glu Asn Ser Leu Gln Asn Leu Lys Val Gly Ala Gln Arg Ser Leu
                515                 520                 525

agg aag ctt cgg gaa aag gtg gac cca aat ctc tgg atc atc ggg gcg 1633
Arg Lys Leu Arg Glu Lys Val Asp Pro Asn Leu Trp Ile Ile Gly Ala
            530                 535                 540

gcg gtg gtc aat gcg ttc tac tcc cca aac cga aac cag att gta ttc 1681
Ala Val Val Asn Ala Phe Tyr Ser Pro Asn Arg Asn Gln Ile Val Phe
        545                 550                 555

cct gcc ggg atc ctc cag ccc ccc ttc ttc agc aag gag cag cca cag 1729
Pro Ala Gly Ile Leu Gln Pro Pro Phe Phe Ser Lys Glu Gln Pro Gln
    560                 565                 570

gcc ttg aac ttt gga ggc att ggg atg gtg atc ggg cac gag atc acg 1777
Ala Leu Asn Phe Gly Gly Ile Gly Met Val Ile Gly His Glu Ile Thr
575                 580                 585                 590

cac ggc ttt gac gac aat ggc cgg aac ttc gac aag aat ggc aac atg 1825
His Gly Phe Asp Asp Asn Gly Arg Asn Phe Asp Lys Asn Gly Asn Met
                595                 600                 605

atg gat tgg tgg agt aac ttc tcc acc cag cac ttc cgg gag cag tca 1873
Met Asp Trp Trp Ser Asn Phe Ser Thr Gln His Phe Arg Glu Gln Ser
            610                 615                 620

gag tgc atg atc tac cag tac ggc aac tac tcc tgg gac ctg gca gac 1921
Glu Cys Met Ile Tyr Gln Tyr Gly Asn Tyr Ser Trp Asp Leu Ala Asp
        625                 630                 635

gaa cag aac gtg aac gga ttc aac acc ctt ggg gaa aac att gct gac 1969

Glu Gln Asn Val Asn Gly Phe Asn Thr Leu Gly Glu Asn Ile Ala Asp
    640                 645                 650

aac gga ggg gtg cgg caa gcc tat aag gcc tac ctc aag tgg atg gca 2017
Asn Gly Gly Val Arg Gln Ala Tyr Lys Ala Tyr Leu Lys Trp Met Ala
655                 660                 665                 670

gag ggt ggc aag gac cag cag ctg ccc ggc ctg gat ctc acc cat gag 2065
Glu Gly Gly Lys Asp Gln Gln Leu Pro Gly Leu Asp Leu Thr His Glu
                675                 680                 685

cag ctc ttc ttc atc aac tat gcc cag gtg tgg tgc ggg tcc tac cgg 2113
Gln Leu Phe Phe Ile Asn Tyr Ala Gln Val Trp Cys Gly Ser Tyr Arg
            690                 695                 700

ccc gag ttc gcc atc caa tcc atc aag aca gac gtc cac agt ccc ctg 2161
Pro Glu Phe Ala Ile Gln Ser Ile Lys Thr Asp Val His Ser Pro Leu
        705                 710                 715

aag tac agg gta ctg ggg tcg ctg cag aac ctg gcc gcc ttc gca gac 2209
Lys Tyr Arg Val Leu Gly Ser Leu Gln Asn Leu Ala Ala Phe Ala Asp
    720                 725                 730

acg ttc cac tgt gcc cgg ggc acc ccc atg cac ccc aag gag cga tgc 2257
Thr Phe His Cys Ala Arg Gly Thr Pro Met His Pro Lys Glu Arg Cys
735                 740                 745                 750

cgc gtg tgg tag ccaaggccct gccgcgctgt gcggcccacg cccacctgct 2309
Arg Val Trp

gctcggaggc atctgtgcga aggtgcagct agcggcgacc cagtgtacgt cccgccccgg 2369

ccaaccatgc caagcctgcc tgccaggcct ctgcgcctgg cctagggtgc agccacctgc 2429

ctgacaccca gggatgagca gtgtccagtg cagtacctgg accggagccc cctccacaga 2489

cacccgcggg gctcagtgcc cccgtcacag ctctgtagag acaatcaact gtgtcctgcc 2549

caccctccaa ggtgcattgt cttccagtat ctacagcttc agacttgagc taagtaaatg 2609

cttcaaagaa aaaaaaaaaa aaaaaad 2636

<210> 4
<211> 753
<212> PRT
<213> Homo sapiens

<400> 4
Met Gly Lys Ser Glu Gly Pro Val Gly Met Val Glu Ser Ala Gly Arg
1               5                   10                  15

Ala Gly Gln Lys Arg Pro Gly Phe Leu Glu Gly Gly Leu Leu Leu Leu
            20                  25                  30

Leu Leu Leu Val Thr Ala Ala Leu Val Ala Leu Gly Val Leu Tyr Ala
        35                  40                  45

Asp Arg Arg Gly Ile Pro Glu Ala Gln Glu Val Ser Glu Val Cys Thr
    50                  55                  60

Thr Pro Gly Cys Val Ile Ala Ala Ala Arg Ile Leu Gln Asn Met Asp
65                  70                  75                  80

Pro Thr Thr Glu Pro Cys Asp Asp Phe Tyr Gln Phe Ala Cys Gly Gly
                85                  90                  95

Trp Leu Arg Arg His Val Ile Pro Glu Thr Asn Ser Arg Tyr Ser Ile
            100                 105                 110

Phe Asp Val Leu Arg Asp Glu Leu Glu Val Ile Leu Lys Ala Val Leu
        115                 120                 125

Glu Asn Ser Thr Ala Lys Asp Arg Pro Ala Val Glu Lys Ala Arg Thr
    130                 135                 140

Leu Tyr Arg Ser Cys Met Asn Gln Ser Val Ile Glu Lys Arg Gly Ser
145                 150                 155                 160

Gln Pro Leu Leu Asp Ile Leu Glu Val Val Gly Gly Trp Pro Val Ala
                165                 170                 175

Met Asp Arg Trp Asn Glu Thr Val Gly Leu Glu Trp Glu Leu Glu Arg
            180                 185                 190

Gln Leu Ala Leu Met Asn Ser Gln Phe Asn Arg Arg Val Leu Ile Asp
        195                 200                 205

Leu Phe Ile Trp Asn Asp Asp Gln Asn Ser Ser Arg His Ile Ile Tyr
    210                 215                 220

Ile Asp Gln Pro Thr Leu Gly Met Pro Ser Arg Glu Tyr Tyr Phe Asn
225                 230                 235                 240

Gly Gly Ser Asn Arg Lys Val Arg Glu Ala Tyr Leu Gln Phe Met Val
                245                 250                 255

Ser Val Ala Thr Leu Leu Arg Glu Asp Ala Asn Leu Pro Arg Asp Ser
            260                 265                 270

Cys Leu Val Gln Glu Asp Met Val Gln Val Leu Glu Leu Glu Thr Gln
        275                 280                 285

Leu Ala Lys Ala Thr Val Pro Gln Glu Glu Arg His Asp Val Ile Ala
    290                 295                 300

Leu Tyr His Arg Met Gly Leu Glu Glu Leu Gln Ser Gln Phe Gly Leu
305                 310                 315                 320

Lys Gly Phe Asp Trp Thr Leu Phe Ile Gln Thr Val Leu Ser Ser Val
                325                 330                 335

Lys Ile Lys Leu Leu Pro Asp Glu Glu Val Val Val Tyr Gly Ile Pro
            340                 345                 350

Tyr Leu Gln Asn Leu Glu Asn Ile Ile Asp Thr Tyr Ser Ala Arg Thr
        355                 360                 365

Ile Gln Asn Tyr Leu Val Trp Arg Leu Val Leu Asp Arg Ile Gly Ser
    370                 375                 380

Leu Ser Gln Arg Phe Lys Asp Thr Arg Val Asn Tyr Arg Lys Ala Leu
385                 390                 395                 400

Phe Gly Thr Met Val Glu Glu Val Arg Trp Arg Glu Cys Val Gly Tyr
                405                 410                 415

Val Asn Ser Asn Met Glu Asn Ala Val Gly Ser Leu Tyr Val Arg Glu

420                 425                 430

Ala Phe Pro Gly Asp Ser Lys Ser Met Val Arg Glu Leu Ile Asp Lys
        435                 440                 445

Val Arg Thr Val Phe Val Glu Thr Leu Asp Glu Leu Gly Trp Met Asp
    450                 455                 460

Glu Glu Ser Lys Lys Lys Ala Gln Glu Lys Ala Met Ser Ile Arg Glu
465                 470                 475                 480

Gln Ile Gly His Pro Asp Tyr Ile Leu Glu Glu Thr Asn Arg Arg Leu
                485                 490                 495

Asp Glu Glu Tyr Ser Asn Leu Asn Phe Ser Glu Asp Leu Tyr Phe Glu
            500                 505                 510

Asn Ser Leu Gln Asn Leu Lys Val Gly Ala Gln Arg Ser Leu Arg Lys
        515                 520                 525

Leu Arg Glu Lys Val Asp Pro Asn Leu Trp Ile Ile Gly Ala Ala Val
    530                 535                 540

Val Asn Ala Phe Tyr Ser Pro Asn Arg Asn Gln Ile Val Phe Pro Ala
545                 550                 555                 560

Gly Ile Leu Gln Pro Pro Phe Phe Ser Lys Glu Gln Pro Gln Ala Leu
                565                 570                 575

Asn Phe Gly Gly Ile Gly Met Val Ile Gly His Glu Ile Thr His Gly
            580                 585                 590

Phe Asp Asp Asn Gly Arg Asn Phe Asp Lys Asn Gly Asn Met Met Asp
        595                 600                 605

Trp Trp Ser Asn Phe Ser Thr Gln His Phe Arg Glu Gln Ser Glu Cys
    610                 615                 620

Met Ile Tyr Gln Tyr Gly Asn Tyr Ser Trp Asp Leu Ala Asp Glu Gln
625                 630                 635                 640

Asn Val Asn Gly Phe Asn Thr Leu Gly Glu Asn Ile Ala Asp Asn Gly
                645                 650                 655

Gly Val Arg Gln Ala Tyr Lys Ala Tyr Leu Lys Trp Met Ala Glu Gly
            660                 665                 670

Gly Lys Asp Gln Gln Leu Pro Gly Leu Asp Leu Thr His Glu Gln Leu
        675                 680                 685

Phe Phe Ile Asn Tyr Ala Gln Val Trp Cys Gly Ser Tyr Arg Pro Glu
    690                 695                 700

Phe Ala Ile Gln Ser Ile Lys Thr Asp Val His Ser Pro Leu Lys Tyr
705                 710                 715                 720

Arg Val Leu Gly Ser Leu Gln Asn Leu Ala Ala Phe Ala Asp Thr Phe
                725                 730                 735

His Cys Ala Arg Gly Thr Pro Met His Pro Lys Glu Arg Cys Arg Val
            740                 745                 750

Trp

<210> 5
<211> 2663
<212> DNA
<213> Homo sapiens

<220>
<221> 5'UTR
<222> (1)..(7)

<220>
<221> CDS
<222> (8)..(2293)

<220>
<221> 3'UTR
<222> (2294)..(2663)

<400> 5
cctgctg atg ggg aag tcc gaa ggc ccc gtg ggg atg gtg gag agc gct 49
Met Gly Lys Ser Glu Gly Pro Val Gly Met Val Glu Ser Ala
1               5                   10

ggc cgt gca ggg cag aag cgc ccg ggg ttc ctg gag ggg ggg ctg ctg 97
Gly Arg Ala Gly Gln Lys Arg Pro Gly Phe Leu Glu Gly Gly Leu Leu
15                  20                  25                  30

ctg ctg ctg ctg ctg gtg acc gct gcc ctg gtg gcc ttg ggt gtc ctc 145
Leu Leu Leu Leu Leu Val Thr Ala Ala Leu Val Ala Leu Gly Val Leu
                35                  40                  45

tac gcc gac cgc aga ggg aag cag ctg cca cgc ctt gct agc cgg ctg 193
Tyr Ala Asp Arg Arg Gly Lys Gln Leu Pro Arg Leu Ala Ser Arg Leu
            50                  55                  60

tgc ttc tta cag gag gag agg acc ttt gta aaa cga aaa ccc cga ggg 241
Cys Phe Leu Gln Glu Glu Arg Thr Phe Val Lys Arg Lys Pro Arg Gly
        65                  70                  75

atc cca gag gcc caa gag gtg agc gag gtc tgc acc acc cct ggc tgc 289
Ile Pro Glu Ala Gln Glu Val Ser Glu Val Cys Thr Thr Pro Gly Cys
    80                  85                  90

gtg ata gca gct gcc agg atc ctc cag aac atg gac ccg acc acg gaa 337
Val Ile Ala Ala Ala Arg Ile Leu Gln Asn Met Asp Pro Thr Thr Glu
95                  100                 105                 110

ccg tgt gac gac ttc tac cag ttt gca tgc gga ggc tgg ctg cgg cgc 385
Pro Cys Asp Asp Phe Tyr Gln Phe Ala Cys Gly Gly Trp Leu Arg Arg
                115                 120                 125

cac gtg atc cct gag acc aac tca aga tac agc atc ttt gac gtc ctc 433
His Val Ile Pro Glu Thr Asn Ser Arg Tyr Ser Ile Phe Asp Val Leu
            130                 135                 140

cgc gac gag ctg gag gtc atc ctc aaa gcg gtg ctg gag aat tcg act 481
Arg Asp Glu Leu Glu Val Ile Leu Lys Ala Val Leu Glu Asn Ser Thr
        145                 150                 155

gcc aag gac cgg ccg gct gtg gag aag gcc agg acg ctg tac cgc tcc 529
Ala Lys Asp Arg Pro Ala Val Glu Lys Ala Arg Thr Leu Tyr Arg Ser
    160                 165                 170

tgc atg aac cag agt gtg ata gag aag cga ggc tct cag ccc ctg ctg 577

Cys Met Asn Gln Ser Val Ile Glu Lys Arg Gly Ser Gln Pro Leu Leu
175                 180                 185                 190

gac atc ttg gag gtg gtg gga ggc tgg ccg gtg gcg atg gac agg tgg    625
Asp Ile Leu Glu Val Val Gly Gly Trp Pro Val Ala Met Asp Arg Trp
                195                 200                 205

aac gag acc gta gtt caa cag gcg cgt cct cat cga cct ctt cat ctg    673
Asn Glu Thr Val Val Gln Gln Ala Arg Pro His Arg Pro Leu His Leu
            210                 215                 220

gaa cga cga cca gaa ctc cag ccg gca cat cat cta cac cag ccc acc    721
Glu Arg Arg Pro Glu Leu Gln Pro Ala His His Leu His Gln Pro Thr
        225                 230                 235

ttg ggc atg ccc tcc cga gag tac tac ttc aac ggc ggc agc aac cgg    769
Leu Gly Met Pro Ser Arg Glu Tyr Tyr Phe Asn Gly Gly Ser Asn Arg
    240                 245                 250

aag gtg cgg gaa gcc tac ctg cag ttc atg gtg tca gtg gcc acg ttg    817
Lys Val Arg Glu Ala Tyr Leu Gln Phe Met Val Ser Val Ala Thr Leu
255                 260                 265                 270

ctg cgg gag gat gca aac ctg ccc agg gac agc tgc ctg gtg cag gag    865
Leu Arg Glu Asp Ala Asn Leu Pro Arg Asp Ser Cys Leu Val Gln Glu
                275                 280                 285

gac atg gtg cag gtg ctg gag ctg gag aca cag ctg gcc aag gcc acg    913
Asp Met Val Gln Val Leu Glu Leu Glu Thr Gln Leu Ala Lys Ala Thr
            290                 295                 300

gta ccc cag gag gag aga cac gac gtc atc gcc ttg tac cac cgg atg    961
Val Pro Gln Glu Glu Arg His Asp Val Ile Ala Leu Tyr His Arg Met
        305                 310                 315

gga ctg gag gag ctg caa agc cag ttt ggc ctg aag gga ttt gac tgg    1009
Gly Leu Glu Glu Leu Gln Ser Gln Phe Gly Leu Lys Gly Phe Asp Trp
    320                 325                 330

act ctg ttc ata caa act gtg cta tcc tct gtc aaa atc aag ctg ctg    1057
Thr Leu Phe Ile Gln Thr Val Leu Ser Ser Val Lys Ile Lys Leu Leu
335                 340                 345                 350

cca gat gag gaa gtg gtg gtc tat ggc atc ccc tac ctg cag aac ctt    1105
Pro Asp Glu Glu Val Val Val Tyr Gly Ile Pro Tyr Leu Gln Asn Leu
                355                 360                 365

gaa aac atc atc gac acc tac tca gcc agg acc ata cag aac tac ctg    1153
Glu Asn Ile Ile Asp Thr Tyr Ser Ala Arg Thr Ile Gln Asn Tyr Leu
            370                 375                 380

gtc tgg cgc ctg gtg ctg gac cgc att ggt agc cta agc cag aga ttc    1201
Val Trp Arg Leu Val Leu Asp Arg Ile Gly Ser Leu Ser Gln Arg Phe
        385                 390                 395

aag gac aca cga gtg aac tac cgc aag gcg ctg ttt ggc aca atg gtg    1249
Lys Asp Thr Arg Val Asn Tyr Arg Lys Ala Leu Phe Gly Thr Met Val
    400                 405                 410

gag gag gtg cgc tgg cgt gaa tgt gtg ggc tac gtc aac agc aac atg    1297
Glu Glu Val Arg Trp Arg Glu Cys Val Gly Tyr Val Asn Ser Asn Met
415                 420                 425                 430

gag aac gcc gtg ggc tcc ctc tac gtc agg gag gcg ttc cct gga gac    1345

Glu Asn Ala Val Gly Ser Leu Tyr Val Arg Glu Ala Phe Pro Gly Asp
                435                 440                 445

agc aag agc atg gtc aga gaa ctc att gac aag gtg cgg aca gtg ttt    1393
Ser Lys Ser Met Val Arg Glu Leu Ile Asp Lys Val Arg Thr Val Phe
            450                 455                 460

gtg gag acg ctg gac gag ctg ggc tgg atg gac gag gag tcc aag aag    1441
Val Glu Thr Leu Asp Glu Leu Gly Trp Met Asp Glu Glu Ser Lys Lys
        465                 470                 475

aag gcg cag gag aag gcc atg agc atc cgg gag cag atc ggg cac cct    1489
Lys Ala Gln Glu Lys Ala Met Ser Ile Arg Glu Gln Ile Gly His Pro
    480                 485                 490

gac tac atc ctg gag gag acg aac agg cgc ctg gac gag gag tac tcc    1537
Asp Tyr Ile Leu Glu Glu Thr Asn Arg Arg Leu Asp Glu Glu Tyr Ser
495                 500                 505                 510

aat ctg aac ttc tca gag gac ctg tac ttt gag aac agt ctg cag aac    1585
Asn Leu Asn Phe Ser Glu Asp Leu Tyr Phe Glu Asn Ser Leu Gln Asn
                515                 520                 525

ctc aag gtg ggc gcc cag cgg agc ctc agg aag ctt cgg gaa aag gtg    1633
Leu Lys Val Gly Ala Gln Arg Ser Leu Arg Lys Leu Arg Glu Lys Val
            530                 535                 540

gac cca aat ctc tgg atc atc ggg gcg gcg gtg gtc aat gcg ttc tac    1681
Asp Pro Asn Leu Trp Ile Ile Gly Ala Ala Val Val Asn Ala Phe Tyr
        545                 550                 555

tcc cca aac cga aac cag att gta ttc cct gcc ggg atc ctc cag ccc    1729
Ser Pro Asn Arg Asn Gln Ile Val Phe Pro Ala Gly Ile Leu Gln Pro
    560                 565                 570

ccc ttc ttc agc aag gag cag cca cag gcc ttg aac ttt gga ggc att    1777
Pro Phe Phe Ser Lys Glu Gln Pro Gln Ala Leu Asn Phe Gly Gly Ile
575                 580                 585                 590

ggg atg gtg atc ggg cac gag atc acg cac ggc ttt gac gac aat ggc    1825
Gly Met Val Ile Gly His Glu Ile Thr His Gly Phe Asp Asp Asn Gly
                595                 600                 605

cgg aac ttc gac aag aat ggc aac atg atg gat tgg tgg agt aac ttc    1873
Arg Asn Phe Asp Lys Asn Gly Asn Met Met Asp Trp Trp Ser Asn Phe
            610                 615                 620

tcc acc cag cac ttc cgg gag cag tca gag tgc atg atc tac cag tac    1921
Ser Thr Gln His Phe Arg Glu Gln Ser Glu Cys Met Ile Tyr Gln Tyr
        625                 630                 635

ggc aac tac tcc tgg gac ctg gca gac gaa cag aac gtg aac gga ttc    1969
Gly Asn Tyr Ser Trp Asp Leu Ala Asp Glu Gln Asn Val Asn Gly Phe
    640                 645                 650

aac acc ctt ggg gaa aac att gct gac aac gga ggg gtg cgg caa gcc    2017
Asn Thr Leu Gly Glu Asn Ile Ala Asp Asn Gly Gly Val Arg Gln Ala
655                 660                 665                 670

tat aag gcc tac ctc aag tgg atg gca gag ggt ggc aag gac cag cag    2065
Tyr Lys Ala Tyr Leu Lys Trp Met Ala Glu Gly Gly Lys Asp Gln Gln
                675                 680                 685

ctg ccc ggc ctg gat ctc acc cat gag cag ctc ttc ttc atc aac tat    2113

Leu Pro Gly Leu Asp Leu Thr His Glu Gln Leu Phe Phe Ile Asn Tyr
            690                 695                 700

gcc cag gtg tgg tgc ggg tcc tac cgg ccc gag ttc gcc atc caa tcc     2161
Ala Gln Val Trp Cys Gly Ser Tyr Arg Pro Glu Phe Ala Ile Gln Ser
        705                 710                 715

atc aag aca gac gtc cac agt ccc ctg aag tac agg gta ctg ggg tcg     2209
Ile Lys Thr Asp Val His Ser Pro Leu Lys Tyr Arg Val Leu Gly Ser
    720                 725                 730

ctg cag aac ctg gcc gcc ttc gca gac acg ttc cac tgt gcc cgg ggc     2257
Leu Gln Asn Leu Ala Ala Phe Ala Asp Thr Phe His Cys Ala Arg Gly
735                 740                 745                 750

acc ccc atg cac ccc aag gag cga tgc cgc gtg tgg tagccaaggc          2303
Thr Pro Met His Pro Lys Glu Arg Cys Arg Val Trp
                755                 760

cctgccgcgc tgtgcggccc acgcccacct gctgctcgga ggcatctgtg cgaaggtgca 2363

gctagcggcg acccagtgta cgtcccgccc cggccaacca tgccaagcct gcctgccagg 2423

cctctgcgcc tggcctaggg tgcagccacc tgcctgacac ccagggatga gcagtgtcca 2483

gtgcagtacc tggaccggag ccccctccac agacacccgc ggggctcagt gcccccgtca 2543

cagctctgta gagacaatca actgtgtcct gcccaccctc caaggtgcat tgtcttccag 2603

tatctacagc ttcagacttg agctaagtaa atgcttcaaa gaaaaaaaaa aaaaaaaaad 2663

<210> 6
<211> 762
<212> PRT
<213> Homo sapiens

<400> 6
Met Gly Lys Ser Glu Gly Pro Val Gly Met Val Glu Ser Ala Gly Arg
1               5                   10                  15

Ala Gly Gln Lys Arg Pro Gly Phe Leu Glu Gly Gly Leu Leu Leu Leu
            20                  25                  30

Leu Leu Leu Val Thr Ala Ala Leu Val Ala Leu Gly Val Leu Tyr Ala
        35                  40                  45

Asp Arg Arg Gly Lys Gln Leu Pro Arg Leu Ala Ser Arg Leu Cys Phe
    50                  55                  60

Leu Gln Glu Glu Arg Thr Phe Val Lys Arg Lys Pro Arg Gly Ile Pro
65                  70                  75                  80

Glu Ala Gln Glu Val Ser Glu Val Cys Thr Thr Pro Gly Cys Val Ile
                85                  90                  95

Ala Ala Ala Arg Ile Leu Gln Asn Met Asp Pro Thr Thr Glu Pro Cys
            100                 105                 110

Asp Asp Phe Tyr Gln Phe Ala Cys Gly Gly Trp Leu Arg Arg His Val
        115                 120                 125

Ile Pro Glu Thr Asn Ser Arg Tyr Ser Ile Phe Asp Val Leu Arg Asp
    130                 135                 140

Glu Leu Glu Val Ile Leu Lys Ala Val Leu Glu Asn Ser Thr Ala Lys
145                 150                 155                 160

Asp Arg Pro Ala Val Glu Lys Ala Arg Thr Leu Tyr Arg Ser Cys Met
                165                 170                 175

Asn Gln Ser Val Ile Glu Lys Arg Gly Ser Gln Pro Leu Leu Asp Ile
            180                 185                 190

Leu Glu Val Val Gly Gly Trp Pro Val Ala Met Asp Arg Trp Asn Glu
        195                 200                 205

Thr Val Val Gln Gln Ala Arg Pro His Arg Pro Leu His Leu Glu Arg
    210                 215                 220

Arg Pro Glu Leu Gln Pro Ala His His Leu His Gln Pro Thr Leu Gly
225                 230                 235                 240

Met Pro Ser Arg Glu Tyr Tyr Phe Asn Gly Gly Ser Asn Arg Lys Val
                245                 250                 255

Arg Glu Ala Tyr Leu Gln Phe Met Val Ser Val Ala Thr Leu Leu Arg
            260                 265                 270

Glu Asp Ala Asn Leu Pro Arg Asp Ser Cys Leu Val Gln Glu Asp Met
        275                 280                 285

Val Gln Val Leu Glu Leu Glu Thr Gln Leu Ala Lys Ala Thr Val Pro
    290                 295                 300

Gln Glu Glu Arg His Asp Val Ile Ala Leu Tyr His Arg Met Gly Leu
305                 310                 315                 320

Glu Glu Leu Gln Ser Gln Phe Gly Leu Lys Gly Phe Asp Trp Thr Leu
                325                 330                 335

Phe Ile Gln Thr Val Leu Ser Ser Val Lys Ile Lys Leu Leu Pro Asp
            340                 345                 350

Glu Glu Val Val Val Tyr Gly Ile Pro Tyr Leu Gln Asn Leu Glu Asn
        355                 360                 365

Ile Ile Asp Thr Tyr Ser Ala Arg Thr Ile Gln Asn Tyr Leu Val Trp
    370                 375                 380

Arg Leu Val Leu Asp Arg Ile Gly Ser Leu Ser Gln Arg Phe Lys Asp
385                 390                 395                 400

Thr Arg Val Asn Tyr Arg Lys Ala Leu Phe Gly Thr Met Val Glu Glu
                405                 410                 415

Val Arg Trp Arg Glu Cys Val Gly Tyr Val Asn Ser Asn Met Glu Asn
            420                 425                 430

Ala Val Gly Ser Leu Tyr Val Arg Glu Ala Phe Pro Gly Asp Ser Lys
        435                 440                 445

Ser Met Val Arg Glu Leu Ile Asp Lys Val Arg Thr Val Phe Val Glu
    450                 455                 460

Thr Leu Asp Glu Leu Gly Trp Met Asp Glu Glu Ser Lys Lys Lys Ala
465                 470                 475                 480

Gln Glu Lys Ala Met Ser Ile Arg Glu Gln Ile Gly His Pro Asp Tyr
                485                 490                 495

Ile Leu Glu Glu Thr Asn Arg Arg Leu Asp Glu Glu Tyr Ser Asn Leu
            500                 505                 510

Asn Phe Ser Glu Asp Leu Tyr Phe Glu Asn Ser Leu Gln Asn Leu Lys
        515                 520                 525

Val Gly Ala Gln Arg Ser Leu Arg Lys Leu Arg Glu Lys Val Asp Pro
    530                 535                 540

Asn Leu Trp Ile Ile Gly Ala Ala Val Val Asn Ala Phe Tyr Ser Pro
545                 550                 555                 560

Asn Arg Asn Gln Ile Val Phe Pro Ala Gly Ile Leu Gln Pro Pro Phe
                565                 570                 575

Phe Ser Lys Glu Gln Pro Gln Ala Leu Asn Phe Gly Gly Ile Gly Met
            580                 585                 590

Val Ile Gly His Glu Ile Thr His Gly Phe Asp Asp Asn Gly Arg Asn
        595                 600                 605

Phe Asp Lys Asn Gly Asn Met Met Asp Trp Trp Ser Asn Phe Ser Thr
    610                 615                 620

Gln His Phe Arg Glu Gln Ser Glu Cys Met Ile Tyr Gln Tyr Gly Asn
625                 630                 635                 640

Tyr Ser Trp Asp Leu Ala Asp Glu Gln Asn Val Asn Gly Phe Asn Thr
                645                 650                 655

Leu Gly Glu Asn Ile Ala Asp Asn Gly Gly Val Arg Gln Ala Tyr Lys
            660                 665                 670

Ala Tyr Leu Lys Trp Met Ala Glu Gly Gly Lys Asp Gln Gln Leu Pro
        675                 680                 685

Gly Leu Asp Leu Thr His Glu Gln Leu Phe Phe Ile Asn Tyr Ala Gln
    690                 695                 700

Val Trp Cys Gly Ser Tyr Arg Pro Glu Phe Ala Ile Gln Ser Ile Lys
705                 710                 715                 720

Thr Asp Val His Ser Pro Leu Lys Tyr Arg Val Leu Gly Ser Leu Gln
                725                 730                 735

Asn Leu Ala Ala Phe Ala Asp Thr Phe His Cys Ala Arg Gly Thr Pro
            740                 745                 750

Met His Pro Lys Glu Arg Cys Arg Val Trp
        755                 760

## Description

### FIELD OF THE INVENTION

The invention relates to newly identified polynucleotides, polypeptides encoded by them and to the use of such polynucleotides and polypeptides, and to their production. More particularly, the polypeptides of the present invention are novel membrane metallopeptidase proteins, hereinafter referred to as human SNEP. Three variants are described and referred to as human SNEPa, SNEPb and SNEPc.

### BACKGROUND OF THE INVENTION

Membrane-associated metallopeptidases belonging to the neprilysin family (M13) (Rawling N.D. and Barrett A.J. (1993) Biochem.J., 290:205-218) play key roles in the post-traductionnal processing and/or metabolism of neuropeptides and peptide hormones. Neutral endopeptidase-24.11 (NEP) is the prototype of this family of zinc metallopeptidases that also includes the endothelin-converting enzymes (ECE), the erythocyte cell-surface antigen KELL, the product of the PEX gene and the newly described XCE enzyme. This family of mammalian proteases is structurally related to the bacterial enzymes thermolysin and lactococcal endopeptidase O.

These proteins are type II membrane-bound proteases and share structural similarities including a short N-terminal intracellular region, the presence of an unique transmembrane spanning domain and a large extracellular C-terminal part where the catalytic site is located. These proteins share also a consensus His-Glu-Xaa-Xaa-His (or HEXXH) motif as well as a conserved Glu residue located 50 to 60 residues C-terminal to the HEXXH motif.

Neutral endopeptidase has been shown to degrade enkephalins and many other biologically active peptides (lijima H. et al. (1992) Am.J.Physiol. 262:L725-733). Several alternatively spliced forms of NEP-24.11 have been described (for a review, see Rawling N.D. and Barrett A.J. (1993) Biochem.J., 290:205-218). ECE is expressed predominantly in endothelial cells and is responsible for the conversion of big-endothelins into endothelins (Yanagisawa et al. (1988) Nature 332:411-415). Two ECE homologs have been described, named ECE-1 (Xu D. et al. (1994) Cell 78:473-485) and ECE-2 (Emoto N. and Yanagisawa M. (1995) J.Biol.Chem. 270: 15262-15268). Three human variants of ECE-1 having distinct subcellular localization (ECE-1a, ECE-1b and ECE-1c), have also been recently described (Schweizer A. et al. (1997) Biochem.J. 328:871-877). It was shown that in addition to the production of mature endothelins, ECE-1 could also act as a peptidyl dipeptidase in hydrolysing bradikinin, suggesting a broader specificity for such enzymes (Hoang M.V. and Turner A.J. (1997) Biochem.J. 327:23-26).

The erythrocyte cell-surface antigen KELL is classified as a member of the neprilysin family of zinc endopeptidases because of its sequence homology to neprilysin. KELL possesses all of the potential catalytic residues found in the neprilysin familly, but no activity has so far been described for it.

The product of the PEX gene was localized in bone and kidney and has been associated with X-linked hypophosphatemic rickets. This enzyme was recently shown to degrade parathyroid hormone-derived peptides, suggesting its potential implication in metabolic bone diseases (Lipman et al. (1998) J.Biol.Chem. 273:13729-13737).

XCE is a newly described protein consisting of 775 amino-acids and showing sequence homologies with both ECE-1 and NEP (Valdenaire et al. (1999) Mol. Brain Res. 64:211-221). XCE is expressed preferentially in the central nervous system, although its substrate remains to be elucidated.

We have identified a novel transcript that codes for new human members of this family of metallopeptidases, that are mainly expressed in the brain and kidney, but also found in other regions such as heart, lung, pancreas and liver. Since the deduced amino acid sequence of this transcript is most closely related to that of the human neutral endopeptidase-24.11 sequence, we have called these proteins SNEP. Due to alternative splicing, three variants are described and referred to as human SNEPa, SNEPb and SNEPc. The substrates for SNEP have not been identified. However, since the deduced amino acid sequences of these SNEPs are related to the neprilysin family of metalloproteases, this strongly suggests a role for these proteins in peptide maturation and/or degradation.

Activators or inhibitors of these proteins may be used for example, to treat both acute and chronic renal insufficiency, renal and hepatic ischemia, pain, stroke, hypertensive disease, cancer, inflammation, as well as in cardiovascular, neuronal, pancreatic, prostatic, renal, respiratory or hepatic diseases, in modulating peptide activation and/or degradation in brain or kidney or another organ or to diagnose or treat any disorder related to abnormal expression of said SNEPa, SNEPb or SNEPc proteins.

### SUMMARY OF THE INVENTION

The present inventors have found three new membrane metallopeptidases belonging to the neprilysin family of proteins. These new proteins may be responsible for some cardiovascular, hepatic, neuronal, pancreatic, renal or respiratory disorders, or may be targets to regulate abnormal peptide maturation and/or degradation and abnormal function of the metallopeptidases linked to various pathologies.

In accordance with one aspect of the present invention, there is provided novel mature polypeptides which are membrane metallopeptidase proteins, as well as fragments, analogs, other variants or derivatives. The polypeptides of the present invention are of human origin.

In accordance with another aspect of the present invention, there are provided polynucleotides which encode such polypeptides.

In accordance with yet a further aspect of the present invention, there is provided a process for producing such polypeptides by recombinant techniques.

In still another aspect, the invention relates to methods to identify activators and inhibitors using the materials provided by the invention, and means of treatment of physiological and/or pathalogical conditions associated with the variants of SNEP.

Yet another aspect of the invention relates to diagnostic assays for detecting diseases associated with inappropriate SNEPa, SNEPb or SNEPc activity or levels.

These and other aspects of the present invention should be apparent to those skilled in the art from the teachings herein.

### BRIEF DESCRIPTION OF THE FIGURES

The following drawings are illustrative of the embodiments of the invention and are not meant to limit the scope of the invention as encompassed by the claims.

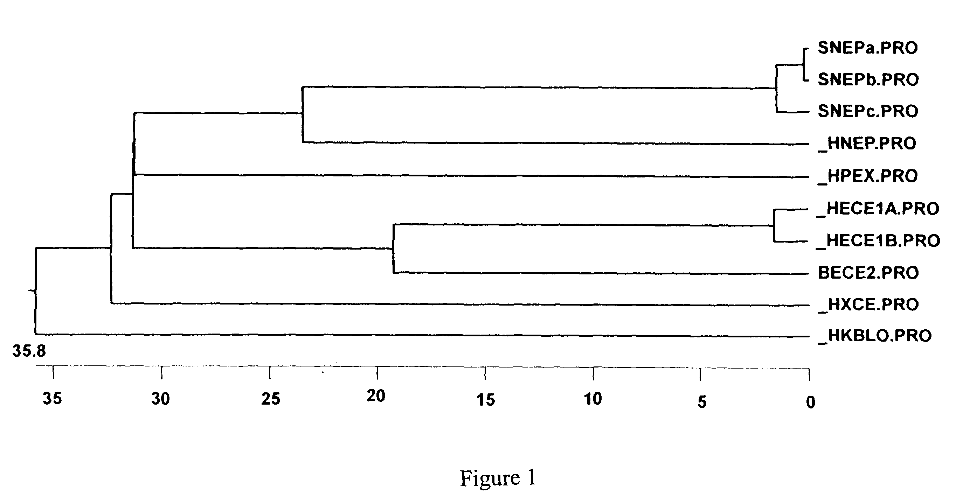

Figure 1 illustrates the phylogenic relationship between SNEPa, SNEPb and SNEPc and other members of the neprilysin family. The length of each of the branches represents the distance between sequence pairs. Alignment was performed with the clustal algorythm of MEGALIGN (version 3.10a) from DNASTAR.
_HNEP: human neutral endopeptidase (accession M81591)
_HKBLO: human KELL blood protein (accession M64934)
_HPEX: human PEX protein (accession U75645)
_HECE1A: human endothelin converting enzyme 1A (accession D29683)
_HECE1B: human endothelin converting enzyme 1B (accession D29683)
_HXCE: human XCE protein (accession Y16187)
BECE2: bovin endothelin converting enzyme 2 (accession U27341)

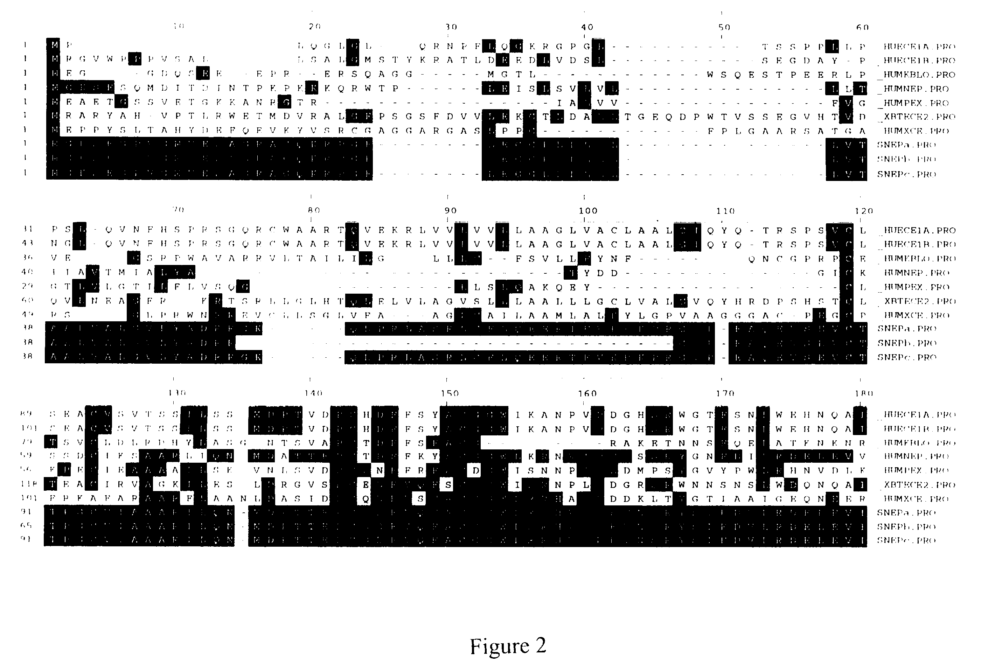

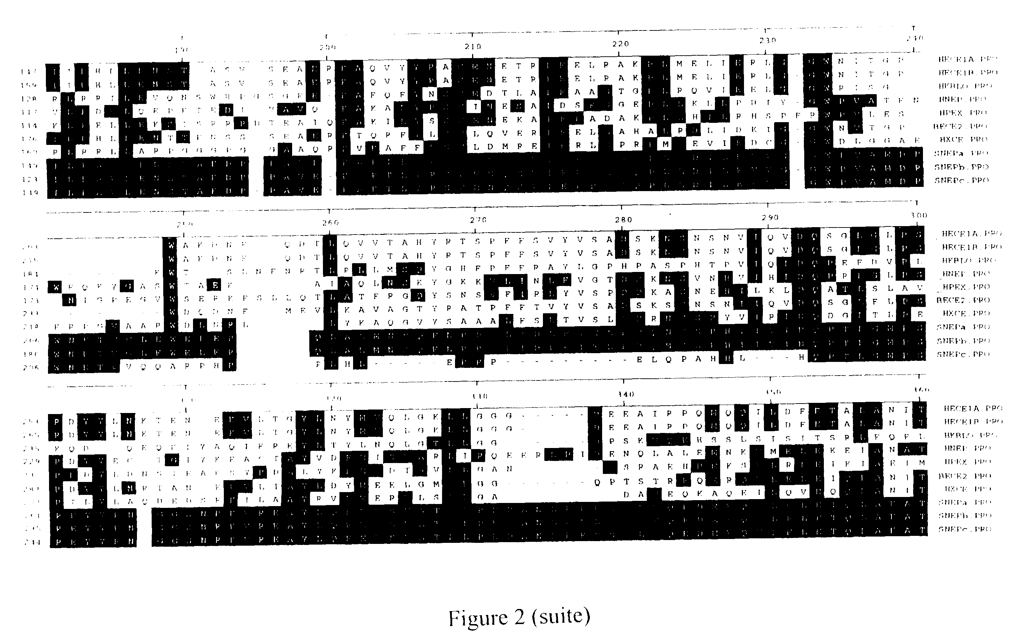

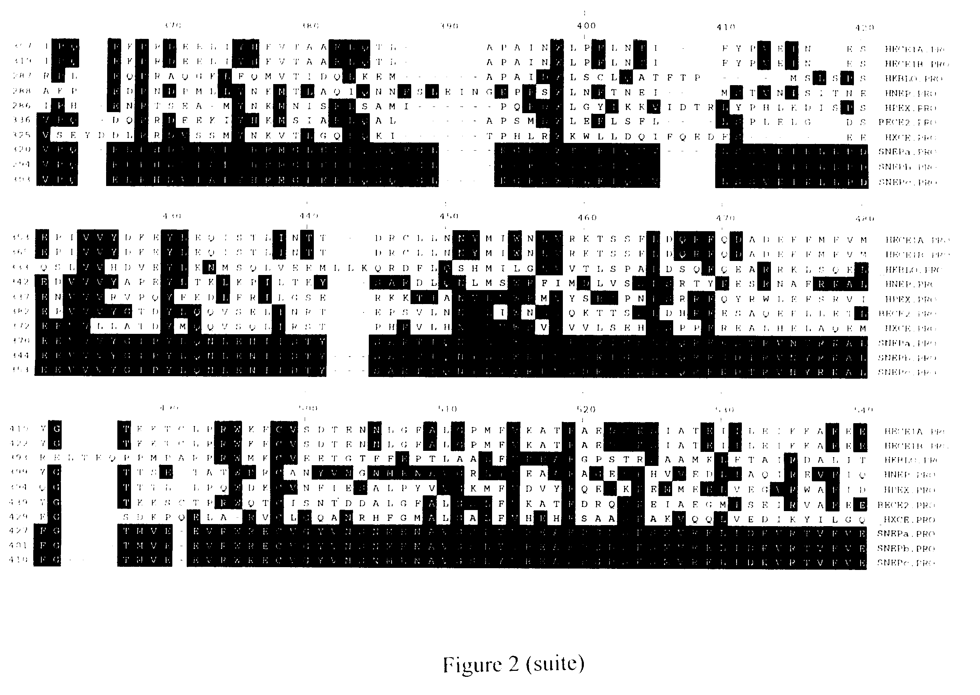

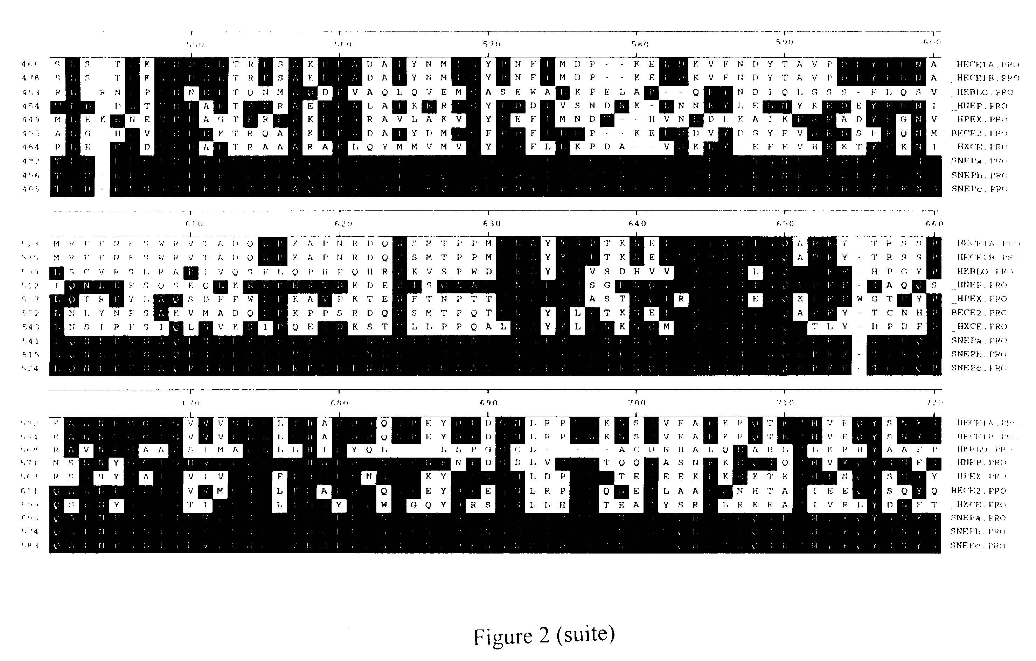

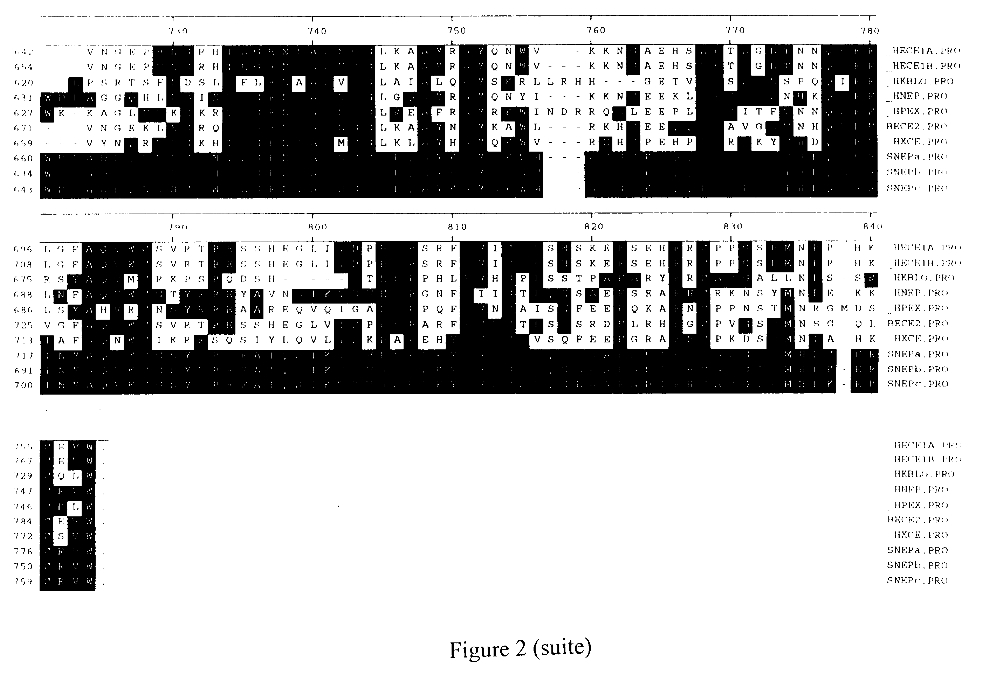

Figure 2 shows an amino acid sequence comparison of the SNEP metallopeptidase variants with other members of the neprilysin family of metallopeptidases, from the phylogenic tree of Figure 1. The alignment was performed with the clustal algorithm of MEGALIGN (version 3.10a ) from DNASTAR. Regions of homology to other members of the membrane metallopeptidase protein family are shaded in black.
_HNEP: human neutral endopeptidase (accession M81591)
_HKBLO: human KELL blood protein (accession M64934)
_HPEX: human PEX protein (accession U75645)
_HECE1A: human endothelin converting enzyme 1A (accession D29683)
_HECE1B: human endothelin converting enzyme 1B (accession D29683)
_HXCE: human XCE protein (accession Y16187)
BECE2: bovin endothelin converting enzyme 2 (accession U27341)

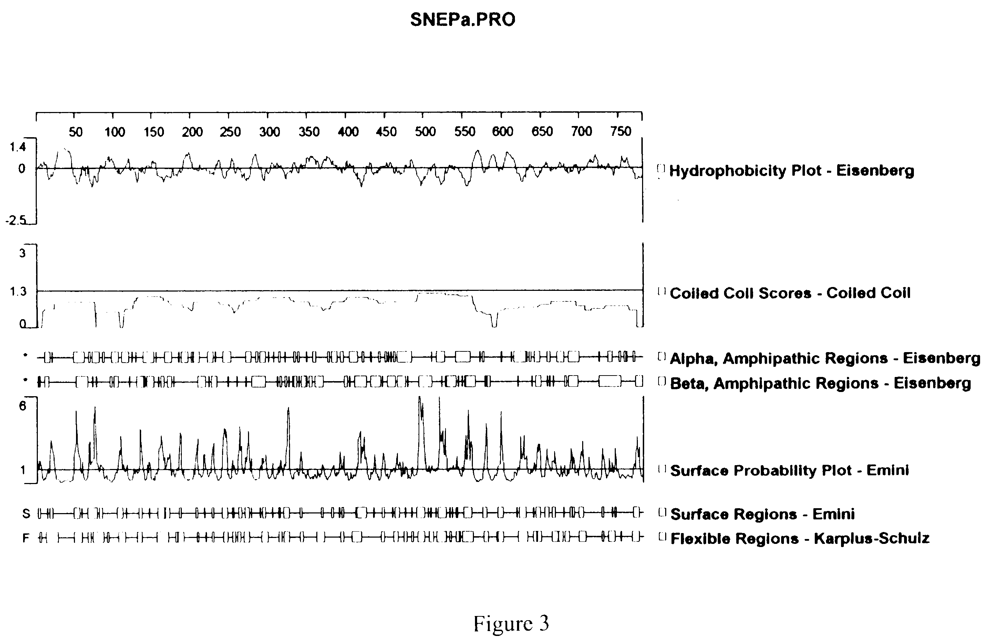

Figure 3 illustrates secondary structural features of SNEPa protein with the hydrophilicity, hydrophobicity, the propensity to generate alpha helices, beta sheets, turns or coiled regions, the propensity to be on the surface of the protein and the flexible regions. The boxed areas are the areas which correspond to the regions indicated. The hydrophobicity plot illustrates hydrophobic areas of the protein sequence which are in the lipid bilayer and hydrophilic areas which are outside the lipid bilayer. The antigenicity of the protein fragments is higher in hydrophilic and flexible areas exposed to the surface. The analysis was performed with PROTEAN (version 3.08a) from DNASTAR.

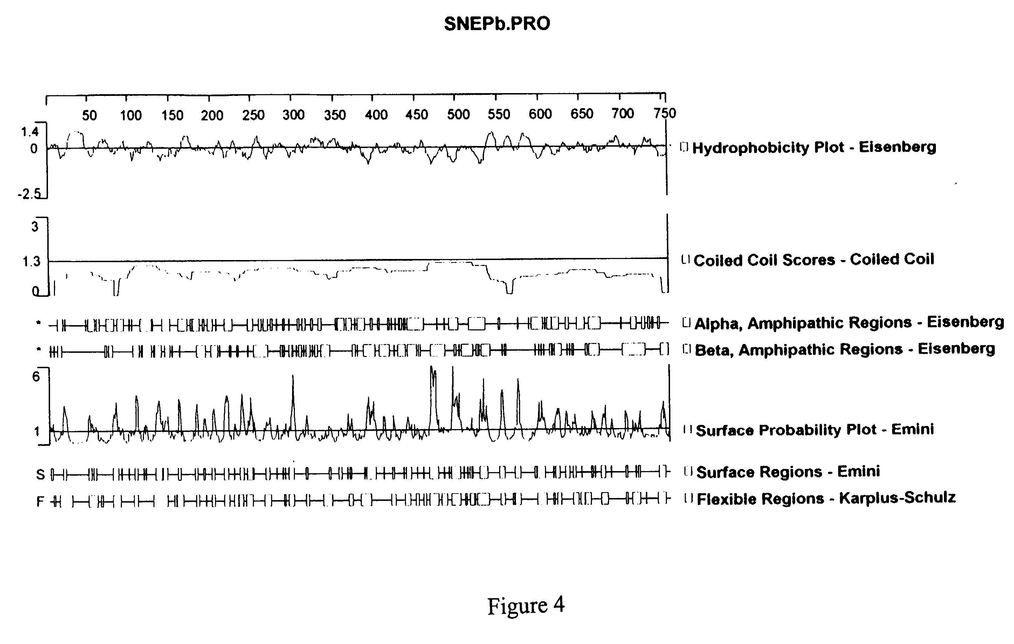

Figure 4 illustrates secondary structural features of SNEPb protein with the hydrophilicity, hydrophobicity, the propensity to generate alpha helices, beta sheets, turns or coiled regions, the propensity to be on the surface of the protein and the flexible regions. The boxed areas are the areas which correspond to the regions indicated. The hydrophobicity plot illustrates hydrophobic areas of the protein sequence which are in the lipid bilayer and hydrophilic areas which are outside the lipid bilayer. The antigenicity of the protein fragments is higher in hydrophilic and flexible areas exposed to the surface. The analysis was performed with PROTEAN (version 3.08a) from DNASTAR.

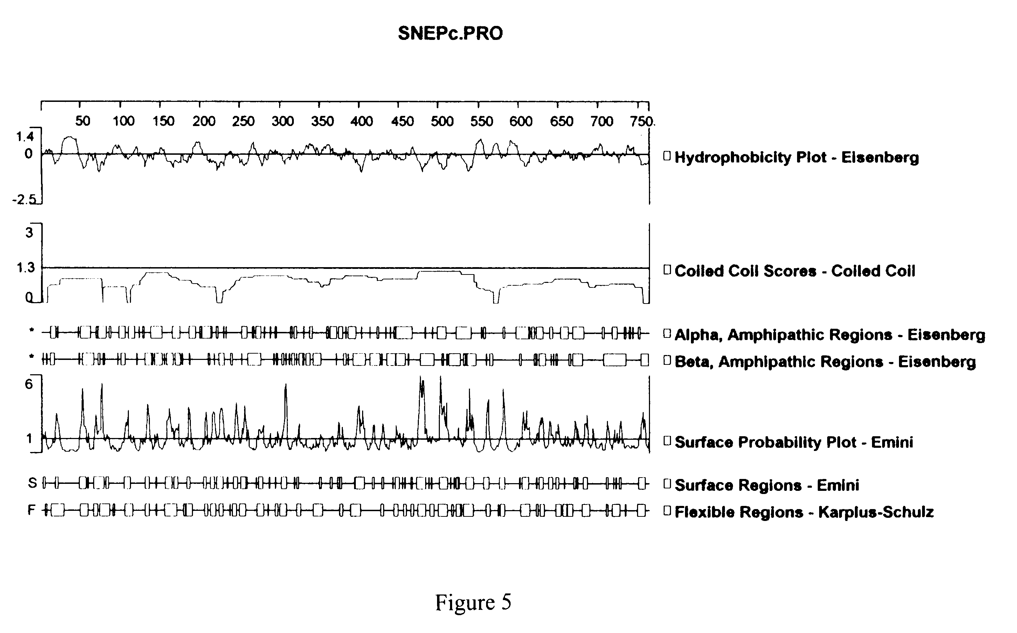

Figure 5 illustrates secondary structural features of SNEPc protein with the hydrophilicity, hydrophobicity, the propensity to generate alpha helices, beta sheets, turns or coiled regions, the propensity to be on the surface of the protein and the flexible regions. The boxed areas are the areas which correspond to the regions indicated. The hydrophobicity plot illustrates hydrophobic areas of the protein sequence which are in the lipid bilayer and hydrophilic areas which are outside the lipid bilayer. The antigenicity of the protein fragments is higher in areas hydrophilic and flexible exposed to the surface. The analysis was performed with PROTEAN (version 3.08a) from DNASTAR.

### DESCRIPTION OF THE INVENTION

### Definitions

The following definitions are provided to facilitate understanding of certain terms used frequently herein.

"Metallopeptidase Activity" or "Biological Activity of the metallopeptidase" refers to the metabolic or physiologic function of said SNEP variants including similar activities or improved activities or these activities with decreased undesirable side-effects. Also included are antigenic and immunogenic activities of said SNEP variants.

"SNEP polypeptide" refers among others to a polypeptide comprising the amino acid sequence set forth in SEQ ID NO : 2, SEQ ID NO : 4 or SEQ ID NO : 6 or an allelic variant thereof.

"SNEP gene" or "SNEP polynucleotide" refer to a polynucleotide comprising the nucleotide sequence set forth in SEQ ID NO : 1, SEQ ID NO : 3 or SEQ ID NO : 5 or variants or allelic variants thereof and/or their complements.

"Antibodies" as used herein include polyclonal and monoclonal antibodies, chimeric, single chain, and humanized antibodies, as well as Fab fragments, including the products of an Fab or other immunoglobulin expression library.

"Isolated" means altered by the hand of man from the natural state. If an "isolated" composition or substance occurs in nature, it has been changed or removed from its original environment, or both. For example, a polynucleotide or a polypeptide naturally present in a living animal is not "isolated" but the same polynucleotide or polypeptide separated from the coexisting materials of its natural state is "isolated", as the term is employed herein.

"Polynucleotide" generally refers to any polyribonucleotide or polydeoxyribonucleotide, which may be unmodified RNA or DNA or modified RNA or DNA. "Polynucleotides" include, without limitation single-and double-stranded DNA, DNA that is a mixture of single-and double-stranded regions, single-and double-stranded RNA, and RNA that is a mixture of single-and double-stranded regions, hybrid molecules comprising DNA and RNA that may be single-stranded or, more typically, double-stranded or a mixture of single-and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS -STRUCTURE AND MOLECULAR PROPERTIES, 2nd Ed., T. E. Creighton, W. H. Freeman and Company, New York, 1993 and Wold, F., Post-translational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al*., "Analysis for protein modifications and nonprotein cofactors", *Meth Enzymol* (1990) 182:626-646 and Rattan *et al*., "Protein Synthesis: Post-translational Modifications and Aging", *Ann NY Acad Sci* (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exists a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCS program package (Devereux, J., *et al*., *Nucleic Acids Research* (1984) 12(1):387), BLASTP, BLASTN, FASTA (Atschul, S.F. *et al., J Molec Biol* (1990) 215:403).

As an illustration, by a polynucleotide having a nucleotide sequence having at least, for example, 95% "identity" to a reference nucleotide sequence of SEQ ID NO : 1, SEQ ID NO : 3 or SEQ ID NO : 5 is intended that the nucleotide sequence of the polynucleotide is identical to the reference sequence except that the polynucleotide sequence may include up to five point mutations per each 100 nucleotides of the reference nucleotide sequence of SEQ ID NO : 1, SEQ ID NO : 3 or SEQ ID NO : 5. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence is intended that the amino acid sequence of the polypeptide is identical to the reference sequence except that the polypeptide sequence may include up to five amino acid alterations per each 100 amino acids of the reference amino acid of SEQ ID NO : 2, SEQ ID NO : 4 or SEQ ID NO : 6. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### Polypeptides of the Invention.

In one aspect, the present invention relates to SNEPa, SNEPb and SNEPc polypeptides. The SNEPa, SNEPb and SNEPc polypeptides include the polypeptides of SEQ ID NO : 2, SEQ ID NO : 4 or SEQ ID NO : 6; as well as polypeptides comprising the amino acid sequences of SEQ ID NO : 2, SEQ ID NO : 4 or SEQ ID NO : 6; and polypeptides comprising the amino acid sequences which have at least 80% identity to that of SEQ ID NO : 2, SEQ ID NO : 4 or SEQ ID NO : 6 over their entire length, and still more preferably at least 90% identity, and even still more preferably at least 95% identity to SEQ ID NO : 2, SEQ ID NO : 4 or SEQ ID NO : 6. Furthermore, those with at least 97-99% are highly preferred. Also included within SNEPa, SNEPb and SNEPc polypeptides are polypeptides having the amino acid sequence which have at least 80% identity to the polypeptide having the amino acid sequence of SEQ ID NO : 2, SEQ ID NO : 4 or SEQ ID NO : 6 over its entire length, and still more preferably at least 90% identity, and even still more preferably at least 95% identity to SEQ ID NO : 2, SEQ ID NO : 4 or SEQ ID NO : 6. Furthermore, those with at least 97-99% are highly preferred. SNEPa, SNEPb and SNEPc polypeptides comprising an amino acid sequence having at least 80% identity, preferably at least 90% identity, and even still more preferably at least 95% to the amino acid sequences encoded by the cDNA contained in pcDNA3/SNEPa, pcDNA3/SNEPb and pcDNA3/SNEPc described hereafter are also included within the present invention. Preferably SNEPa, SNEPb and SNEPc polypeptides exhibit at least one biological activity of a peptidase.

The SNEPa, SNEPb or SNEPc polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the SNEPa, SNEPb or SNEPc polypeptides are also included in the invention. A fragment refers to a polypeptide having an amino acid sequence that entirely is the same as part, but not all, of the amino acid sequence of the aforementioned SNEPa, SNEPb or SNEPc polypeptides. As with SNEPa, SNEPb and SNEPc polypeptides, fragments may be "free-standing" or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of SNEPa, SNEPb or SNEPc polypeptides. In this context "about" includes the particularly recited ranges larger or smaller by several, 5, 4, 3, 2 or 1 amino acids at either extreme or at both extremes.

Preferred fragments include, for example, truncation polypeptides having the amino acid sequence of SNEPa, SNEPb or SNEPc polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate peptidase activity, including those with a similar activity or an improved activity, or with a decreased undesirable activity. Also included are those that are antigenic or immunogenic in an animal, especially in a human.

Preferably, all of these polypeptide fragments retain the biological activity of the peptidases, including antigenic activity. Variants of the defined sequence and fragments also form part of the present invention. Preferred variants are those that vary from the referents by conservative amino acid substitutions i.e., those that substitute a residue with another of like characteristics. Typical such substitutions are among Ala, Val, Leu and lle; among Ser and Thr; among the acidic residues Asp and Glu; among Asn and GIn; and among the basic residues Lys and Arg; or aromatic residues Phe and Tyr. Particularly preferred are variants in which several, 5-10, 1-5, or 1-2 amino acids are substituted, deleted, or added in any combination.

The SNEPa, SNEPb or SNEPc polypeptides of the invention can be prepared in any suitable manner. Such polypeptides include isolated naturally occuring polypeptides, recombinantly produced polypeptides, synthetically produced polypeptides, or polypeptides produced by a combination of these methods. Means for preparing such polypeptides are well understood in the art.

### Polynucleotides of the Invention

Another aspect of the invention relates to SNEPa, SNEPb or SNEPc polynucleotides. SNEPa, SNEPb or SNEPc polynucleotides include isolated polynucleotides which encode the SNEPa, SNEPb or SNEPc polypeptides and fragments, and polynucleotides closely related thereto. More specifically, SNEPa polynucleotides of the invention include a polynucleotide comprising the nucleotide sequence set forth in SEQ ID NO : 1 encoding a SNEPa polypeptide of SEQ ID NO : 2, and polynucleotide having the particular sequence of SEQ ID NO : 1.

SNEPb polynucleotides of the invention include a polynucleotide comprising the nucleotide sequence set forth in SEQ ID NO : 3 encoding a SNEPa polypeptide of SEQ ID NO : 4, and polynucleotide having the particular sequence of SEQ ID NO : 3.

SNEPc polynucleotides of the invention include a polynucleotide comprising the nucleotide sequence set forth in SEQ ID NO : 5 encoding a SNEPa polypeptide of SEQ ID NO : 6, and polynucleotide having the particular sequence of SEQ ID NO : 5.

SNEPa, SNEPb or SNEPc polynucleotides further include a polynucleotide comprising a nucleotide sequence that has at least 80% identity to a nucleotide sequence encoding the SNEPa, SNEPb and SNEPc polypeptides of SEQ ID NO : 2, SEQ ID NO : 4 or SEQ ID NO : 6 respectively over their entire length, and a polynucleotide that is at least 80% identical to that having SEQ ID NO : 1, SEQ ID NO : 3 or SEQ ID NO : 5 respectively over their entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under SNEPa, SNEPb or SNEPc polynucleotides of the invention is a nucleotide sequence which has sufficient identity to a nucleotide sequence contained in SEQ ID NO : 1, SEQ ID NO : 3 or SEQ ID NO : 5 or contained in the cDNA inserts in the plasmids deposited with the ATCC Deposit numbers PTA-187, PTA-189 and 203984 (see herein-below) to hybridize under conditions useable for amplification or for use as a probe or marker. Moreover, SNEPa, SNEPb or SNEPc polynucleotides include a nucleotide sequence which has at least 80% identity to a nucleotide sequence encoding the SNEPa, SNEPb or SNEPc polypeptides expressed by the cDNA inserts deposited at the ATCC with Deposit Numbers PTA-187, PTA-189 and 203984 respectively, and a nucleotide sequence comprising at least 15 contiguous nucleotides of such cDNA inserts. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. The invention also provides polynucleotides which are complementary to all the above SNEPa, SNEPb or SNEPc polynucleotides.

A deposit containing a SNEPa cDNA has been deposited with the American Type Culture Collection (ATCC), 12301 Park Lawn Drive, Rockville, Maryland 20852, USA, on June 08, 1999, and assigned ATCC Deposit Number PTA-187. The deposited material (clone) is a plasmid pcDNA3 which can be obtained from Invitrogen, Inc. containing the full length SNEPa cDNA, referred to as pcDNA3/SNEPa upon deposit. A deposit containing a SNEPb cDNA has been deposited with the American Type Culture Collection (ATCC), 12301 Park Lawn Drive, Rockville, Maryland 20852, USA, on June 08, 1999, and assigned ATCC Deposit Number PTA-189. The deposited material (clone) is a plasmid pcDNA3 which can be obtained from Invitrogen, Inc. containing the full length SNEPb cDNA, referred to as pcDNA3/SNEPb upon deposit. A deposit containing a SNEPc cDNA has been deposited with the American Type Culture Collection (ATCC), 12301 Park Lawn Drive, Rockville, Maryland 20852, USA, on April 30, 1999, and assigned ATCC Deposit Number 203984. The deposited material (clone) is a plasmid pcDNA3 which can be obtained from Invitrogen, Inc. containing the full length SNEPc cDNA, referred to as pcDNA3/SNEPc upon deposit.

The cDNA inserts are located within *Eco*RV/*Not*l sites in the vector. The nucleotide sequence of the polynucleotides contained in the deposited materials, as well as the amino acid sequence of the polypeptides encoded thereby, are controlling in the event of any conflict with any description of sequences herein.

The deposits have been made under the terms of the Budapest Treaty on the international recognition of the deposit of micro-organisms for purposes of patent procedure.

SNEPa, SNEPb and SNEPc of the invention are structurally related to other proteins of the membrane metallopeptidase protein family, as shown by the results of sequencing the cDNA of SEQ ID NO : 1, SEQ ID NO : 3 or SEQ ID NO : 5.

The cDNA sequence of SEQ ID NO : 1 contains an open reading frame (nucleotide number 8 to 2347) encoding a polypeptide of 780 amino acids of SEQ ID NO : 2. The cDNA sequence of SEQ ID NO : 3 contains an open reading frame (nucleotide number 8 to 2269) encoding a polypeptide of 754 amino acids of SEQ ID NO : 4. The cDNA sequence of SEQ ID NO : 5 contains an open reading frame (nucleotide number 8 to 2296) encoding a polypeptide of 763 amino acids of SEQ ID NO : 6.

Amino acid sequences of SEQ ID NO : 2, SEQ ID NO : 4 and SEQ ID NO : 6 have about 50.6, 51.7 and 47.9 % similarity , respectively (using MEGALIGN version 3.10a) in amino acid residues with human NEP-24.11 (Lopez-Nieto et *al*. (1997) J.Biol.Chem. 272, 6471-6478). Nucleotide sequences of SEQ ID NO : 1, SEQ ID NO : 3 and SEQ ID NO : 5 have about 47.1, 47.5 and 46.1 % similarity respectively (using MEGALIGN version 3.10a) in nucleotide with human NEP-24.11 (Lopez-Nieto et *al*. (1997) J.Biol.Chem. 272, 6471-6478).

Amino acid sequences of SEQ ID NO : 2, SEQ ID NO : 4 and SEQ ID NO : 6 have about 35.0, 34.9 and 34.4 % similarity , respectively (using MEGALIGN version 3.10a) in amino acid residues with human ECE-1A (Schweizer A. et al. (1997) Biochem.J. 328:871-877). Nucleotide sequences of SEQ ID NO : 1, SEQ ID NO : 3 and SEQ ID NO : 5 have about 39.1, 38.3 and 38.9 % similarity respectively (using MEGALIGN version 3.10a) in nucleotide with human ECE-1A (Schweizer A. et al. (1997) Biochem.J. 328:871-877).

Amino acid sequences of SEQ ID NO : 2, SEQ ID NO : 4 and SEQ ID NO : 6 have about 34.9, 33.0, 33.9 % similarity , respectively (using MEGALIGN version 3.10a) in amino acid residues with human ECE-1B (Schweizer A. et al. (1997) Biochem.J. 328:871-877). Nucleotide sequences of SEQ ID NO : 1, SEQ ID NO : 3 and SEQ ID NO : 5 have about 38.0, 37.9 and 38.0 % similarity respectively (using MEGALIGN version 3.10a) in nucleotide with human ECE-1B (Schweizer A. et al. (1997) Biochem.J. 328:871-877).

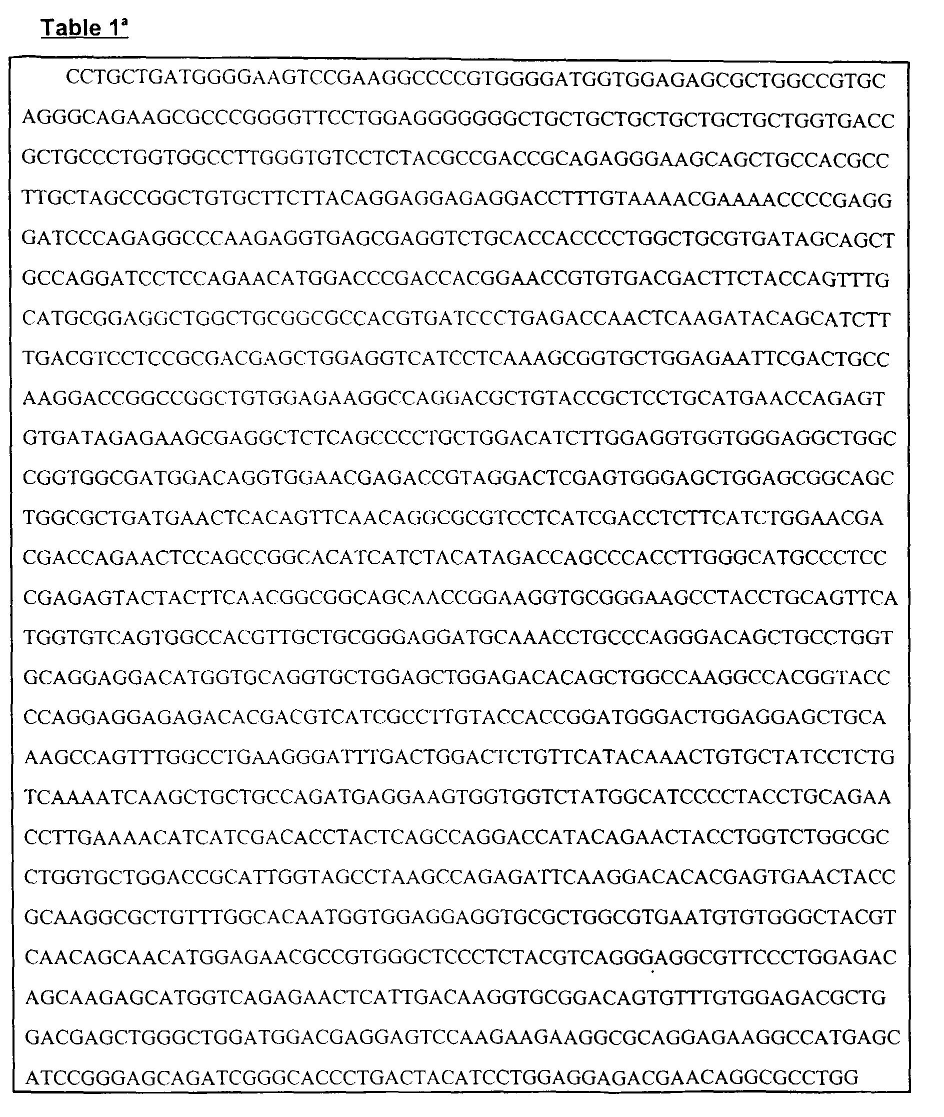

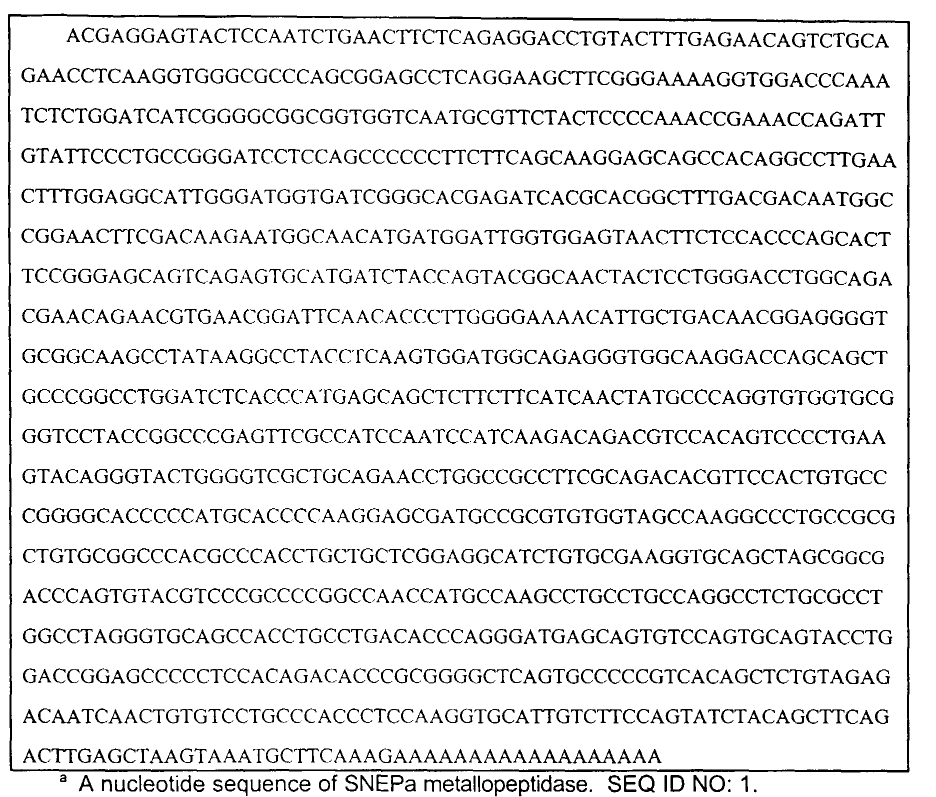

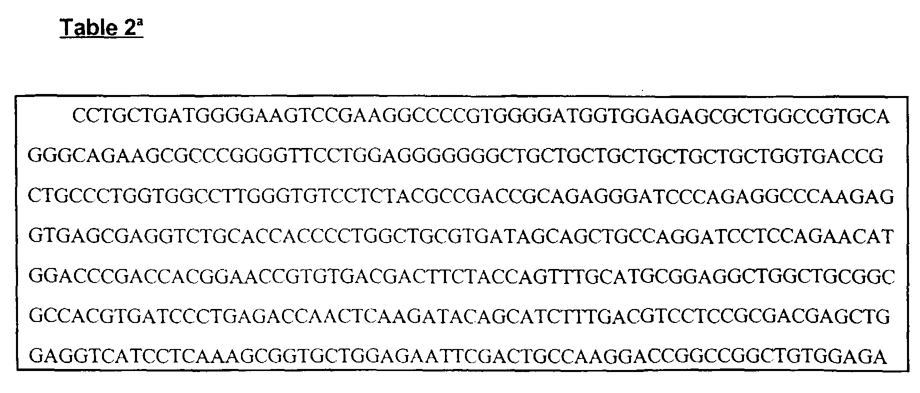

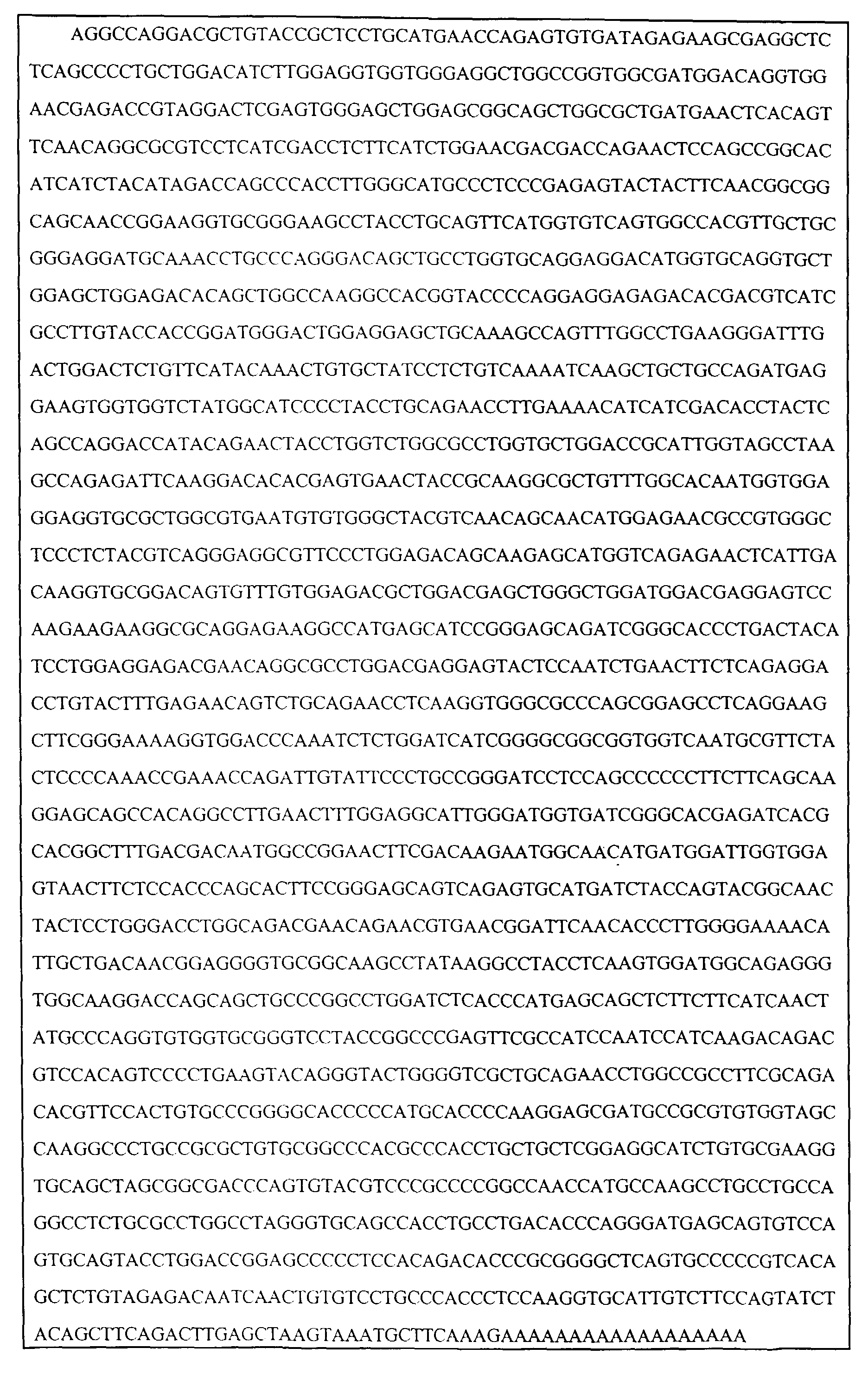

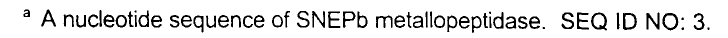

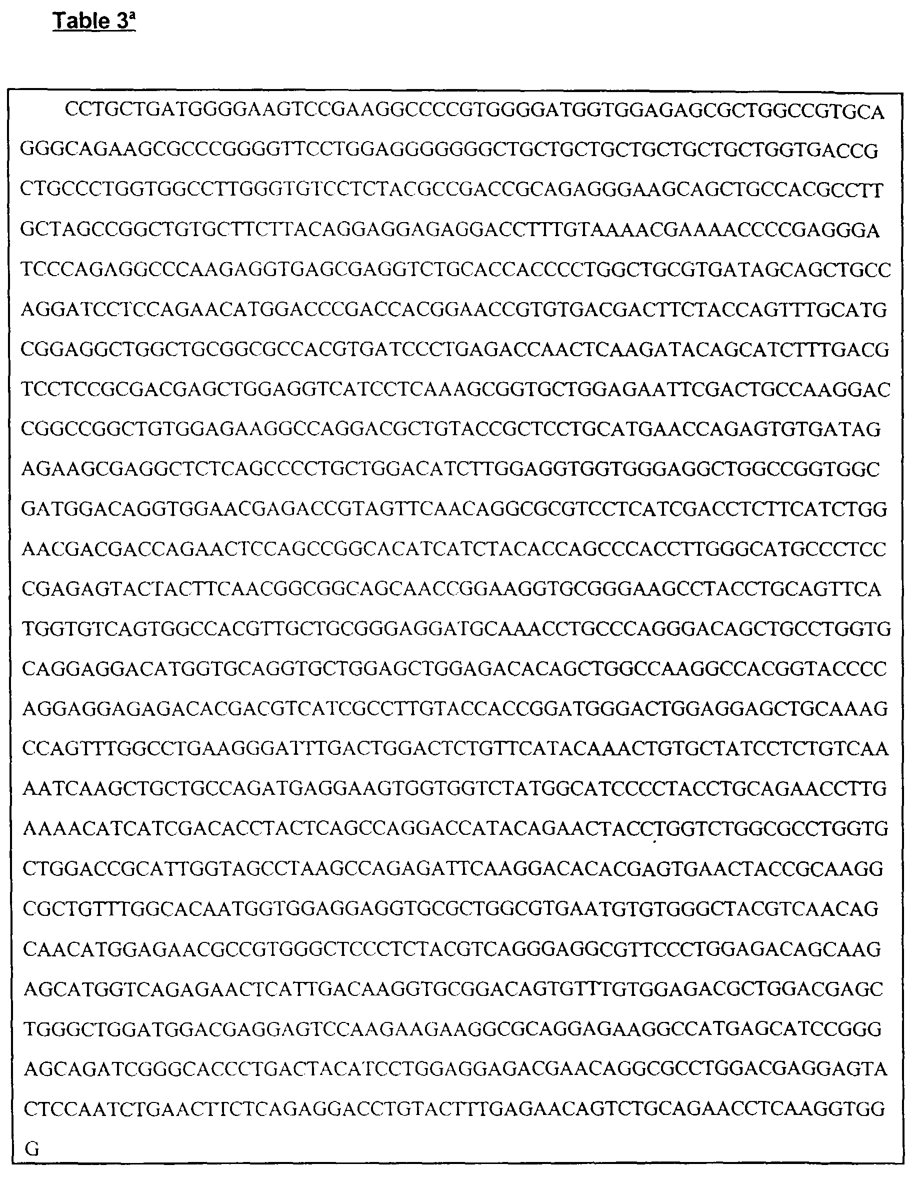

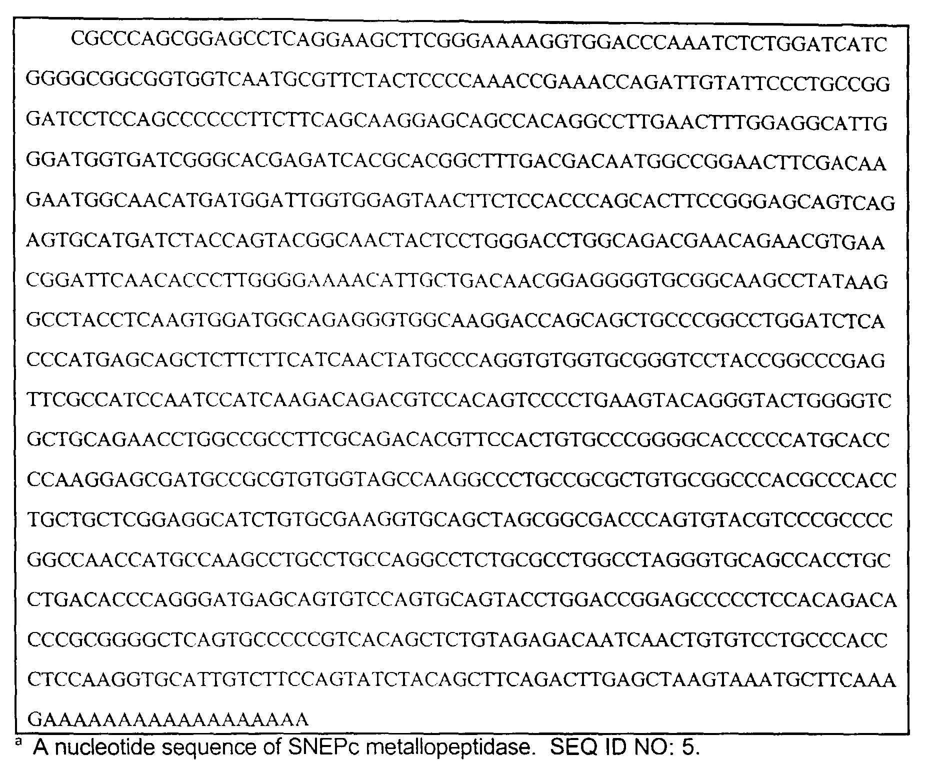

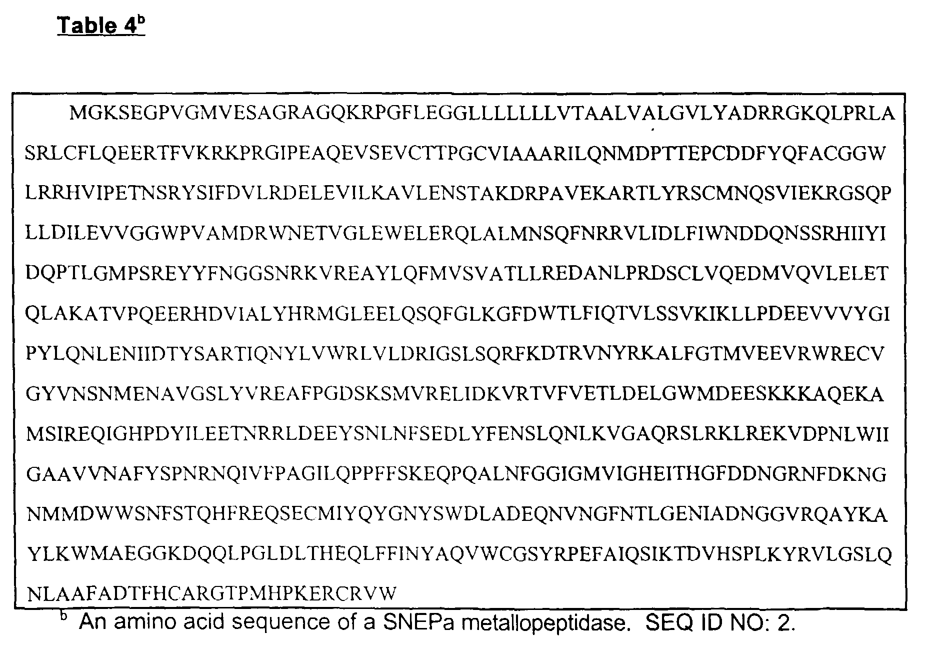

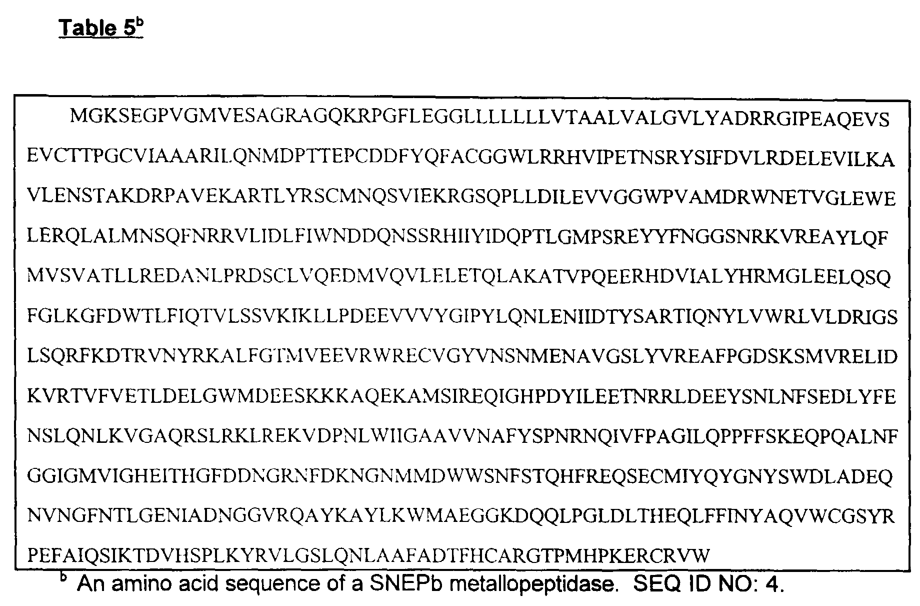

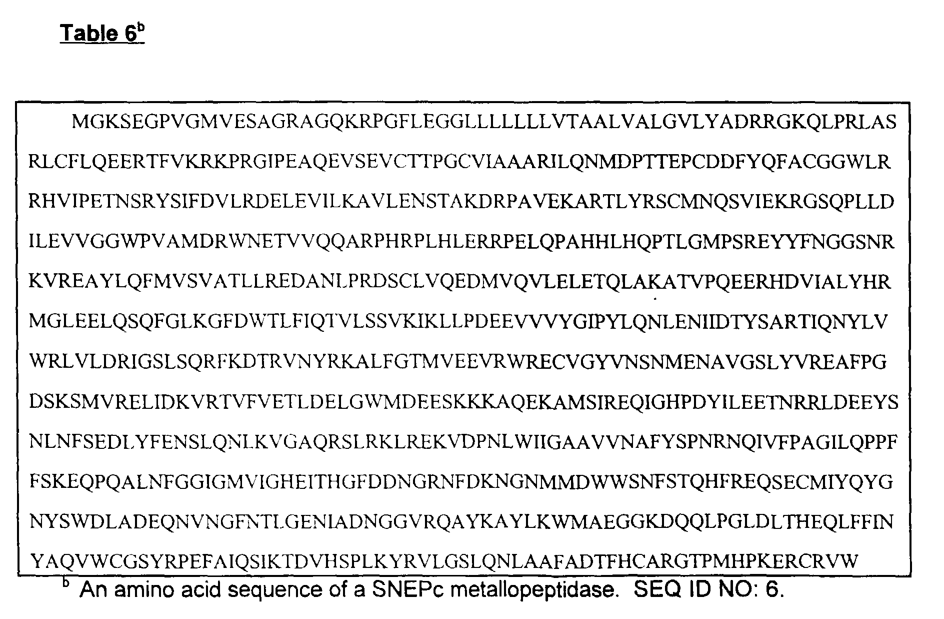

One polynucleotide of the present invention encoding SNEPa, SNEPb or SNEPc may be obtained from a cDNA library derived from mRNA of cells from human brain or kidney using standard cloning and screening methods, or using the expressed sequence tag (EST) analysis (Adams, M.D., et al. Science (1991) 252:1651-1656; Adams, M.D. et al., Nature, (1992) 355:632-634; Adams, M.D., et al., Nature (1995) 377 Supp:3-174). Polynucleotides of the invention can also be obtained from natural sources such as genomic DNA libraries or can be synthesized using well known and commercially available techniques.

The nucleotide sequences encoding SNEPa, SNEPb and SNEPc polypeptides of SEQ ID NO : 2, SEQ ID NO : 4 or SEQ ID NO : 6 may be identical to the polypeptides encoding sequences contained in SEQ ID NO : 1, SEQ ID NO : 3 or SEQ ID NO : 5, or it may be a sequence, which as a result of the redundancy (degeneracy) of the genetic code, also encodes the polypeptides of SEQ ID NO : 2, SEQ ID NO : 4 or SEQ ID NO : 6.

When the polynucleotides of the invention are used for the recombinant production of SNEPa, SNEPb and SNEPc polypeptides, the polynucleotides may include the coding sequence for the mature polypeptides or a fragment thereof, by itself; the coding sequences for the mature polypeptides or fragments in reading frame with other coding sequences, such as those encoding a leader or secretory sequence, a pre-, or pro-or prepro-protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz et al., Proc Natl Acad Sci USA (1989) 86:821-824, or is an HA tag, or a Flag tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding SNEPa, SNEPb and SNEPc variants comprising the amino acid sequence of SNEPa, SNEPb and SNEPc polypeptides of SEQ ID NO : 2, SEQ ID NO : 4 or SEQ ID NO : 6 in which several, 20-50, 10-20, 5-10, 1-5, 1-3, 1-2 or 1 amino acid residues are substituted, deleted or added, in any combination.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

Polynucleotides of the invention, which are identical or sufficiently identical to a nucleotide sequence disclosed in SEQ ID NO : 1, SEQ ID NO : 3 or SEQ ID NO : 5 or a fragment thereof, or to the cDNA inserts in the plasmids deposited at the ATCC with Deposit Numbers PTA-187, PTA-189 and 203984 or a fragment thereof, may be used as hybridization probes for cDNA and genomic DNA, to isolate full-length cDNAs and genomic clones encoding SNEPa, SNEPb or SNEPc and to isolate cDNA and genomic clones of other genes that have a high sequence similarity to the SNEP gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, a method to obtain a polynucleotide encoding SNEPa, SNEPb or SNEPc polypeptides comprises the steps of screening an appropriate library under stringent hybridization conditions with a labeled probe having the SEQ ID NO : 1, the SEQ ID NO : 3, or the SEQ ID NO : 5 or a fragment thereof; and isolating full-length cDNA and genomic clones containing said polynucleotide sequences. Such hybridization techniques are well known to those of skill in the art. Stringent hybridization conditions are as defined above or alternatively conditions under overnight incubation at 42°C in a solution comprising: 50% formamide, 5xSSC (150mM NaCI, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

### Vectors and Host Cells

The present invention also relates to vectors which include the isolated DNA molecules of the present invention, host cells which are genetically engineered with the recombinant vectors, and the production of SNEPa, SNEPb or SNEPc polypeptides or fragments thereof by recombinant techniques.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The DNA insert should be operatively linked to an appropriate promoter, such as the phage lambda PL promoter, the E. coli lac, trp and tac promoters, the SV40 early and late promoters and promoters of retroviral LTRs, to name a few. Other suitable promoters will be known to the skilled artisan. The expression constructs will further contain sites for transcription initiation, termination and, in the transcribed region, a ribosome binding site for translation. The coding portion of the mature transcripts expressed by the constructs will preferably include a translation initiating at the beginning and a termination codon (UAA, UGA or UAG) appropriately positioned at the end of the polypeptide to be translated.

As indicated, the expression vectors will preferably include at least one selectable marker. Such markers include dihydrofolate reductase, zeocin, hygromycin, or neomycin resistance for eukaryotic cell culture and tetracycline or ampicillin resistance genes for culturing in E. coli and other bacteria. Representative examples of appropriate heterologous hosts include, but are not limited to, bacterial cells, such as E. coli, Streptomyces and Salmonella typhimurium cells; fungal cells, such as yeast cells; insect cells such as Drosophila S2 and Spodoptera Sf9 cells; animal cells such as CHO, COS and Bowes melanoma cells; and plant cells. Appropriate culture media and conditions for the above-described host cells are known in the art.

Among vectors preferred for use in bacteria include pQE70, pQE60 and pQE-9, available from Qiagen; pBS vectors, Phagescript vectors, Bluescript vectors, pNH8A, pNH16a, pNH18A, pNH46A, available from Stratagene; ptrc99a, pKK223-3, pKK233-3, pDR540, and pRIT5 available from Pharmacia. Among preferred eukaryotic vectors are pWLNEO, pSV2CAT, pOG44, pXT1 and pSG available from Stratagene; pSVK3, pBPV, pMSG and pSVL available from Pharmacia; pcDNA3 available from InVitrogen. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals, such as Davis et al., Basic Methods In Molecular Biology (1986).

The SNEPa, SNEPb or SNEPc polypeptides may be expressed in a modified form, such as a fusion protein, and may include not only secretion signals, but also additional heterologous functional regions. For instance, a region of additional amino acids, particularly charged amino acids, may be added to the N-terminus of the polypeptides to improve stability and persistence in the host cell, during purification, or during subsequent handling and storage. Also, peptide moieties may be added to the polypeptides to facilitate purification. Such regions may be removed prior to final preparation of the polypeptides. The addition of peptide moieties to polypeptides to engender secretion or excretion, to improve stability and to facilitate purification, among others, are familiar and routine techniques in the art. A preferred fusion protein comprises a heterologous region from immunoglobulin that is useful to solubilize proteins. For example, EP-A-O 464 533 (Canadian counterpart 2045869) discloses fusion proteins comprising various portions of constant region of immunoglobulin molecules together with another human protein or part thereof. In many cases, the Fc part in a fusion protein is thoroughly advantageous for use in therapy and diagnosis and thus results, for example, in improved pharmacokinetic properties (EP-A 0232 262). On the other hand, for some uses it would be desirable to be able to delete the Fc part after the fusion protein has been expressed, detected and purified in the advantageous manner described. This is the case when Fc portion proves to be a hindrance to use in therapy and diagnosis, for example when the fusion protein is to be used as antigen for immunizations. In drug discovery, for example, human proteins, such as hlL5-, has been fused with Fc portions for the purpose of high-throughput screening assays to identify antagonists of hIL-5. See, D. Bennett et al., Journal of Molecular Recognition, Vol. 8:52-58 (1995) and K. Johanson et al., The Journal of Biological Chemistry, Vol. 270, No. 16:9459-9471 (1995).

The SNEP metallopeptidase variants can be recovered and purified from recombinant cell cultures by well-known methods, including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification. Polypeptides of the present invention include naturally purified products, products of chemical synthetic procedures, and products produced by recombinant techniques from a prokaryotic or eukaryotic host, including, for example, bacterial, yeast, higher plant, insect and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes.

### Diagnostic Assays

This invention also relates to the use of SNEPa, SNEPb or SNEPc polynucleotides for use as diagnostic reagents. Detection of a mutated form of SNEP gene associated with a dysfunction will provide a diagnostic tool that can add to or define a diagnosis of a disease or susceptibility to a disease which results from under-expression, over-expression or altered expression of SNEPa, SNEPb or SNEPc. Individuals carrying mutations in the SNEP gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled SNEPa, SNEPb or SNEPc nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers et al., Science (1985) 230:1242. Sequence changes at specific locations may also be revealed by nuclease protection assays, such as RNase and S1 protection or the chemical cleavage method. See Cotton et al., Proc Natl Acad Sci USA (1985) 85: 4397-4401. In another embodiment, an array of oligonucleotides probes comprising SNEPa, SNEPb or SNEPc nucleotide sequences or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee et al., Science, Vol 274, pp 610-613 (1996)).

The diagnostic assays offer a process for diagnosing or determining a susceptibility to the treatment of diseases through detection of mutation in the SNEP gene by the methods described. Said diseases are, in particular, chosen among the following: neuronal diseases, renal diseases, stroke, cardiovascular diseases, hepatic diseases, hypertensive disease, pancreatic diseases, prostatic diseases, respiratory diseases, inflammation, pain, cancer, in modulating drug maturation and/or degradation from the brain or kidney or another organ, to mimic or inhibit effect of some endogenous peptidases in the brain or kidney or another organ disorder related to abnormal expression of SNEPa, SNEPb or SNEPc proteins.

In addition, the same diseases can be diagnosed by methods determining from a sample derived from a subject an abnormally decreased or increased level of SNEPa, SNEPb or SNEPc polypeptides or SNEPa, SNEPb or SNEPc mRNAs. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as SNEPa, SNEPb or SNEPc proteins, in a sample derived from a host are well-known to those of skill in the art. Such assay methods include radioimmunoassays, enzymatic assays, Western Blot analysis and ELISA assays.

### Detection of SNEP Gene Expression

The expression level of the SNEP gene can be readily assayed by one of ordinary skill in the art. By "assaying the expression level of the gene encoding the SNEPa, SNEPb or SNEPc polypeptides" is intended qualitatively or quantitatively measuring or estimating the level of SNEPa, SNEPb or SNEPc polypeptides or the level of the mRNA encoding SNEPa, SNEPb or SNEPc polypeptides in a biological sample (e.g., by determining or estimating absolute protein level or mRNA level).

By "biological sample" is intended any biological sample obtained from an individual, cell line, tissue culture, or other source which contains SNEPa, SNEPb or SNEPc polypeptides or SNEPa, SNEPb or SNEPc mRNAs. Such tissues include cerebellum, brain, midbrain, spinal cord, nerve endings, breast, pituitary, colon, prostate, stomach, testis, adrenal gland, small intestine, spleen, heart, placenta, lung, liver, kidney, and pancreas. Biological samples include mammalian tissues which contain SNEPa, SNEPb or SNEPc polypeptides. Preferred mammals include monkeys, apes, cats, dogs, cows, pigs, horses, rabbits, and humans. Particularly preferred are humans.

Total cellular RNA can be isolated from a biological sample using the single-step guanidinium-thiocyanate-phenol-chloroform method described in Chomczynski and Sacchi (Anal. Biochem. 162:156-159 (1987)). Levels of mRNAs encoding the SNEP metallopeptidase variants are then assayed using any appropriate method. These include Northern blot analysis (Harada et al., Cell 63:303-312 (1990)), S1 nuclease mapping (Harada et al., Cell 63:303-312 (1990)), the polymerase chain reaction (PCR), reverse transcription in combination with the polymerase chain reaction (RT-PCR) (Fujita et al., Cell 49:35-36 (1990)), and reverse transcription in combination with the ligase chain reaction (RT-LCR).

As discussed here-above, assaying SNEPa, SNEPb or SNEPc polypeptides levels in a biological sample can occur using antibody-based techniques. For example,SNEPa, SNEPb or SNEPc polypeptides expression in tissues can be studied with classical immunohistological methods (Jalkanen, M. et al., J. Cell. Biol. 101:976-985 (1985); Jalkanen, M. et al., J. Cell. Biol. 105: 3087-3096 (1987)). Other antibody-based methods useful for detecting SNEPa, SNEPb or SNEPc polypeptides gene expression include immunoassays, such as the enzyme linked immunosorbent assay (ELISA) and the radioimmunoassay (RIA). Suitable labels are known in the art and include enzyme labels, such as glucose oxidase, and radioisotopes, such as iodine (¹²⁵I, ¹²¹I), carbon (¹⁴C), sulfur (³⁵S), tritium (³H), indium (¹¹²In), and technetium (^{99m}Tc), biotin and fluorescent labels, such as fluorescein and rhodamine.

### Chromosome Assays

The nucleic acid molecules of the present invention are also valuable for chromosome identification. The sequence is specifically targeted to and can hybridize with a particular location on an individual human chromosome. The mapping of DNAs to chromosomes according to the present invention is an important first step in correlating those sequences with genes associated with disease.

In certain preferred embodiments in this regard, the cDNA herein disclosed is used to clone genomic DNA of SNEPa, SNEPb or SNEPc polypeptides gene. This can be accomplished using a variety of well known techniques and libraries, which generally are available commercially. The genomic DNA then is used for *in situ* chromosome mapping using well known techniques for this purpose.

In addition, in some cases, sequences can be mapped to chromosomes by preparing PCR primers (preferably 15-25 bp) from the cDNA. Computer analysis of the 3' untranslated region of the gene is used to rapidly select primers that do not span more than one exon in the genomic DNA, thus complicating the amplification process. These primers are then used for PCR screening of somatic cell hybrids containing individual human chromosomes.

Fluorescence *in situ* hybridization ("FISH") of a cDNA clone to a metaphase chromosomal spread can be used to provide a precise chromosomal location in one step. This technique can be used with probes from the cDNA as short as 50 or 60 bp. For a review of this technique, see Verma et al., Human Chromosomes: A Manual Of Basic Techniques, Pergamon Press, New York (1988).

Once a sequence has been mapped to a precise chromosomal location, the physical position of the sequence on the chromosome can be correlated with genetic map data. Such data are found, for example, in V. McKusick, Mendelian Inheritance In Man, available on-line through Johns Hopkins University, Welch Medical Library. The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

Having generally described the invention, the same will be more readily understood by reference to the following examples, which are provided by way of illustration and are not intended as limiting.

### Antibodies

The polypeptides of the invention or their fragments or analogs thereof, or cells expressing them can also be used as immunogens to produce antibodies immunospecific for SNEPa, SNEPb or SNEPc polypeptides. The term "immunospecific" means that the antibodies have substantiall greater affinity for the polypeptides of the invention than their affinity for other related polypeptides in the prior art.

Antibodies generated against SNEPa, SNEPb or SNEPc polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., Nature (1975) 256:495-497), the trioma technique, the human B-cell hybridoma technique (Kozbor et al., Immunology Today (1983) 4:72) and the EBV-hybridoma technique (Cole et al., MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

Non-limiting examples of polypeptides or peptides that can be used to generate SNEPa, SNEPb or SNEPc polypeptide-specific antibodies include: a polypeptide comprising amino acid residues from about 1 to about 28 of SEQ ID NO : 2 ; a polypeptide comprising amino acid residues from about 48 to about 61 of SEQ ID NO : 2; a polypeptide comprising amino acid residues from about 67 to about 94 of SEQ ID NO : 2; a polypeptide comprising amino acid residues from about 104 to about 117 of SEQ ID NO : 2 ; a polypeptide comprising amino acid residues from about 128 to about 148 of SEQ ID NO : 2; a polypeptide comprising amino acid residues from about 156 to about 172 of SEQ ID NO : 2; a polypeptide comprising amino acid residues from about 175 to about 187 of SEQ ID NO: 2; a polypeptide comprising amino acid residues from about 205 to about 233 of SEQ ID NO : 2; a polypeptide comprising amino acid residues from about 255 to about 276 of SEQ ID NO : 2; a polypeptide comprising amino acid residues from about 288 to about 308 of SEQ ID NO : 2; a polypeptide comprising amino acid residues from about 336 to about 351 of SEQ ID NO : 2; a polypeptide comprising amino acid residues from about 404 to about 427 of SEQ ID NO : 2; a polypeptide comprising amino acid residues from about 444 to about 451 of SEQ ID NO : 2; a polypeptide comprising amino acid residues from about 459 to about 477 of SEQ ID NO : 2; a polypeptide comprising amino acid residues from about 487 to about 511 of SEQ ID NO : 2; a polypeptide comprising amino acid residues from about 514 to about 537 of SEQ ID NO : 2; a polypeptide comprising amino acid residues from about 551 to about 562 of SEQ ID NO : 2; a polypeptide comprising amino acid residues from about 575 to about 582 of SEQ ID NO : 2; a polypeptide comprising amino acid residues from about 617 to about 632 of SEQ ID NO : 2; a polypeptide comprising amino acid residues from about 644 to about 671 of SEQ ID NO : 2; a polypeptide comprising amino acid residues from about 677 to about 689 of SEQ ID NO : 2; a polypeptide comprising amino acid residues from about 697 to about 711 of SEQ ID NO : 2; a polypeptide comprising amino acid residues from about 726 to about 749 of SEQ ID NO : 2; a polypeptide comprising amino acid residues from about 764 to about 779 of SEQ ID NO : 2; and a polypeptide comprising amino acid residues from about 214 to about 230 of SEQ ID NO : 6. As indicated above, the inventors have determined that the above polypeptide fragments are antigenic regions of SNEPa, SNEPb or SNEPc polypeptides.

The above-mentioned antibodies may be employed to isolate or to identify clones expressing the polypeptide or to purify the polypeptides by affinity chromatography.

Antibodies against SNEPa, SNEPb or SNEPc polypeptides may also be employed to treat, among others, patients with neuronal diseases, renal diseases, stroke, cardiovascular diseases, hepatic diseases, hypertensive disease, pancreatic diseases, respiratory diseases, inflammation, pain, cancer, in modulating drug maturation and/or degradation from brain or kidney or another organ or to treat any disorder related to abnormal expression of said SNEPa, SNEPb or SNEPc polypeptides.

### Vaccines

Another aspect of the invention relates to a method for inducing an immunological response in a mammal which comprises inoculating the mammal with SNEPa, SNEPb or SNEPc polypeptides, or a fragment thereof, adequate to produce antibody and/or T cell immune response to protect said animal from, among others, patients with neuronal diseases, renal diseases, stroke, cardiovascular diseases, hepatic diseases, hypertensive disease, pancreatic diseases, respiratory diseases, inflammation, pain, cancer, in modulating drug maturation and/or degradation from brain or kidney or another organ or to treat any disorder related to abnormal expression of said SNEPa, SNEPb or SNEPc polypeptides.

Yet another aspect of the invention relates to a method of inducing immunological response in a mammal which comprises, delivering SNEPa, SNEPb or SNEPc polypeptides via a vector directing expression of SNEPa, SNEPb or SNEPc polynucleotides in vivo in order to induce such an immunological response to produce antibody to protect said animal from diseases.

Further aspect of the invention relates to an immunological/vaccine formulation (composition) which, when introduced into a mammalian host, induces an immunological response in that mammal to SNEPa, SNEPb or SNEPc polypeptides wherein the composition comprises SNEPa, SNEPb or SNEPc polypeptides or SNEP gene. The vaccine formulation may further comprise a suitable carrier. Since SNEPa, SNEPb or SNEPc polypeptides may be broken down in the stomach, it is preferably administered parenterally (including subcutaneous, intramuscular, intravenous or intradermal injection). Formulations suitable for parenteral administration include aqueous and non-aqueous sterile injection solutions which may contain anti-oxidants, buffers, bacteriostats and solutes which render the formulation isotonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

### Screening Assays

SNEPa, SNEPb or SNEPc polypeptides of the present invention may be employed in a screening process for compounds which bind the peptidase and which activate (activators) or inhibit activation (inhibitors) of the enzyme polypeptides of the present invention. Thus, polypeptides of the invention may also be used to assess the enzymatic cleavage of small molecule substrates in, for example, cells, cell-free preparations, chemical libraries, and natural product mixtures. These substrates may be natural substrates or may be structural or functional mimetics. See Coligan et al., Current Protocols in Immunology 1 (2):Chapter 5 (1991).

SNEPa, SNEPb or SNEPc polypeptides may be responsible for many biological functions, including many pathologies. Accordingly, it is desirous to find compounds and drugs which stimulate SNEPa, SNEPb or SNEPc on the one hand or which can inhibit the function of SNEPa, SNEPb or SNEPc on the other hand. In general, activators are employed for therapeutic and prophylactic purposes for such conditions as, among others to treat patients with brain disorders, renal disorders, pain, hypertensive disease, stroke, cardiac diseases, pancreatic disease, respiratory disease, cancer and pain, in modulating peptide maturation and/or degradation from brain or kidney or another organ or to treat any disorder related to abnormal expression of said SNEPa, SNEPb or SNEPc polypeptides.

Inhibitors may be employed for a variety of therapeutic and prophylactic purposes for such conditions as, among others, brain disorders, renal disorders, hypertensive disease, stroke, cardiac diseases, pancreatic disease, respiratory disease, cancer and pain, in modulating peptide maturation and/or degradation from brain or kidney or another organ or to treat any disorder related to abnormal expression of said SNEPa, SNEPb or SNEPc polypeptide.

In general, such screening procedures involve producing appropriate cells which express the enzyme polypeptide of the present invention on the surface thereof. Such cells include cells from mammals, yeast, Drosophila or E. coli. Cells expressing the enzyme (or cell membrane containing the expressed enzyme) are then contacted with a test compound to observe maturation and/or degradation of substrates, or stimulation or inhibition of a functional response.

The assays may simply test binding or enzymatic degradation of a candidate compound wherein adherence to the cells bearing the enzyme is detected by means of a label directly or indirectly associated with the candidate compound or in an assay involving competition with a labeled competitor. Further, these assays may test whether the candidate compound results in a signal generated by activation of the enzyme, using detection systems appropriate to the cells bearing the enzyme at their surfaces. Inhibitors of activation are generally assayed in the presence of a known substrate and the effect on activation by the substrate by the presence of the candidate compound is observed. Standard methods for conducting such screening assays are well understood in the art.

Examples of potential SNEPa, SNEPb or SNEPc inhibitors include drugs, antibodies or, in some cases, oligonucleotides or proteins which are closely related to the substrate of SNEPa, SNEPb or SNEPc, e.g., a fragment of the substrate, or small molecules which bind to the enzymes but do not elicit a response, so that the activity of the enzymes is prevented.

### Prophylactic and Therapeutic Methods

The invention provides methods of treating abnormal conditions related to both an excess of and insufficient amounts of SNEPa, SNEPb or SNEPc activity.

If the activity of SNEPa, SNEPb or SNEPc is in excess, several approaches are available. One approach comprises administering to a subject an inhibitor compound as hereinabove described along with a pharmaceutically acceptable carrier in an amount effective to inhibit activation by blocking binding of substrate to SNEPa, SNEPb or SNEPc, or by inhibiting a catalytic event, and thereby alleviating the abnormal condition.

In another approach, soluble forms of SNEPa, SNEPb or SNEPc polypeptides still capable of binding and/or degrading the substrate in competition with endogenous SNEPa, SNEPb or SNEPc may be administered. Typical embodiments of such competitors comprise fragments of SNEPa, SNEPb or SNEPc polypeptides.

In still another approach, expression of the gene encoding endogenous SNEPa, SNEPb or SNEPc can be inhibited using expression blocking techniques. Known such techniques involve the use of antisense sequences, either internally generated or separately administered. See, for example, O'Connor, J Neurochem (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee et al., Nucleic Acids Res (1979) 6:3073; Cooney et al., Science (1988) 241:456; Dervan et al., Science (1991) 251:1360. These oligomers can be administered per se or the relevant oligomers can be expressed in vivo.

For treating abnormal conditions related to an under-expression of SNEPa, SNEPb or SNEPc and their activity, several approaches are also available. One approach comprises administering to a subject a therapeutically effective amount of a compound which activates SNEPa, SNEPb or SNEPc, i.e., an activator as described above; in combination with a pharmaceutically acceptable carrier, to thereby alleviate the abnormal condition. In another approach, soluble forms of SNEPa, SNEPb or SNEPc polypeptides still capable of binding and/or degrading the substrate in competition with endogenous SNEPa, SNEPb or SNEPc may be administered. Typical embodiments of such competitors comprise fragments of SNEPa, SNEPb or SNEPc polypeptides. Alternatively, gene therapy may be employed to effect the endogenous production of SNEPa, SNEPb or SNEPc by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells in vivo and expression of the polypeptide in vivo. For overview of gene therapy, see Chapter 20, Gene Therapy and other Molecular Genetic-based Therapeutic Approaches, (and references cited therein) in Human Molecular Genetics, T Strachan and A P Read, BIOS Scientific Publishers Ltd (1996).

### Formulation and administration.

Peptides, such as the soluble form of SNEPa, SNEPb or SNEPc polypeptides, and activator and inhibitor peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 mg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide ex vivo, and for example, by the use of a retroviral plasmid vector. The cells are then introduced into the subject.

### Examples

The examples below are carried out using standard techniques, which are well known and routine to those of skill in the art, except where otherwise described in detail. The examples illustrate, but do not limit the invention.

### Example 1

### Cloning the human membrane metallopeptidase variants.

The sequence of the SNEP was first identified by searching in a private database containing over 2 million human ESTs, which was generated using high throughput automated DNA sequence analysis of randomly selected human cDNA clones (Adams, M.D. et al., Nature 377:3-174 (1995); Adams, M.D. et al., Nature 355:632-634 (1992); and Adams, M.D. et al., Science 252:1651-1656 (1991)). Sequence homology comparisons of each EST were performed against the GenBank database using the blastn and tblastn algorithms (Altschul, S.F. et al., J. Mol. Biol. 215:403-410 (1990)). A specific homology search using the known human NEP-24.11 amino acid sequence against this human EST database revealed one EST, named HGS459814, from a human adult small intestine cDNA library, with approximatively 61.2% similarity to the human NEP-24.11. This EST clone, HGS459814, contains an insert of 1.15 kb and the sequence comparison suggested that it contained a partial region of the C-terminal part of the open reading frame of a new metallopeptidase belonging to the neprilysin family (M13) (Rawling N.D. and Barrett A.J. (1993) Biochem.J., 290:205-218). In order to amplify the missing N-terminal part of the SNEP coding region, 5'RACE experiments were performed using human kidney and human total brain Marathon-ready cDNAs (Clontech Inc.) as template and a KlenTaq DNA polymerase (Clontech Inc.).

A first amplification product of 1.1 Kb was cloned into pT-Adv vector (Clontech Inc.) and sequenced to verify its specificity. Based on this new sequence, a second round of 5'RACE experiments was performed as described above to generate amplification products of 1 kb which were sequenced. Using the sequences thus obtained, a new PCR experiment using Pfu DNA polymerase (Stratagene Inc.) was performed to amplify the entire SNEP coding sequence. Several PCR products ranging from 2.5 to 2.6 kb were generated and sequenced. Sequencing of several cDNAs showed the existence of at least three variants as indicated in SEQ ID NO : 1, SEQ ID NO : 3 and SEQ ID NO : 5. These variants were called SNEPa, SNEPb and SNEPc. These cDNAs were inserted into the *Eco*RV/*Not*l restriction sites of the expression vector pcDNA3 (Invitrogen, Inc). The constructs were named pcDNA3/SNEPa, pcDNA3/SNEPb and pcDNA3/SNEPc.

These sequences were confirmed by double strand DNA sequencing and the cDNAs were shown to be completely new by a blast search against Genbank release 111.

### Example 2

### Cloning and Expression of SNEPa, SNEPb and SNEPc in Mammalian Cells.

A typical mammalian expression vector contains the promoter element, which mediates the initiation of transcription of mRNA, the protein coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRS) from Retroviruses, e.g., RSV, HTLVI, HIVI and the early promoter of the cytomegalovirus (CMV). However, cellular elements can also be used (e.g., the human actin promoter). Suitable expression vectors for use in practicing the present invention include, for example, vectors such as PSVL and PMSG (Pharmacia, Uppsala, Sweden), pRSVcat (ATCC 37152), pSV2dhfr (ATCC 37146) and pBC12MI (ATCC 67109) and pcDNA3(-) (Invitrogen). Mammalian host cells that could be used include, human Hela, HEK 293, H9 and Jurkat cells, mouse NIH3T3 and C127 cells, Cos 1, Cos 7 and CV 1, quail QC1-3 cells, mouse L cells and Chinese hamster ovary (CHO) cells.

Alternatively, the gene can be expressed in stable cell lines that contain the gene integrated into a chromosome. The co-transfection with a selectable marker such as dhfr, gpt, neomycin, zeocin or hygromycin allows the identification and isolation of the transfected cells.

The transfected gene can also be amplified to express large amounts of the encoded protein. The DHFR (dihydrofolate reductase) marker is useful to develop cell lines that carry several hundred or even several thousand copies of the gene of interest. Another useful selection marker is the enzyme glutamine synthase (GS) (Murphy et al., Biochem. J. 227:277-279 (1991); Bebbington et al., Bio/Technology 10:169-175 (1992)). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

The expression vector pcDNA3(-) contains the strong promoter (CMV) of the Cytomegalovirus. Multiple cloning sites, e.g., with the restriction enzyme cleavage sites EcoRV and Notl, facilitate the cloning of the gene of interest. The vectors contain in addition the 3' intron, the polyadenylation and termination signal of the bovine growth hormone gene.

### Example 2(a)

### Cloning and Expression in COS Cells.

The expression plasmids pcDNA3/SNEPa, pcDNA3/SNEPb or pcDNA3/SNEPc are made by cloning a cDNA encoding SNEPa to c into the expression vector pcDNA3(-) (which can be obtained from Invitrogen, Inc.).

The expression vector pcDNA3(-) contains: (1) an E. coli origin of replication effective for propagation in E. coli and other prokaryotic cells; (2) an ampicillin resistance gene for selection of plasmid-containing prokaryotic cells; (3) an SV40 origin of replication for propagation in eukaryotic cells; (4) a CMV promoter, a polylinker ; (5) a polyadenylation from the bovine growth hormone gene arranged so that a cDNA can be conveniently placed under expression control of the CMV promoter and operably linked to the polyadenylation signal by means of restriction sites in the polylinker. pcDNA3(-) contains, in addition, the selectable neomycin marker.

A DNA fragment encoding the SNEPa or SNEPb or SNEPc polypeptide is cloned into the polylinker region of the vector so that recombinant protein expression is directed by the CMV promoter. The plasmid construction strategy is as follows. The SNEPa or SNEPb or SNEPc cDNA is first cloned in the pBluescript vector (Stratagene), it is then excised from the pBluescript vector with EcoRV and Notl. The vector, pcDNA3(-), is digested with EcoRV and Notl. The digested SNEPa or SNEPb or SNEPc cDNA fragment and the linearized vector are then ligated. The ligation mixture is transformed into E. coli strain DH5alpha (available from GIBCO BRL), and the transformed culture is plated on ampicillin media plates which then are incubated to allow growth of ampicillin resistant colonies. Plasmid DNA is isolated from resistant colonies and examined by restriction analysis or other means for the presence of the SNEP-encoding fragment.

For expression of recombinant SNEPa or SNEPb or SNEPc polypeptides, COS cells are transfected with the above described pcDNA3/SNEPa, pcDNA3/SNEPb or pcDNA3/SNEPc expression vectors, using DEAE-Dextran, as described, for instance, in Sambrook et al., Molecular Cloning: a Laboratory Manual, Cold Spring Laboratory Press, Cold Spring Harbor, New York (1989), or lipofectamine (available from GIBCO BRL). Cells are incubated under conditions for expression of SNEPa, SNEPb or SNEPc by the cells.

### Example 2(b)

### Cloning and Expression in HEK293 Cells.

The vectors pcDNA3/SNEPa, pcDNA3/SNEPb or pcDNA3/SNEPc are used for the expression of SNEPa, SNEPb or SNEPc proteins. Plasmids pcDNA3/SNEPa, pcDNA3/SNEPb or pcDNA3/SNEPc are described in examples 1 and 2. The plasmids contain the mouse neomycin resistance gene under control of the SV40 early promoter. HEK293 Cells transfected with these plasmids can be selected by growing the cells in a selective medium (containing 1 mg/ml Geneticin, Life Technologies). Cells grown in presence of 1 mg/ml concentrations of Geneticin develop resistance to the drug by producing the target enzyme, Neomycin Resistance. If a second gene is linked to the Neomycin Resistance gene, it is usually co-expressed. It is known in the art that this approach may be used to develop cell lines expressing large amounts of the protein of interest, up to 1 pmole per mg of cell membrane in the case of a receptor. Subsequently, when the Geneticin is withdrawn, cell lines are obtained which contain the amplified gene integrated into one or more chromosome(s) of the host cell.

Clontech's Tet-Off and Tet-On gene expression systems and similar systems can be used to express SNEPa, SNEPb or SNEPc in a regulated way in mammalian cells (Gossen, M. and Bujard, H., Proc. Natl. Acad. Sci. USA 89: 5547-5551(1992)). For the polyadenylation of the mRNA, other signals, e.g., from the human growth hormone or globin genes can be used as well. Stable cell lines carrying a gene of interest integrated into the chromosomes can also be selected upon co-transfection with a selectable marker such as gpt, G418 or hygromycin.

HEK293 cells are used for transfection. 20 µg of the expression plasmid pcDNA3/SNEPa, pcDNA3/SNEPb or pcDNA3/SNEPc are transfected using calcium phosphate (Chen C, Okayama H, (1987) Mol. Cell. Biol. ; 7: 2745-2752.). The plasmid pcDNA3(-) contains a dominant selectable marker, the neomycin resistance gene from Tn5 encoding an enzyme that confers resistance to a group of antibiotics including Geneticin. The cells are seeded in MEM supplemented with 1 mg/ml Geneticin. After 2 days, the cells are trypsinized and seeded in cloning plates in MEM supplemented with 1 mg/ml Geneticin. After about 10-14 days, single clones are trypsinized and then seeded in Swell petri dishes or 10 ml flasks. Growing clones are then transferred to new 6-well plates. Expression of the desired gene product is analyzed, for instance, by Northern blot.

### Example 3

### Tissue distribution of SNEP variants mRNA expression.

Northern blot analysis can be carried out to examine SNEP gene expression in human tissues, using methods described by, among others, Sambrook et al., cited above. A cDNA probe containing the entire or a fraction of nucleotide sequence of the SNEPa, SNEPb or SNEPc proteins (SEQ ID NO : 1, SEQ IN NO : 3 or SEQ ID NO : 5) can be labeled with ³²P using the Megaprime DNA labeling system (Amersham Life Science), according to manufacturer's instructions. After labeling, the probe can be purified using a CHROMA SPIN-200 column (Clontech Laboratories, Inc.), according to manufacturer's protocol. The purified labeled probe is then used to examine various human tissues for SNEP variants mRNA expression.

Multiple Tissue Northern (MTN) blots containing various human tissues can be obtained from Clontech and examined with the labeled probe using ExpressHyb hybridization solution (Clontech) according to manufacturer's instructions. Following hybridization and washing, the blots can be mounted and exposed to film at -70°C overnight, and films developed according to standard procedures.

Using such methodologies, SNEP mRNAs were found in several regions such as brain areas, kidney, heart, lung, prostate, and pancreas.

It will be dear that the invention may be practiced otherwise than as particularly described in the foregoing description and examples.

Numerous modifications and variations of the present invention are possible in light of the above teachings and, therefore, are within the scope of the appended claims.

The entire disclosure of all publications (including patents, patent applications, journal articles, laboratory manuals, books, or other documents) cited herein are incorporated by reference.

## Claims

1. An isolated polynucleotide selected from the group consisting of:
(a) a polynucleotide comprising a nucleotide sequence that has at least 80% identity to a nucleotide sequence encoding SNEPa, SNEPb or SNEPc polypeptides of SEQ ID NO : 2, SEQ ID NO : 4 or SEQ ID NO : 6 over their entire length; or a nucleotide sequence complementary to said nucleotide sequences; or
(b) a polynucleotide comprising a nucleotide sequence that has at least 80% identity to a nucleotide sequence encoding SNEPa, SNEPb or SNEPc polypeptides expressed by the cDNA inserts deposited at the ATCC with Deposit Numbers PTA-187, PTA-189 and 203984 respectively ; or a nucleotide sequence complementary to said nucleotide sequences.

2. A polynucleotide according to claim 1 which is DNA or RNA.

3. A polynucleotide according to claim 1 or 2 wherein said nucleotide sequence is at least 80% identical to that contained in SEQ ID NO : 1, SEQ ID NO : 3 or SEQ ID NO : 5.

4. A polynucleotide according to claim 3 wherein said nucleotide sequences comprise SNEPa, SNEPb or SNEPc polypeptides encoding sequence contained in SEQ ID NO : 1, SEQ ID NO : 3 or SEQ ID NO : 5.

5. A polynucleotide according to claim 3 which is polynucleotide of SEQ ID NO : 1, SEQ ID NO : 3 or SEQ ID NO : 5.

6. A DNA or RNA molecule comprising an expression system, wherein said expression system is capable of producing SNEPa, SNEPb or SNEPc polypeptides comprising an amino acid sequence, which has at least 80% identity with the polypeptides of SEQ ID NO : 2, SEQ ID NO : 4 or SEQ ID NO : 6 when said expression system is present in a compatible host cell.

7. A host cell comprising the expression system of claim 6.

8. A process for producing SNEPa, SNEPb or SNEPc polypeptides comprising culturing a host of claim 7 under conditions sufficient for the production of said polypeptides and recovering the polypeptides from the culture.

9. A process for producing a cell which produces SNEPa, SNEPb or SNEPc polypeptides thereof comprising transforming or transfecting a host cell with the expression system of claim 6 such that the host cell, under appropriate culture conditions, produces SNEPa, SNEPb or SNEPc polypeptides.

10. Isolated SNEPa, SNEPb or SNEPc polypeptides comprising an amino acid sequence which is at least 80% identical to the amino acid sequences of SEQ ID NO : 2, SEQ ID NO : 4 or SEQ ID NO : 6 over its entire length.

11. Polypeptide according to claim 10 which comprises the amino acid sequences of SEQ ID NO : 2, SEQ ID NO : 4 or SEQ ID NO : 6.

12. Polypeptide according to claim 10 or 11 comprising an amino acid sequence which is at least 80% identical to the amino acid sequence encoded by the cDNAs contained in ATCC Deposit Nummbers PTA-187, PTA-189 or 203984.

13. Antibodies immunospecific for the SNEPa, SNEPb or SNEPc polypeptides of any of claims 10 to 12.

14. A method for the treatment of a subject in need of enhanced activity or expression of SNEPa, SNEPb or SNEPc polypeptides of any of claims 10 to 12 comprising:
(a) administering to the subject a therapeutically effective amount of an activator to said SNEPa, SNEPb or SNEPc polypeptides; and/or
(b) providing to the subject a polynucleotide according to any of claims 1 to 5 in a form so as to effect production of said SNEPa, SNEPb or SNEPc polypeptides activity in vivo.

15. A method for the treatment of a subject having need to inhibit activity or expression of SNEPa, SNEPb or SNEPc polypeptides of any of claims 10 to 12 comprising:
(a) administering to the subject a therapeutically effective amount of an inhibitor to said SNEPa, SNEPb or SNEPc polypeptides; and/or
(b) administering to the subject a nucleic acid molecule that inhibits the expression of the nucleotide sequence encoding said SNEPa, SNEPb or SNEPc polypeptides; and/or
(c) administering to the subject a therapeutically effective amount of a polypeptide that competes with said SNEPa, SNEPb or SNEPc polypeptides for its substrate.

16. A process for diagnosing a disease or a susceptibility to a disease in a subject related to expression or activity of SNEPa, SNEPb or SNEPc polypeptides of any of claims 10 to 12 in a subject comprising:
(a) determining the presence or absence of a mutation in the nucleotide sequence encoding said SNEPa, SNEPb or SNEPc polypeptides in the genome of said subject; and/or
(b) analyzing for the presence or amount of SNEPa, SNEPb or SNEPc polypeptides expression in a sample derived from said subject.

17. A method for identifying activator to SNEPa, SNEPb or SNEPc polypeptides of any of claims 10 to 12 comprising:
(a) contacting cells produced by claim 9 with a candidate compound; and
(b) determining whether the candidate compound effects a signal generated by activation of the SNEPa, SNEPb or SNEPc polypeptides.

18. An activator identified by the method of claim 17.

19. The method for identifying inhibitors to SNEPa, SNEPb or SNEPc polypeptides of any of claims 10 to 12 comprising:
(a) contacting said cell produced by claim 9 with an activator; and
(b) determining whether the signal generated by said agonist is diminished in the presence of a candidate compound.

20. An inhibitor identified by the method of claim 19.
